(19) Europäisches Patentamt
European Patent Office
Office européen des brevets

(11) **EP 2 616 816 B1**

(12) **EUROPÄISCHE PATENTSCHRIFT**

(45) Veröffentlichungstag und Bekanntmachung des Hinweises auf die Patenterteilung:
**02.11.2016 Patentblatt 2016/44**

(21) Anmeldenummer: **11808564.6**

(22) Anmeldetag: **16.09.2011**

(51) Int Cl.:
*G01N 33/68* (2006.01)     *G01N 33/50* (2006.01)
*C12Q 1/68* (2006.01)

(86) Internationale Anmeldenummer:
**PCT/DE2011/075225**

(87) Internationale Veröffentlichungsnummer:
**WO 2012/037937 (29.03.2012 Gazette 2012/13)**

(54) **VERFAHREN ZUM NACHWEIS, DIFFERENZIEREN UND QUANTIFIZIEREN VON T-ZELLPOPULATIONEN MITTELS DER REVERSE TRANSKRIPTION QUANTITATIVEN REAL-TIME PCR (RT-QPCR) TECHNOLOGIE**

METHOD FOR DETECTING, DIFFERENTIATING, AND QUANTIFYING T CELL POPULATIONS BY MEANS OF REVERSE TRANSCRIPTION QUANTITATIVE REAL-TIME PCR (RT-QPCR) TECHNOLOGY

PROCÉDÉ POUR DÉTECTER, DIFFÉRENCIER ET QUANTIFIER DES POPULATIONS DE LYMPHOCYTES T AU MOYEN DE LA TECHNOLOGIE DE LA RÉACTION EN CHAÎNE PAR POLYMÉRASE QUANTITATIVE EN TEMPS RÉEL À TRANSCRIPTION INVERSE (RT-QPCR)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.09.2010 DE 102010037622**

(43) Veröffentlichungstag der Anmeldung:
**24.07.2013 Patentblatt 2013/30**

(73) Patentinhaber: **Lophius Biosciences GmbH
93053 Regensburg (DE)**

(72) Erfinder:
• **DEML, Ludwig
  93128 Regenstauf (DE)**
• **NAUMANN, Kristina
  92224 Amberg (DE)**
• **SCHLOMBS, Kornelia
  93051 Regensburg (DE)**
• **BARABAS, Sascha
  93077 Bad Abbach (DE)**

(74) Vertreter: **Drexl, Janna
  Dehmel & Bettenhausen
  Patentanwälte PartmbB
  Herzogspitalstraße 11
  80331 München (DE)**

(56) Entgegenhaltungen:
EP-A2- 1 448 781          WO-A1-00/57705
WO-A1-2008/121420     WO-A2-03/012061
US-A1- 2002 193 296

• HABIB-AGAHI MOJTABA ET AL: "Co-stimulation with 4-1BB ligand allows extended T-cell proliferation, synergizes with CD80/CD86 and can reactivate anergic T cells", INTERNATIONAL IMMUNOLOGY, Bd. 19, Nr. 12, Dezember 2007 (2007-12), Seiten 1383-1394, XP002672615, ISSN: 0953-8178
• WEN T ET AL: "4-1BB ligand-mediated costimulation of human T cells induces CD4 and CD8 T cell expansion, cytokine production, and the development of cytolytic effector function", JOURNAL OF IMMUNOLOGY, AMERICAN ASSOCIATION OF IMMUNOLOGISTS, US, Bd. 168, Nr. 10, 15. Mai 2002 (2002-05-15) , Seiten 4897-4906, XP002225279, ISSN: 0022-1767
• RISSOAN M-C ET AL: "RECIPROCAL CONTROL OF T HELPER CELL AND DENDRITIC CELL DIFFERENTIATION", SCIENCE, AMERICAN ASSOCIATION FOR THE ADVANCEMENT OF SCIENCE, WASHINGTON, DC; US, Bd. 283, Nr. 5405, 19. Februar 1999 (1999-02-19), Seiten 1183-1186, XP000999147, ISSN: 0036-8075, DOI: 10.1126/SCIENCE.283.5405.1183

<110> Lophius Biosciences GmbH

<120> Verfahren zum Nachweis, Differenzieren und Quantifizieren von T-Zellpopulationen mittels der Reverse Transkription quantitativen Real-Time PCR (RT-qPCR) Technologie

<130> LOP-004 PCT

<140> Not yet known
<141> 2011-09-16

<150> 10 2010 037 622.1
<151> 2010-09-17

<160> 23

<170> PatentIn version 3.3

<210> 1
<211> 18
<212> DNA
<213> Artificial Sequence

<220>
<223> 4-1BBL Forward Primer

<400> 1
gagggtcccg agctttcg          18

<210> 2
<211> 19
<212> DNA
<213> Artificial Sequence

<220>
<223> 4-1BBL Reverse Primer

<400> 2
gcccatcgat cagcagaac          19

<210> 3
<211> 21
<212> DNA
<213> Artificial Sequence

<220>
<223> 4-1BBL Sonde

<400> 3
ccaccagctg cgcaaacatg c          21

<210> 4
<211> 19
<212> DNA
<213> Artificial Sequence

<220>
<223> GAPDH Forward Primer

<400> 4
gaaggtgaag gtcggagtc          19

<210> 5
<211> 21
<212> DNA
<213> Artificial Sequence

<220>
<223> GAPDH Reverse Primer

<400> 5
gtaaaccatg tagttgaggt c          21

<210> 6
<211> 24
<212> DNA

<213> Artificial Sequence

<220>
<223> GAPDH Sonde

<400> 6
tcattgatgg caacaatatc cact        24

<210> 7
<211> 23
<212> DNA
<213> Artificial Sequence

<220>
<223> IFNG Forward Primer

<400> 7
gtggagacca tcaaggaaga cat        23

<210> 8
<211> 19
<212> DNA
<213> Artificial Sequence

<220>
<223> IFNG Reverse Primer

<400> 8
ggcgacagtt cagccatca        19

<210> 9
<211> 30
<212> DNA
<213> Artificial Sequence

<220>
<223> IFNG Sonde

<400> 9
ttcatgtatt gctttgcgtt ggacattcaa        30

<210> 10
<211> 19
<212> DNA
<213> Artificial Sequence

<220>
<223> huP0 Forward Primer

<400> 10
gtggtgctga tgggcaaga        19

<210> 11
<211> 21
<212> DNA
<213> Artificial Sequence

<220>

<223> huP0 Reverse Primer

<400> 11
gcagcagttt ctccagagct g        21

<210> 12
<211> 22
<212> DNA
<213> Artificial Sequence

<220>
<223> huP0 Sonde

<400> 12
accatgatgc gcaaggccat cc        22

<210> 13
<211> 18
<212> DNA
<213> Artificial Sequence

<220>
<223> PBGD Forward Primer

<400> 13
ccagctccct gcgaagag        18

<210> 14
<211> 20
<212> DNA
<213> Artificial Sequence

<220>
<223> PBGD Reverse Primer

<400> 14
cactgaactc ctgctgctcg        20

<210> 15
<211> 24
<212> DNA
<213> Artificial Sequence

<220>
<223> PBGD Sonde

<400> 15
cccagctgca gagaaagttc ccgc        24

<210> 16
<211> 17
<212> DNA
<213> Artificial Sequence

<220>
<223> FCGR1A/B/C Forward Primer

<400> 16

gaaggggtgc accggaa          17

<210> 17
<211> 18
<212> DNA
<213> Artificial Sequence

<220>
<223> FCGR1A/B/C Reverse Primer

<400> 17
ctcacgggga gcaagtgg          18

<210> 18
<211> 22
<212> DNA
<213> Artificial Sequence

<220>
<223> CXCL9 Forward Primer

<400> 18
gagtgcaagg aaccccagta gt          22

<210> 19
<211> 22
<212> DNA
<213> Artificial Sequence

<220>
<223> CXCL9 Reverse Primer

<400> 19
ggtggatagt cccttggttg gt          22

<210> 20
<211> 21
<212> DNA
<213> Artificial Sequence

<220>
<223> CXCL10 Forward Primer

<400> 20
tccacgtgtt gagatcattg c          21

<210> 21
<211> 21
<212> DNA
<213> Artificial Sequence

<220>
<223> CXCL10 Reverse Primer

<400> 21
tcttgatggc cttcgattct g          21

<210> 22

<211> 19
<212> DNA
<213> Artificial Sequence

<220>
<223> CXCL11 Forward Primer

<400> 22
caaggcttcc ccatgttca          19

<210> 23
<211> 19
<212> DNA
<213> Artificial Sequence

<220>
<223> CXCL11 Reverse Primer

<400> 23
cccagggcgt atgcaaaga          19


**Beschreibung**

Die vorliegende Erfindung betrifft Verfahren zum Nachweis, Differenzieren und Quantifizieren von T-Zellpopulationen, umfassend die folgenden Schritte a) in Kontakt Bringen eines ersten Aliquots einer Körperflüssigkeit eines Individuums mit mindestens einem Antigen, wobei die Körperflüssigkeit antigenpräsentierende Zellen (APC) und T-Zellen enthält, b) Inkubieren des ersten Aliquots mit dem mindestens einen Antigen über einen bestimmten Zeitraum, c) Nachweis und Differenzieren der T-Zellpopulationen durch Detektieren mindestens eines ersten durch die T-Zellen einer bestimmten T-Zellpopulation induzierten Markers der APC in dem ersten Aliquot und in einem zweiten Aliquot der Körperflüssigkeit des Individuums, das nicht mit dem mindestens einen Antigen inkubiert wurde, durch Reverse Transkription quantitative Real-Time Polymerase Kettenreaktion (RT-qPCR), und d) Nachweis und Quantifizieren der T-Zellpopulationen durch Bestimmen des Verhältnisses des detektierten Markers der APC des ersten Aliquots zu dem zweiten Aliquot.

T-Zellen nehmen in dem komplexen Netzwerk der Immunabwehr mikrobieller Infektionen und Tumorerkrankungen eine zentrale Stellung ein, indem sie die Immunantwort koordinieren und Pathogene sowie Tumorzellen über vielfältige direkte und indirekte Effektorfunktionen kontrollieren und eliminieren. Eine Beeinträchtigung der komplexen T-Zellfunktion kann zur fälschlichen Aktivierung autoaggressiver T-Zellen führen und damit einhergehend schwerwiegende Autoimmunerkrankungen wie Multiple Sklerose (MS), Rheumatoide Arthritis (RA) und die juvenile Zuckerkrankheit (Typ I Diabetes) hervorrufen.

Grundsätzlich weisen T-Zellen phänotypisch eine große Heterogenität sowie ein breites Spektrum von Effektorfunktionen auf. So können T-Zellen anhand der Expression der Oberflächenproteine CD4 und CD8 grob in CD4-positive T-Zellen (T-Helferzellen (Th)) und CD8-positive zytotoxische T-Zellen (CTL) unterteilt werden.

$CD4^+$ T-Zellen haben bei der Aktivierung, Polarisierung und Koordination der Immunabwehr eine zentrale Bedeutung. $CD4^+$ T-Zellen werden infolge einer spezifischen Interaktion ihres T-Zellrezeptors mit auf der Oberfläche von Antigen-präsentierenden Zellen (APC) befindlichen Peptid-beladenen MHC Klasse-II Molekülen aktiviert und steuern nachfolgend durch Zell-Zellkontakt und/oder die Sekretion verschiedener Botenstoffe (beispielsweise Zytokine, Chemokine), die Produktion von Antikörpern durch B-Zellen (humoraler Ast der Immunantwort) und die Aktivierung von CTL (zellulärer Ast der Immunantwort).

$CD4^+$ T-Zellen können anhand der Expression charakteristischer Oberflächenproteine und der Produktion von Markerzytokinen in T-Helfer 1 (Th-1), T-Helfer 2 (Th-2) und T-Helfer 17 (Th-17) Zellen untergliedert werden. Th-1 Zellen sind charakterisiert durch die Produktion der Zytokine IFN-$\gamma$ und TNF-$\alpha$ sowie die Expression des Transkriptionsfaktors T-bet. Th-1 Zellen unterstützen die Ausprägung einer effizienten zellvermittelten Immunantwort, indem sie die Aktivierung und Differenzierung von Makrophagen, $CD4^-CD8^+$ zytotoxischen T-Zellen, $CD4^+CD8^+$ zytotoxischen T-Zellen aber auch natürlichen Killerzellen (NK Zellen) und NKT-Zellen fördern. Th-2 Zellen sind charakterisiert durch die Sekretion der Zytokine IL-4, IL-5, IL-6, IL-10 und IL-13 sowie die Produktion des Transkriptionsfaktors GATA-3 und unterstützen die Produktion sowie den Klassenwechsel von Antikörpern (humoraler Ast des Immunsystems) in B-Zellen. Th-17 Zellen sind gekennzeichnet durch die Produktion der Zytokine IL-17, TNF-□, GM-CSF und IL-6 und scheinen bei rheumatologischen Autoimmunerkrankungen eine bedeutende Rolle zu spielen. Neben Th-1, Th-2 und Th-17 Zellen wurde mit regulatorischen T-Zellen eine weitere Population $CD4^+$ T-Zellen definiert, die bei der Dämpfung von Immunantworten, der oralen Toleranz sowie der Verhinderung von Autoimmunerkrankungen eine bedeutende Rolle spielt. Regulatorische T-Zellen lassen sich in $CD4^+$ $CD25^+$ $CTLA4^+$ natürliche regulatorische T-Zellen (Treg) sowie Th-3 und Tr-1 Zellen untergliedern, die durch die Produktion der Zytokine TGF-ß (Th-3 Zellen) oder IL-10 (Tr-1 Zellen) gekennzeichnet sind.

CTL spielen bei der Bekämpfung von mit Mikroorganismen und Parasiten infizierten Zellen und Geweben sowie von Tumorzellen eine zentrale Rolle, indem sie diese durch direkte Effektormechanismen, wie die Ausschüttung zytotoxischer Substanzen (z.B. Perforin, Granzym) und das Auslösen von Apoptose zerstören. Zudem verfügen CTL mit der Sekretion immunstimulatorischer Zytokine (IFN-□, TNF-□, IL-15) und Chemokine (MIP1□, MIP1ß, Rantes) sowie verschiedener löslicher, antiviraler Faktoren (IFN-□, IFN-ß, IFN-□, CAF) über weitere, zum Teil sehr spezifische Effektorfunktionen, die sehr effizient zur Begrenzung der Replikation und Verbreitung von Pathogenen beitragen. Darüber hinaus wurden weitere Populationen zytotoxischer T-Zellen beschrieben, die einen $CD4^+CD8^+$ Phänotyp ($CD4^+CD8^{dim}$, $CD4^{dim}CD8^{bright}$ oder $CD4^{hi}CD8^{hi}$) aufweisen.

Somit stellen T-Zellen einen bedeutenden Protektionsmechanismus des erworbenen Immunsystems für die Prävention und Kontrolle mikrobieller und insbesondere Virusinduzierter Erkrankungen sowie für die Erkennung und Zerstörung von Tumorzellen dar.

Die Aktivierung, Polarisierung und Regulation spezifischer T-Zellen unterliegt einer strengen Kontrolle durch APC und wird im Wesentlichen durch den Subtyp und Reifungsgrad der APC, den Mechanismus der Antigenaufnahme und Präsentation sowie intrinsische Eigenschaften des jeweiligen Immunogens bestimmt. Hierbei entscheiden die Dosis und Lokalisierung eines Immunogens sowie der Gehalt an immunmodulatorischen Substanzen, ob sich eine Th-1, Th-2 oder Th-17-vermittelte Immunreaktion entwickelt oder ob eine Toleranz induziert wird.

Professionelle APC, wie Dendritische Zellen (DC), Monozyten und Makrophagen, aber auch B-Zellen nehmen an der Schnittstelle zwischen dem angeborenen und erworbenen Immunsystem eine Schlüsselposition ein, indem sie Erreger und Tumorzellen spezifisch erkennen, aufnehmen und deren Bruchstücke in Verbindung mit MHC-Molekülen der Klassen-I und -II an T-Zellen präsentieren. Zudem können Fibroblasten der Haut, Epithelzellen des Thymus und der Schilddrüse, Gliazellen, Betazellen der Bauchspeicheldrüse sowie vaskuläre Endothelzellen als nicht professionelle APC fungieren. Des Weiteren belegen aktuelle Untersuchungen, dass auch T-Zellen als APC fungieren können. Diese APC-T-Zellen entstehen durch den interzellulären Transfer von MHC Klasse-I und -II Molekülen sowie kostimulatorischer Moleküle, z.B. CD80, CD40 Ligand (CD40L), OX40 Ligand (OX40L) und 4-1BB Ligand (4-1BBL, TNFSF9), infolge eines Kontakts mit einer APC, insbesondere einer DC (Sokke Umeshappa et al. (2009), J. Virol. 182:193-206).

Für eine erfolgreiche Stimulation von T-Zellen durch APC sind drei voneinander unabhängige Signale erforderlich: die spezifische Erkennung Peptid-beladener MHC Moleküle mittels des T-Zell Rezeptors (TCR) (Signal 1), die Interaktion von APC- und T-Zell-ständigen kostimulatorischen Molekülen und ihren Liganden (Signal 2), sowie die Anwesenheit T-Zell-polarisierender Zytokine, wie IFN-□, IL-12, IL-4, IL-6 und TGF-ß (Signal 3).

Extrazellulär vorliegende, lösliche Proteine werden üblicherweise über den exogenen Antigen-Prozessierungsweg abgebaut und die erzeugten Peptide im Komplex mit MHC-II Molekülen auf der Oberfläche von APC präsentiert. Mit MHC Klasse II Molekülen komplexierte Peptide werden von CD4+ T-Zellen (T-Helferzellen) erkannt.

Dagegen erfolgt der Abbau zytosolisch vorliegender Proteine mittels des endogenen Prozessierungswegs, der zu einer Präsentation der erzeugten Epitope auf MHC Klasse-I Molekülen führt. Diese Peptid/MHC-I Komplexe werden zur Oberfläche von APC transportiert, wo sie zytotoxischen T-Zellen (CTL) präsentiert werden. Obwohl die Mehrzahl der auf MHC Klasse-I Molekülen präsentierten Epitope endogenen und der mit MHC -II Molekülen komplexierten Peptide exogenen Proteinen entstammen, ist diese Trennung nicht absolut. So gelangen beispielsweise verschiedene exogen vorliegende Immunogene, wie partikuläre Strukturen, verschiedene Viruspartikel, Immunkomplexe und Lipoproteine mittels eines als Kreuz-Präsentation bezeichneten Mechanismus in den endogenen Prozessierungsweg zur Epitoppräsentation auf MHC-I Molekülen.

Für eine spezifische Aktivierung naiver T-Zellen ist neben der Erkennung Peptid-beladener MHC-Moleküle (Signal 1) ein zweites, kostimulatorisches Signal erforderlich (Signal 2). Dieses wird durch die Interaktion verschiedener APC- und T-Zell-ständiger, kostimulatorischer Liganden mit ihren Rezeptoren ausgelöst. Zu den bedeutendsten Vertretern kostimulatorischer Moleküle gehören Mitglieder der TNF/TNF-Rezeptor Superfamilie sowie der Immunglobulin Superfamilie. In Abwesenheit des kostimulatorischen Signals wird die T-Zelle anerg. Anergie beschreibt einen Zustand, in dem die T-Zellen sich nicht vermehren und nicht auf ein Antigen reagieren.

Die Expression von kostimulatorischen Signalen auf APC wird im Wesentlichen durch exogene Reize, z.B. Bestandteilen von Pathogenen oder traumatisierten Geweben, sowie Zytokinen gesteuert. Die Aktivierung und Reifung von APC resultiert in einer gesteigerten Expression proinflammatorischer und T-Zell-polarisierender Zytokine sowie kostimulatorischer Moleküle (CD80, CD86 und CD40), wodurch die Fähigkeit von APC zur Aktivierung zellvermittelter Immunreaktionen drastisch gesteigert wird. Die spezifische Antigenerkennung durch den TCR und die Interaktion mit kostimulatorischen Molekülen induziert eine gerichtete Aktivierung und Proliferation erreger- bzw. krankheitsspezifischer naiver T-Zellen. Diese geht einher mit der gesteigerten Sekretion von IL-2 und der Expression des CD40 Liganden, welche bei der nachfolgenden Aktivierung und Expansion anderer Subpopulationen spezifischer T-Zellen eine wichtige Rolle spielen. Die Polarisierung aktivierter T-Zellen in Th-1 oder Th-2 Effektorzellen erfolgt in Abhängigkeit von dem Reifungsgrad der APC, dem vorherrschenden Zytokinmilieu sowie intrinsischen Eigenschaften und der Dosis des vorliegenden Antigens.

Die Ausprägung einer spezifischen T-Zellantwort im Verlauf einer akuten mikrobiellen Infektion erfolgt üblicherweise in drei Schritten: Während der Effektorphase werden Antigen-spezifische naive T-Zellen durch den Kontakt mit Antigen-beladenen APC aktiviert, was zu einer dramatischen Expansion der spezifischen T-Zellen, der Ausprägung von Effektorfunktionen und dem Einwandern der aktivierten Effektor T-Zellen an den Infektionsort führt. Diese Effektorphase erstreckt sich üblicherweise über 1 bis 2 Wochen bis zur Eliminierung des Erregers. In der nachfolgenden mehrwöchigen Kontraktionsphase sterben mehr als 90% der gebildeten Effektor-T-Zellen ab. Nur wenige Antigen-spezifische T-Zellen überleben und differenzieren zu langlebigen Gedächtnis-T-Zellen. In der Gedächtnisphase persistieren diese Gedächnis-T-Zellen mit relativ stabiler Zellzahl über viele Jahre im Körper. Diese Gedächtnis-T-Zellen können bei einem erneuten Kontakt mit ihrem Antigen schnell reaktiviert werden und ihre Effektorfunktion ausüben.

Zur Vermeidung unerwünschter Immunreaktionen gegen körpereigene Proteine und Gewebe werden autoreaktive T-Zellen frühzeitig durch klonale Depletion eliminiert oder inaktiviert (Anergie). Die Folge ist eine Antigen-spezifische Toleranz gegen körpereigene Strukturen, wie Proteine, Zellen, Gewebe und Organe. Bei Autoimmunerkrankungen sind diese Schutzmechanismen gestört oder nur unzureichend ausgeprägt. Es gibt eine Reihe von Hinweisen darauf, dass Autoimmunkrankheiten durch eine angeborene "Empfänglichkeit", (genetische Disposition), in Kombination mit äußeren Einflüssen, z.B. mikrobielle Infektionen, Schwangerschaft oder aufgrund der Ähnlichkeit körpereigener Strukturen mit Erreger- und Fremdgewebe-spezifischen Polypeptiden, die so genannte molekulare Mimikry, erworben werden.

Daneben spielen aktivierte T-Zellen auch bei der Ausbildung chronischer Virusinfektionen und der Abstoßung von transplantierten Geweben und Organen eine zentrale Rolle.

Die Bestimmung des Phänotyps, der Frequenz, der Spezifität, Funktionalität und des Aktivierungszustandes von T-Zellen stellt eine effiziente Strategie dar, um Aufschluss über einen aktuellen Krankheitsverlauf oder über bereits überwundene Erkrankungen zu erlangen. Des Weiteren sind solche Verfahren beispielsweise zur Beobachtung (Monitoring) spezifischer T-Zellantworten bei therapeutischen und prophylaktischen Impfungen, sowie zum diagnostischen Nachweis der Zahl und Funktionalität von T-Zellen bei chronischen Entzündungen, Autoimmunerkrankungen und bei der Transplantatabstoßung von großer Bedeutung.

In den letzten Jahrzehnten wurden verschiedene Technologien zum Nachweis von T-Zellen entwickelt, die sich grob in zwei Kategorien einteilen lassen. Die erste Gruppe von Verfahren beruht auf der direkten Identifizierung und Quantifizierung Polypeptid-spezifischer T-Zellen unter Einsatz von Peptid-MHC Multimeren, wie beispielsweise Tetrameren (Beckman Coulter), Pentameren (Proimmune) und Streptameren (IBA). Dieses Verfahren ermöglicht eine Bestimmung Epitop-spezifischer T-Zellen mit bekannter MHC-Restriktion. Eine Analyse der Funktionalität von T-Zellen ist mit diesem Verfahren nicht möglich. Die bedeutendsten Einschränkungen bei der Verwendung von Peptid/MHC Multimeren für das Routine-Monitoring Krankheits- oder Erreger-spezifischer T-Zellen ergeben sich daraus, dass die MHC/Peptid Multimere sowohl Epitop als auch HLA-spezifisch sind. Somit erfordert ein umfassendes Monitoring Erreger- oder Krankheits-spezifischer T-Zellen in Probanden mit unterschiedlicher HLA Konstellation den Einsatz eines breiten Spektrums unterschiedlicher Peptid/MHC Multimere, was mit einem hohen Kostenaufwand verbunden ist. Zudem sind diese Peptid/MHC Multimere bislang nur für ein begrenztes Spektrum von MHC Molekülen verfügbar.

Ein weiteres Verfahren zur Bestimmung spezifischer T-Zellen beruht auf dem Einsatz Peptid-beladener HLA-Moleküle, die mit grün fluoreszierendem Protein (GFP) gekoppelt sind. Die epitopspezifische Erkennung und Bindung dieser Komplexe durch den T-Zell-Rezeptor führt zu einer Internalisierung der GFP-markierten Peptide, wodurch die entsprechende CTL sichtbar wird (Tomaru et al. (2003), Nat Med. 9:469).

Im Gegensatz dazu beruhen die "funktionellen" Verfahren zum Monitoring spezifischer T-Zellen auf der *ex vivo* Stimulation von T-Zell- und APC-haltigen Patientenmaterialien mit Stimulatorantigenen und dem anschließenden Nachweis von Reifungsprozessen, wie Proliferation, Produktion von Markerzytokinen, in spezifisch reaktivierten T-Zellen mittels verschiedener Nachweissysteme. Der Nachweis spezifischer CD4$^+$ T-Zellen erfolgt hierbei üblicherweise durch die Stimulation von APC und T-Zell-haltigen Patientenproben, z.B. heparinisiertem Vollblut oder isolierten peripheren mononukleären Zellen des Blutes (PBMC) mit Proteinen, Polypeptiden oder Peptiden mit einer Länge von 15 bis 25 Aminosäuren und dem Nachweis der spezifischen T-Zellaktivierung durch die Bestimmung der Produktion charakteristischer Markerzytokine oder der T-Zell Proliferation. Der Zytokinnachweis erfolgt z.B. mittels durchflusszytometrischer Verfahren, wie intrazelluläre Zytokinfärbung und dem Zytokin-Sekretion Assay sowie der ELISpot- oder ELISA-Technik. Die Bestimmung der T-Zellproliferation kann z.B. mittels eines Bromdeoxyuridin (BrdU)- oder Carboxylfluorescein Diacetat Succinimidylester (CSFE) Proliferations-Assays ermittelt werden.

Dagegen erfolgt der Nachweis spezifischer CD8$^+$ T-Zellen (CTL) üblicherweise durch die Stimulation von APC und T-Zell-haltigen Patientenproben mit kurzen Peptiden mit einer Länge von 8 bis 16 Aminosäuren. Darüber hinaus kann durch die Infektion mit rekombinanten Viren oder Bakterien, die die Zielstrukturen von T-Zellen intrazellulär in APC exprimieren und die anschließende Bestimmung der von Antigen-spezifischen T-Zellen produzierten Markerzytokine, meist IFN-☐ mittels durchflusszytometrischer Verfahren, z.B. intrazelluläre Zytokinfärbung oder unter Einsatz der ELISpot- bzw. ELISA-Technologie, spezifisch CTL nachgewiesen werden. Alternativ kann der spezifische Nachweis von CTL mittels eines $^{51}$Chrom-Freisetzungstests oder unter Einsatz adäquater nicht radioaktiver Verfahren, wie beispielsweise dem Laktat-Dehydrogenase Zytotoxizitätstest (z.B. von Clontech) erfolgen.

Diese verfügbaren Technologien ermöglichen eine Bestimmung krankheits- und erregerspezifischer CD4$^+$ oder CD8$^+$ Gedächtnis-T-Zellen, eignen sich aber nicht oder nur sehr begrenzt zum Nachweis aktivierter T-Helferzellen, die nur transient bei aktiven Krankheitsgeschehen auftreten.

CD4$^+$ T-Zellen werden bei aktiven mikrobiellen Infektionen und Krankheitsgeschehen transient aktiviert. Transiente Aktivierung bedeutet hierbei, dass diese T-Zellen nur über ein bestimmtes, relativ kurzes Zeitfenster hinweg präsent sind. Somit stellen aktivierte T-Helferzellen einen wichtigen Gegenstand für einen Nachweis von aktiven Krankheitsgeschehen dar. Ein möglichst aussagekräftiger Nachweis ist notwendig, um aktivierte T-Zellen im Rahmen einer Diagnose von aktiven infektiösen Erkrankungen und Autoimmunerkrankungen genau zu bestimmen.

Für bestimmte Anwendungen, bei denen zwischen aktivierten und nicht aktivierten T-Zellen unterschieden werden soll, wie beispielsweise bei dem Nachweis aktivierter T-Zellen bei akuten mikrobiellen Infektionen und Reaktivierungen oder dem Nachweis aktivierter autoaggressiver T-Zellen bei Verdacht auf Multiple Sklerose oder Typ 1 Diabetes sind auf der *in vitro* Restimulation von Gedächtnis-T-Zellen basierende Verfahren jedoch nicht oder nur sehr bedingt anwendbar.

Die bisher verfügbaren Methoden zum Nachweis von aktivierten CD4$^+$ T-Zellen weisen bislang nur eine geringe Sensitivität auf und sind daher nachteilhaft. Ursache für diese geringe Sensitivität liegt unter anderem darin, dass erreger- oder krankheitsspezifische aktivierte CD4$^+$ T-Zellen direkt bestimmt werden und üblicherweise nur in geringem Ausmaß in Patientenmaterial vorhanden sind. Diese geringe Anzahl vorhandener spezifisch aktivierter T-Zellen erschwert jedoch einen zuverlässigen und eindeutigen Nachweis, der auch diagnostischen Anforderungen gerecht wird, zumal die Nachweisgrenze dieser bisher verfügbaren Methoden oft unterschritten wird.

So beruhen die bislang verfügbaren Verfahren zum Nachweis Antigen-spezifischer aktivierter T-Helferzellen beispielsweise auf der durchflusszytometrischen Bestimmung von Proteinen, die transient auf der Oberfläche von aktivierten T-Zellen exprimiert werden. Zu diesen gehören insbesondere der CD40 Ligand und das CD25 Protein. Der CD40 Ligand ist jedoch durchflusszytometrisch nur schwer bestimmbar, weil die Bindung von Antikörpern zu einer Internalisierung des CD40 Liganden führt. Im Gegensatz dazu ist das CD25 Protein nicht nur auf aktivierten T-Helferzellen sondern auch auf regulatorischen T-Zellen zu finden und eignet sich somit nicht für eine gesicherte Unterscheidung dieser beiden Subpopulationen von T-Helferzellen. Zudem stellen die gesteigerte Expression von HLA-DR und CD69 sowie eine reduzierte Expression von CD27 weitere Marker für aktivierte T-Zellen dar. Der Nachweis aktivierter Antigen-spezifischer T-Zellen erfordert hier einen gemeinsamen Nachweis von Markern zur Bestimmung der Spezifität, z.B. unter Einsatz spezifischer Tetra-, Penta- oder Streptamere, des Phänotyps, z.B. durch Bestimmung charakteristischer Oberflächenmarker, und/oder der T-Zell-Aktivierung, z.B. durch die Produktion von Markerzytokinen, nach einer spezifischen Restimulation. Nachteilig an den bisher bekannten Verfahren ist weiterhin, dass der verlässliche Nachweis einiger Markerproteine mittels der ELISA, ELISpot oder FACS Technologie aufgrund der Membranlokalisation der Markerproteine, des Vorhandenseins präformierter Markerproteine in intrazellulären Vesikeln und der hohen unspezifischen Reaktivität der verfügbaren Antikörper mit zellulären Proteinen unmöglich ist.

Es besteht daher ein Bedarf an einem Verfahren, das es ermöglicht, spezifische T-Zellpopulationen, die durch bakterielle, virale, parasitäre oder Auto-Antigene aktiviert werden, nachzuweisen, zu differenzieren und zu quantifizieren und dadurch diese T-Zellpopulationen konkreten Erkrankungen und Krankheitsstadien zuordnen zu können.

Der vorliegenden Erfindung liegt die Aufgabe zugrunde, ein Verfahren bereitzustellen, das sowohl einen qualitativen und sensitiven als auch einen quantitativen Nachweis von erreger- oder krankheitsspezifischen T-Zellen bestimmter T-Zellpopulationen ermöglicht.

Der Erfindung liegt zudem die Aufgabe zugrunde, ein Verfahren bereitzustellen, das eine Differenzierung bestimmter T-Zellen, wie z. B. Antigen-spezifischer naiver T-Zellen, aktivierte T-Zellen und Gedächtnis-T-Zellen als unterschiedliche spezifische T-Zellpopulationen ermöglicht.

Die der Erfindung zugrunde liegende Aufgabe wird durch den in den Patentansprüchen definierten Gegenstand gelöst.

Die folgenden Figuren dienen der Erläuterung der Erfindung.

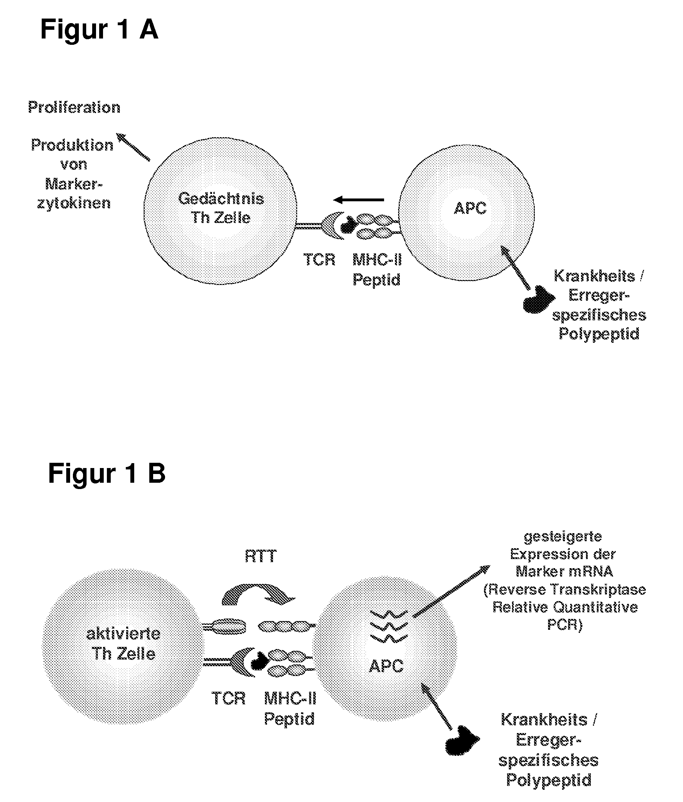

Figur 1 zeigt eine schematische Darstellung (A) des Prinzips bislang verwendeter Technologien gemäß dem bekannten Stand der Technik und (B) des erfindungsgemäßen Verfahrens zum Nachweis spezifischer T-Zellen.

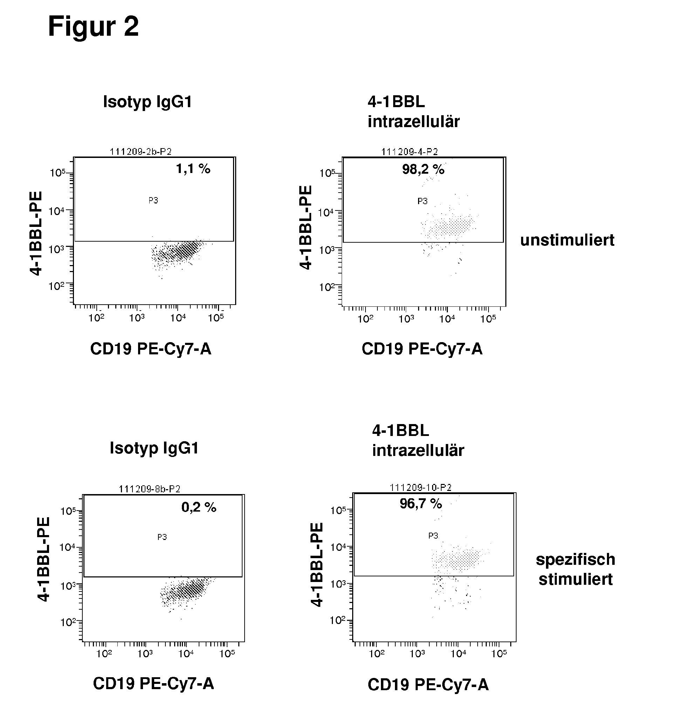

Figur 2 zeigt in der Dot Blot Darstellung die Anzahl der B-Zellen mit intrazellulär nachweisbarem 4-1BBL Protein in spezifisch mit dem ESAT-6/CFP-10 Fusionsprotein stimulierten und nicht stimulierten Kokulturen *ex vivo* voraktivierter ESAT-6/CFP-10 spezifischer T-Zellen und PBMC eines Blutspenders mit einer latenten Tuberkulose. *In vitro* expandierte voraktivierte ESAT-6/CFP-10 spezifische T-Helferzellen wurden 1:1 1 mit autologen PBMC gemischt und in An- oder Abwesenheit von 10 μg/ml ESAT-6/CFP-10 für 18 h kokultiviert. Während der letzten 16 h wurde die Proteinsekretion durch Brefeldin A inhibiert. Der Nachweis von intrazellulärem 4-1BBL in B-Zellen erfolgte durchflusszytometrisch.

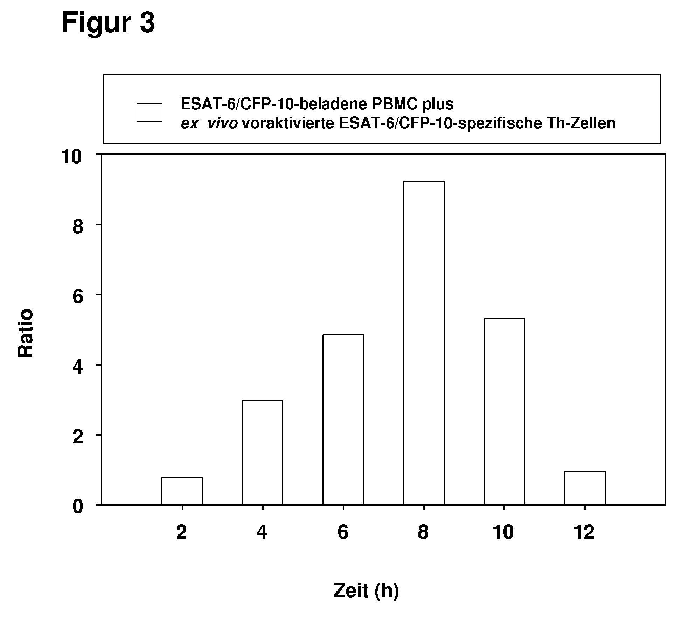

Figur 3 zeigt in einem Diagramm den relativen Anstieg der 4-1BBL mRNA Synthese in ESAT-6/CFP-10 beladenen PBMC im Vergleich mit unbeladenen PBMC nach Kokultur mit autologen *in vitro* expandierten voraktivierten ESAT-6/CFP-10-spezifischen T-Helferzellen ermittelt durch RT-qPCR. Frisch isolierte PBMC eines Spenders mit einer latenten *M. tuberculosis* Infektion wurden für 12 Stunden mit 10 μg/ml ESAT-6/CFP-10 inkubiert und anschließend im Verhältnis von 1:1 mit *ex vivo* expandierten autologen ESAT-6/CFP-10-spezifischen T-Helferzellen kokultiviert. Eine Kokultur von ESAT-6/CFP-10-spezifischen Th-Zellen und unbeladenen PBMC diente als Negativkontrolle. Zu den angezeigten Zeitpunkten wurden die Stimulationsansätze geerntet und der relative Anstieg der 4-1BBL mRNA Expression in spezifisch stimulierten im Vergleich mit nicht stimulierten Zellkulturen mittels RT-qPCR bestimmt. Die Daten der RT-qPCR wurden nach der $2^{-\Delta\Delta Cq}$ Methode ausgewertet, wobei die GAPDH als Referenzgen und die unstimulierte Kontrolle als Kalibrator dienten.

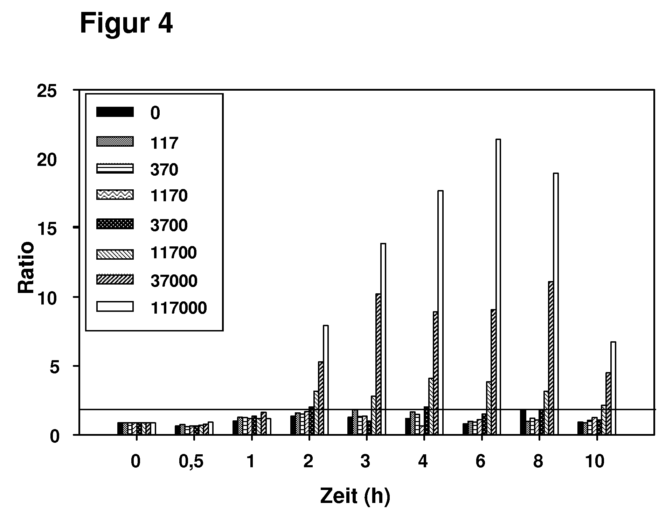

Figur 4 zeigt in einem Diagramm die Korrelation des relativen Anstiegs der 4-1BBL mRNA Produktion in ESAT-6/CFP-10 beladenen PBMC versus unbeladenen PBMC mit der Anzahl Antigen-spezifischer aktivierter ESAT-6/CFP-10-spezifischer T-Helferzellen ermittelt durch RT-qPCR. $1\times10^6$ frisch isolierte PBMC eines Spenders mit einer latenten *M. tuberculosis* Infektion wurden mit steigenden Zahlen *ex vivo* expandierten ESAT-6/CFP-10 spezifischen T-Helferzellen in Anwesenheit von 10 μg/ml ESAT-6/CFP-10 kultiviert. Nicht stimulierte Kokulturen autologer PBMC und voraktivierter ESAT-6/CFP-10-spezifischer Th-Zellen dienten als Kontrolle. Zu den angezeigten

Zeitpunkten wurden $2 \times 10^5$ Zellen geerntet und der relative Anstieg der 4-1BBL mRNA Konzentration in spezifisch stimulierten Zellen im Vergleich mit unstimulierten Zellen mittels RT-qPCR bestimmt. Die Daten der RT-qPCR wurden nach der $2^{-\Delta\Delta Cq}$ Methode ausgewertet, wobei die GAPDH als Referenzgen und die unstimulierte Kontrolle als Kalibrator dienten.

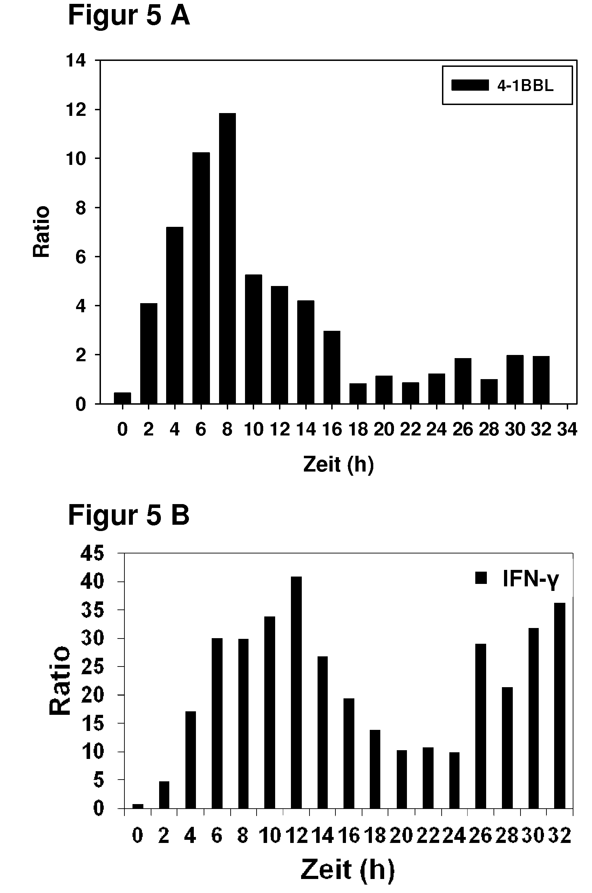

Figur 5 zeigt in (A) in einem Diagramm den relativen Anstieg der 4-1BBL mRNA Produktion in mit ESAT-6/CFP-10-beladenen und unbeladenen PBMC infolge einer Kokultivierung mit *ex vivo* voraktivierten autologen ESAT-6/CFP-10-spezifischen T-Helferzellen ermittelt durch RT-qPCR. $1 \times 10^6$ frisch isolierte PBMC eines Spenders mit einer latenten Tuberkulose wurden mit 50000 *ex vivo* voraktivierten ESAT-6/CFP-10-spezifischen T-Helferzellen in Anund Abwesenheit von 10 μg/ml ESAT-6/CFP-10 kokultiviert. Zu den angegebenen Zeitpunkten wurden $2 \times 10^5$ Zellen entnommen, pelletiert und in flüssigem Stickstoff eingefroren. Der relative Anstieg der 4-1BBL mRNA in spezifisch stimulierten Zellkulturen im Vergleich mit unstimulierten Zellen wurde mittels RT-qPCR bestimmt. Die Daten der RT-qPCR wurden nach der $2^{-\Delta\Delta Cq}$ Methode ausgewertet, wobei GAPDH als Referenzgen und die unstimulierte Kontrolle als Kalibrator diente. (B) zeigt in einem Diagramm den relativen Anstieg der IFN-□ mRNA Produktion im Verlauf der Kokultur ESAT-6/CFP-10 beladener und unbeladener PBMC mit *ex vivo* voraktivierten autologen ESAT-6/CFP-10-spezifischen T-Helferzellen ermittelt mittels der RT-qPCR. $1 \times 10^6$ frisch isolierte PBMC eines Spenders mit einer latenten Tuberkulose wurden mit 50000 *ex vivo* voraktivierten ESAT-6/CFP-10-spezifischen T-Helferzellen in An- und Abwesenheit von 10 μg/ml ESAT-6/CFP-10 kokultiviert. Zu den angegebenen Zeitpunkten wurden $2 \times 10^5$ Zellen entnommen, pelletiert und der relative Anstieg der IFN-γ mRNA in spezifisch stimulierten Zellkulturen im Vergleich mit unstimulierten Zellkulturen mittels RT-qPCR bestimmt. Die Daten der RT-qPCR wurden nach der $2^{-\Delta\Delta Cq}$ Methode ausgewertet, wobei die GAPDH als Referenzgen und die unstimulierte Kontrolle als Kalibrator dienten.

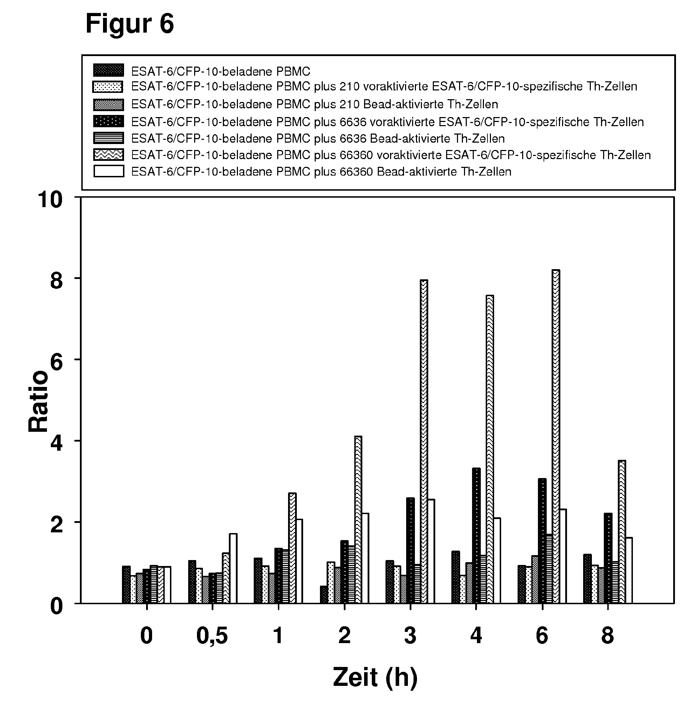

Figur 6 zeigt in einem Diagramm die Antigen-Spezifität der Induktion der 4-1BBL mRNA Synthese durch aktivierte Th-Zellen ermittelt mittels der RT-qPCR Technik. Voraktivierte ESAT-6/CFP-10-spezifische T-Zellen zeigen im Vergleich mit durch Expander Beads voraktivierten nicht ESAT-6/CFP-10-spezifischen T-Zellen eine deutlich verbesserte Fähigkeit zur Induktion der 4-1BBL mRNA Produktion in mit ESAT-6/CFP-10 stimulierten versus nicht stimulierten Zellkulturen. Unspezifisch mit Expander-Beads aktivierte und spezifisch voraktivierte ESAT-6/CFP-10-spezifische Th-Zellen wurden mit autologen PBMC in An- und Abwesenheit von 10 μg/ml ESAT-6/CFP10 kokultiviert, wobei unterschiedliche Zahlen von T-Zellen mit $1 \times 10^6$ frisch isolierten PBMC eingesetzt wurden. Zu den angegebenen Zeitpunkten wurden jeweils $2 \times 10^5$ Zellen entnommen, pelletiert und der relative Gehalt an 4-1BBL mRNA wurde mittels RT-qPCR quantifiziert. Die Daten der RT-qPCR wurden nach der $2^{-\Delta\Delta Cq}$ Methode ausgewertet, wobei die GAPDH als Referenzgen und die unstimulierte Kontrolle als Kalibrator verwendet wurden.

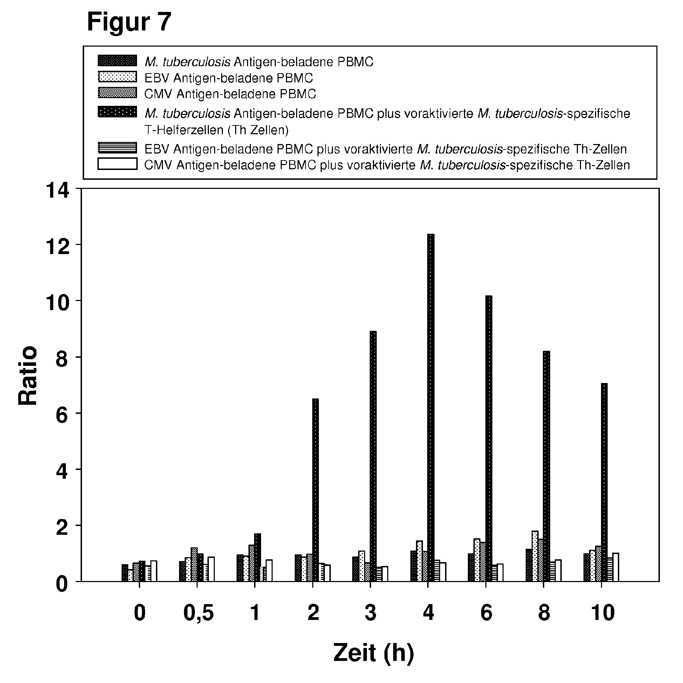

Figur 7 zeigt in einem weiteren Diagramm die Antigen-Spezifität der Induktion der 4-1BBL mRNA Synthese durch aktivierte Th-Zellen ermittelt mittels der RT-qPCR Technik. Voraktivierte ESAT-6/CFP-10-spezifische T-Zellen induzieren nur in mit ESAT-6/CFP-10 stimulierten Zellkulturen, nicht aber in mit EBV BZLF1 oder CMV pp65 Protein stimulierten Zellkulturen eine gesteigerte Induktion der 4-1BBL mRNA Produktion. $1 \times 10^6$ PBMC eines Spenders mit einer persistierenden *M. tuberculosis* Infektion wurden mit ca. 70000 voraktivierten ESAT-6/CFP-10-spezifischen Th-Zellen in Anwesenheit von 10 μg/ml ESAT-6/CFP-10, EBV BZLF1 oder CMV pp65 kokultiviert. Als weitere Kontrolle dienten unstimulierte Kokulturen voraktivierter T-Zellen und PBMC. Zu den angegebenen Zeitpunkten wurden dem Ansatz jeweils $2 \times 10^5$ Zellen entnommen und der relative Gehalt an 4-1BBL mRNA in einer RT-qPCR quantifiziert. Die Daten der RT-qPCR wurden nach der $2^{-\Delta\Delta Cq}$ Methode ausgewertet, wobei die GAPDH als Referenzgen und die unstimulierte Kontrolle als Kalibrator verwendet wurden.

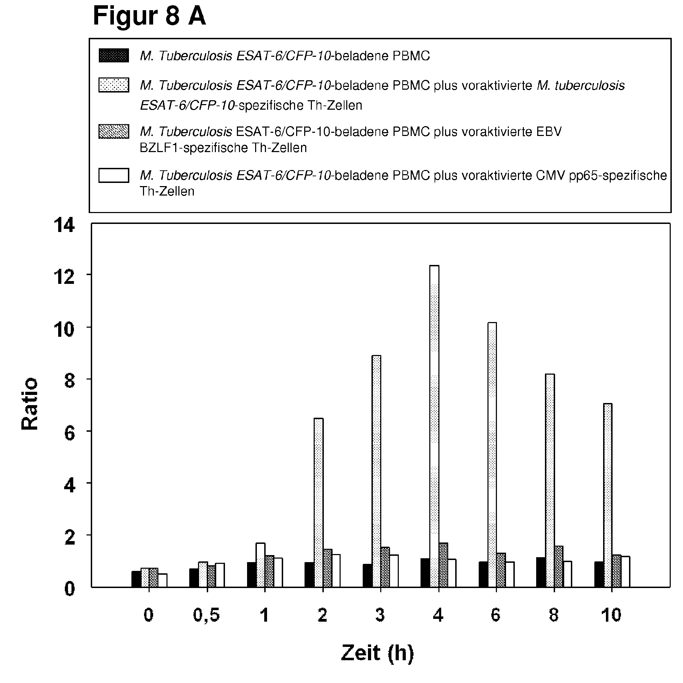

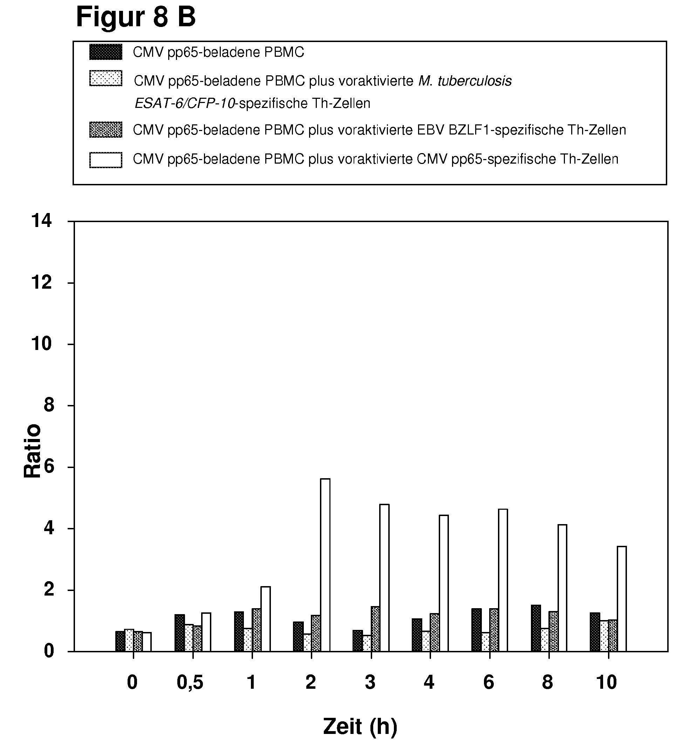

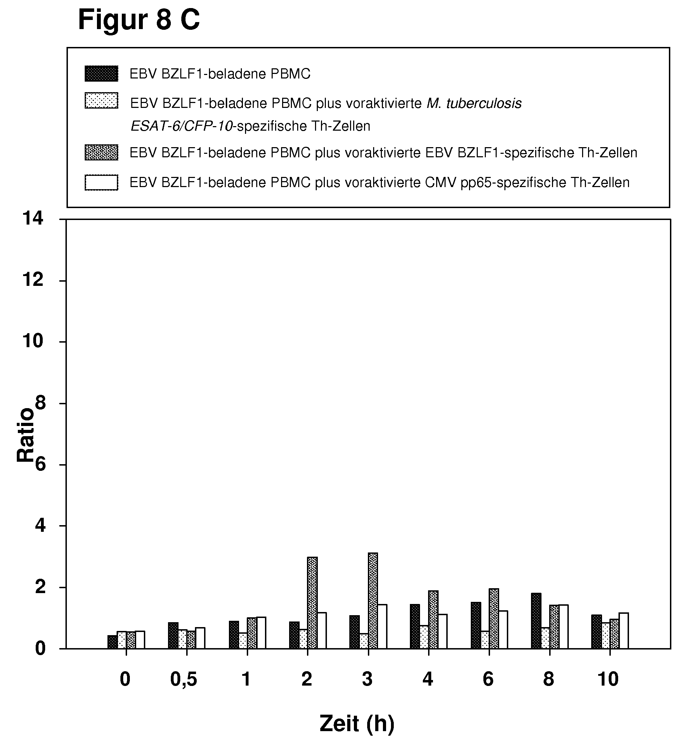

Figur 8 zeigt in einem weiteren Diagramm die Antigen-Spezifität der Induktion der 4-1BBL mRNA Synthese durch aktivierte Th-Zellen ermittelt mittels der RT-qPCR Technik. Voraktivierte ESAT-6/CFP-10-, EBV BZLF1- und CMV pp65-spezifische Th-Zellen zeigen nur in mit ihrem jeweiligen Zielantigen stimulierten Kokulturen eine gesteigerte Induktion der 4-1BBL mRNA Produktion. (A) bis (C) zeigen Diagramme zur Induktion der 4-1BBL mRNA Synthese durch CMV pp65, EBV BZLF1 und *M. tuberculosis* ESAT-6/CFP-10-spezifische Th-Zellen in (A) mit *M. tuberculosis* ESAT-6/CFP-10, (B) CMV pp65 und (C) EBV BZLF1-beladenen PBMC ermittelt durch RT-qPCR. Je $1 \times 10^6$/ml PBMC wurden mit ca. 070000 *ex vivo* expandierten pp65, BZLF1 und ESAT-6/CFP-10 spezifischen Th-Zellen in Anwesenheit und als Kontrolle in Abwesenheit von jeweils 10μg/ml ESAT-6/CFP-10, (B) pp65 oder (C) BZLF1 kokultiviert. Zu den angegebenen Zeitpunkten wurden dem Ansatz $2 \times 10^5$ Zellen entnommen die Gesamt-RNA isoliert und in cDNA umgeschrieben und der relative Gehalt an 4-1BBL mRNA mittels einer RT-qPCR quantifiziert. Ausgewertet wurde nach der $2^{-\Delta\Delta Cq}$ Methode unter Verwendung von GAPDH als Referenzgen und der unstimulierten Kontrolle als Kalibrator.

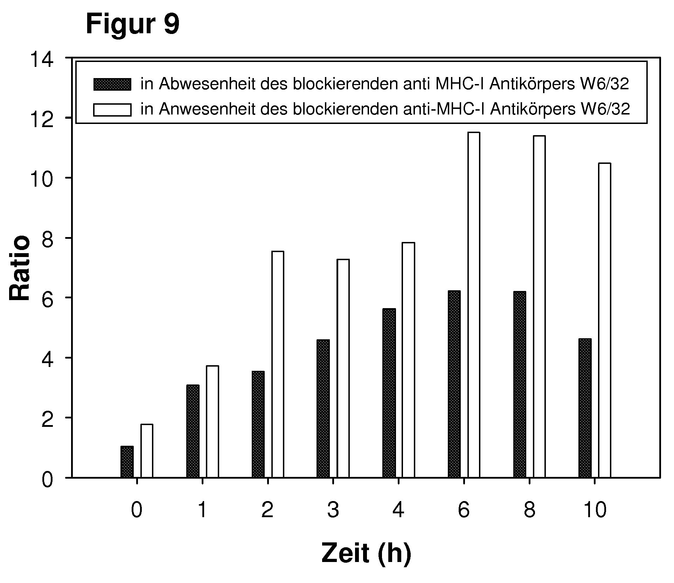

Figur 9 zeigt in einem Diagramm die Steigerung der Sensitivität des erfindungsgemäßen RTT Verfahrens durch den Einsatz eines MHC-I blockierenden Antikörpers (W6/32), ermittelt durch RT-qPCR. Mit 10 μg/ml EBV BZLF1 beladene und unbeladene PBMC wurden mit *in vitro* voraktivierten BZLF1-spezifischen Th-Zellen in An- und Abwesenheit von 10 μg/ml des MHC-I blockierenden Antikörpers W6/32 kokultiviert. Zu den angegebenen Zeitpunkten wurden $2 \times 10^5$ Zellen entnommen und die RNA aufgereinigt und mittels der RT-qPCR Technologie analysiert. Die Daten der RT-qPCR wurden nach der $2^{-\Delta\Delta cq}$ Methode ausgewertet, wobei die GAPDH als Referenzgen verwendet wurde.

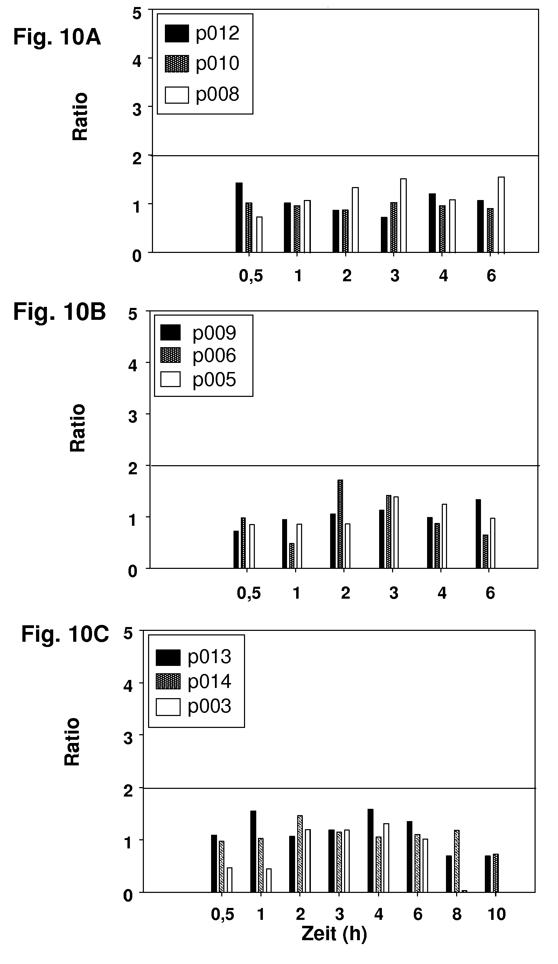

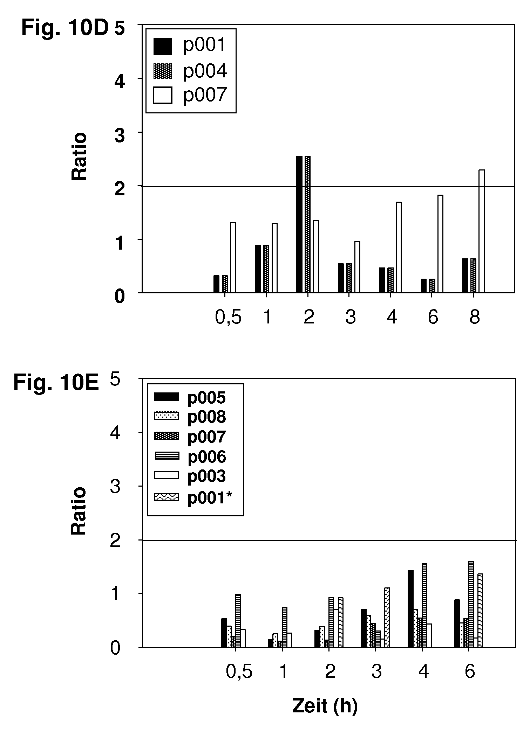

Figur 10 zeigt in (A) bis (E) in Diagrammen, dass Spender mit einer (D) aktiven Tuberkulose im Vergleich zu Spendern mit (B) einer latenten *M. tuberculosis* Infektion, (C) einer behandelten TB Infektion, sowie im Vergleich zu (A) gesunden Spendern eine messbar gesteigerte relative Induktion der 4-1BBL mRNA Produktion in spezifisch stimulierten PBMC verglichen mit unstimulierten PBMC aufweisen. Aus frisch isoliertem, heparinisierten Vollblut dreier (A) nicht mit *M. tuberculosis* infizierter gesunder Spender (p012, p010, p008), (B) gesunder Spender mit einer latenten Tuberkulose Infektion (p009, p006, p005), (C) Spender, die aufgrund einer aktiven Tuberkulose in den letzten 6 Monaten vor der Untersuchung medikamentös behandelt wurden (p013, p014, p003) und (D) Spender mit aktiver Tuberkulose vor oder kurz nach Beginn einer kausalen Therapie (p001, p004, p007) wurden PBMC isoliert und in An- oder Abwesenheit von 10 μg/ml ESAT-6/CFP-10 inkubiert. (E) Zur Kontrole wurden PBMC ausgewählter Spender HIV seronegativer Spender mit 10 μg/ml HIV p24 Kapsidprotein (p005, p008, p007, p006, p003) oder Rinderserum Albumin (p001) stimuliert. Zu den angegebenen Zeitpunkten wurden Zellen geerntet und die Expression von 4-1BBL in stimulierten und nicht-stimulierten Zellen mittels RT-qPCR analysiert. Die Ergebnisse wurden mit der $2^{-\Delta\Delta Cq}$ Methode ausgewertet. Dabei diente GAPDH als Referenzgen.

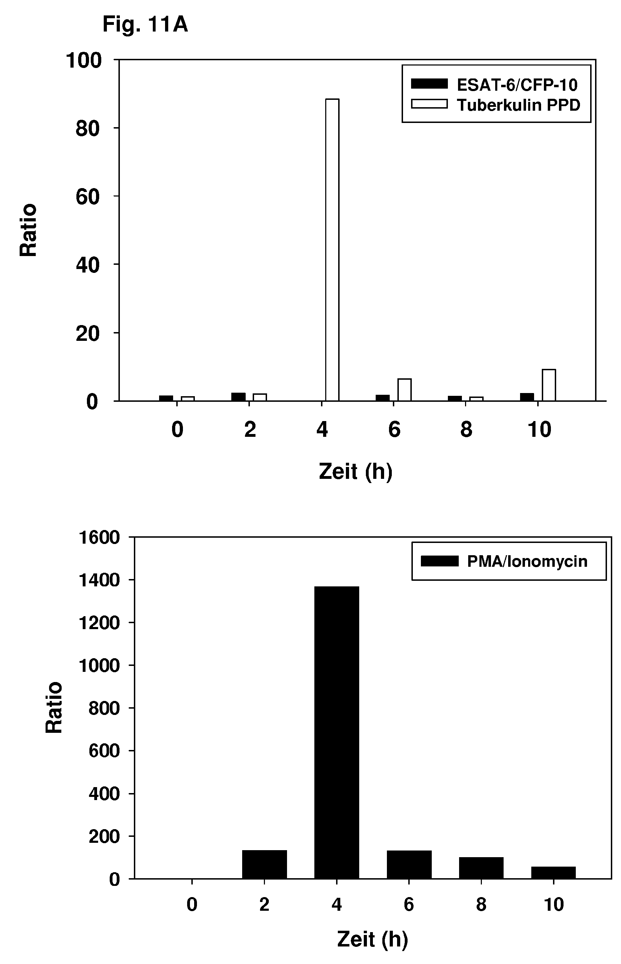

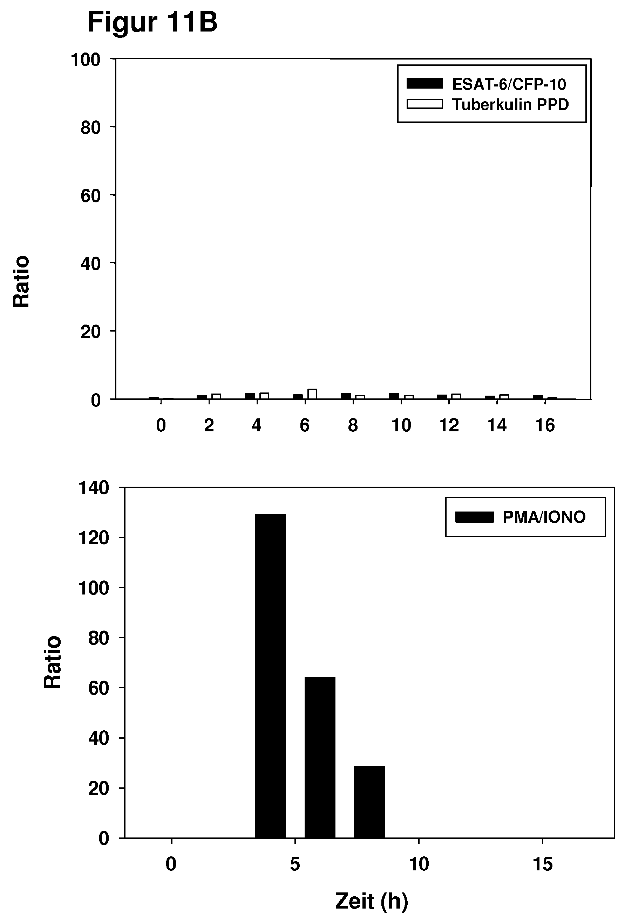

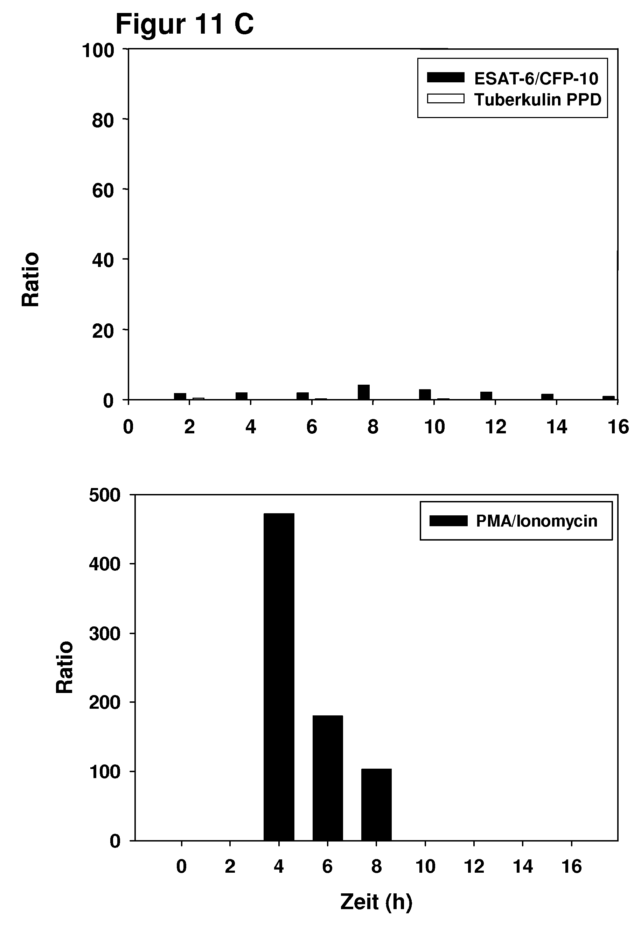

Figur 11 zeigt in (A) bis (C) in Diagrammen, dass mit Tuberkulin PPD stimulierte PBMC eines Spenders mit einer (A) aktiven Tuberkulose im Vergleich zu PBMC eines Spenders mit (B) einer latenten *M. tuberculosis* Infektion sowie (C) eines gesunden Spenders eine gesteigerte relative Induktion der 4-1BBL mRNA Produktion in spezifisch stimulierten PBMC verglichen mit unstimulierten PBMC aufweisen. 1 x $10^6$ frisch isolierte PBMC/ml von Spendern mit einer (A) aktiven TB, einer (B) latenten TB und (C) eines nicht mit M. *tuberculosis* infizierten gesunden Spenders wurden mit 10 μg/ml ESAT-6/CFP-10 bzw. Tuberkulin PPD oder - als Positivkontrolle - mit je 1 μg/ml PMA/Ionomycin stimuliert. Zu den angegebenen Zeitpunkten wurden $0{,}5 \times 10^6$ Zellen entnommen und bei -80°C aufbewahrt. Aus den Zellen wurde die Gesamt-RNA isoliert und in cDNA umgeschrieben und der Gehalt an 4-1BBL mRNA mittels einer RT-qPCR bestimmt. Ausgewertet wurde nach der $2^{-\Delta\Delta Cq}$ Methode unter Verwendung von GAPDH als Referenzgen und der unstimulierten Kontrolle als Kalibrator.

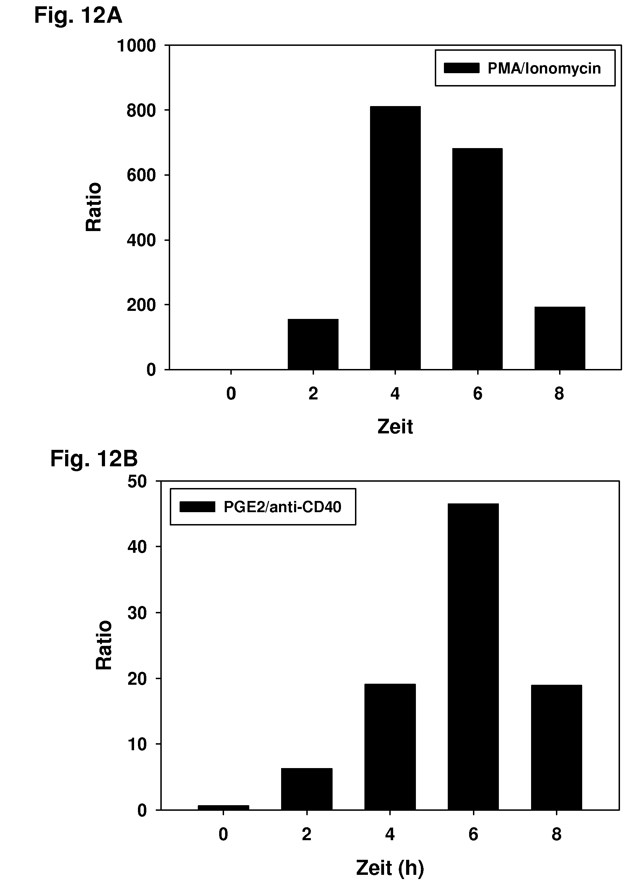

Figur 12 zeigt in (A) und (B) in Diagrammen die unspezifische Induktion der 4-1BBL mRNA Produktion in PBMC eines gesunden Probanden nach Stimulation mit je 1 μg/ml PMA/Ionomycin in (A), oder PGE2/α-CD40 in (B), im Vergleich zur unstimulierten Kontrolle, ermittelt durch RT-qPCR. Frisch isolierte PBMC eines gesunden Probanden wurden mit je 1 μg/ml PMA/Ionomycin in (A) oder mit 5 μg/mL PGE$_2$ und 2 μg/mL α-CD40 (B) inkubiert. Zu den angegebenen Zeitpunkten wurden $0{,}5 \times 10^6$ Zellen herausgenommen, pelletiert und bis zur weiteren Verarbeitung bei -80°C aufbewahrt. Aus den Zellen wurde die Gesamt-RNA isoliert und in cDNA umgeschrieben. Anschließend wurde in einer RT-qPCR der Gehalt der 4-1BBL cDNA bestimmt. Ausgewertet wurde nach der $2^{-\Delta\Delta Cq}$ Methode unter Verwendung von GAPDH als Referenzgen und der unstimulierten Kontrolle als Kalibrator.

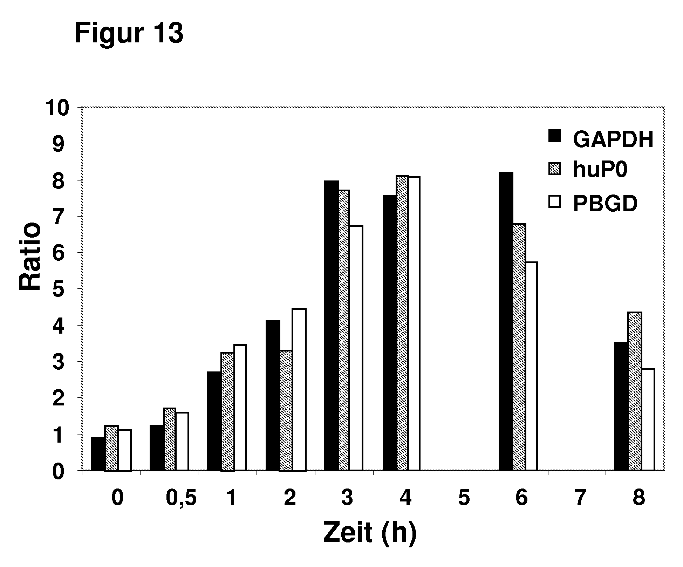

Figur 13 zeigt in einem Diagramm den Einfluss unterschiedlicher Referenzgene auf den ermittelten relativen Anstieg der 4-1BBL mRNA Produktion in ESAT-6/CFP-10 stimulierten versus unstimulierten Kokulturen ESAT-6/CFP-10-spezifischer aktivierter T-Zellen und autologer PBMC eines Spenders mit persistierender Tuberkulose ermittelt durch RT-qPCR. Frisch isolierte PBMC eines Spenders mit einer persistierenden Tuberkulose wurden mit voraktivierten ESAT-6/CFP-10-spezifischen Th-Zellen in An- und Abwesenheit von 10 μg/ml ESAT-6/CFP-10 Protein inkubiert. Zu den angegebenen Zeitpunkten wurden $0{,}5 \times 10^6$ Zellen entnommen, pelletiert und bis zur weiteren Verarbeitung bei -80°C aufbewahrt. Aus den Zellen wurde die Gesamt-RNA isoliert und in cDNA umgeschrieben. Anschließend wurde in einer RT-qPCR der Gehalt der 4-1BBL cDNA bestimmt. Ausgewertet wurde nach der $2^{-\Delta\Delta Cq}$ Methode unter Verwendung von GAPDH, huP0 und PBGD als Referenzgene und der unstimulierten Kontrolle als Kalibrator.

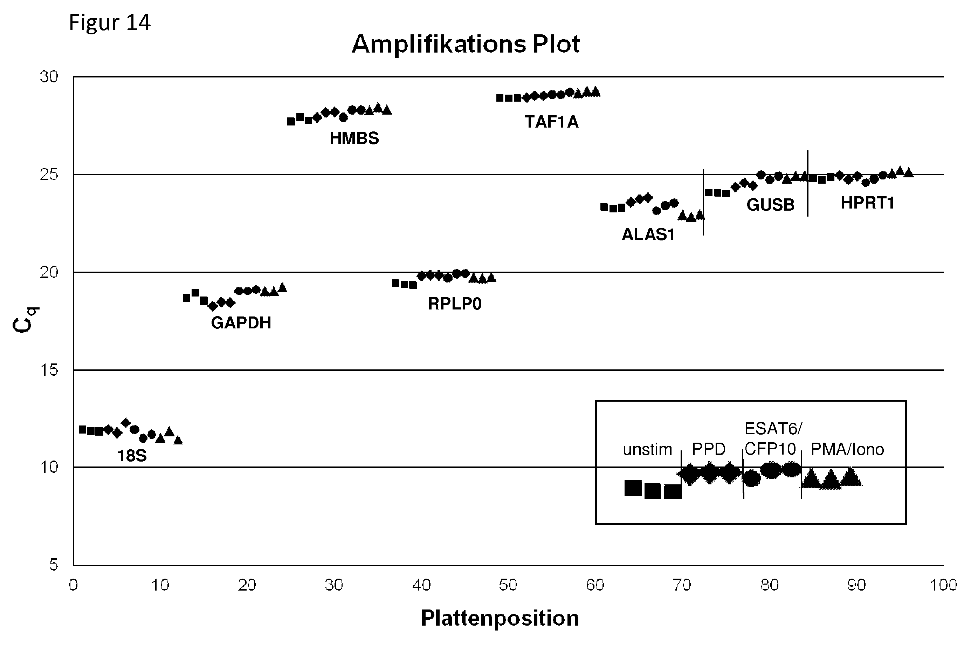

Figur 14 zeigt einen Amplifikationsplot einer Real Time PCR für die Identifizierung eines geeigneten Referenzgens für das RTT Verfahren. Auf der y-Achse sind die Quantifizierungszyklen (Cq) für 7 potentielle Referenzgene unter vier Stimulationsbedingungen (unstimuliert, Tuberkulin (PPD), ESAT6/CFP10, PMA/Ionomycin) in Triplikaten beispielhaft für eine Probe dargestellt. Der vergrößerte Ausschnitt zeigt die Reihenfolge der Stimulationsbedingungen für die jeweils 12 Datenpunkte. Auf der x-Achse ist die Plattenposition auf der 96-Well Platte angegeben. 18S: eukaryotische 18S ribosomale RNA; ALAS: aminolevulinate, delta-, synthase 1; GAPDH: glyceraldehyde-3-phosphate dehydrogenase; GUSB: glucuronidase, beta; HMBS: hydroxymethylbilane synthase; HPRT1: hypoxanthine phosphoribosyltransferase 1; Iono: Ionomycin; PMA: Phorbol-12-myristat-13-acetat; PPD: Tuberkulin PPD; RPLP0: ribosomal protein, large, P0; TAF1A: TATA box binding protein (TBP)-associated factor, RNA polymerase I, A, 48kDa; unstim: nicht stimuliert.

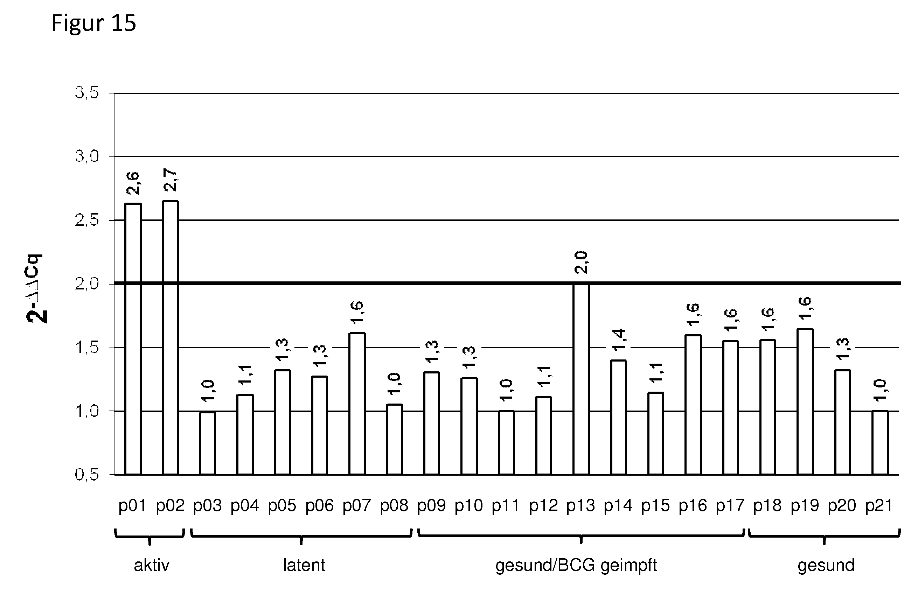

Figur 15 zeigt die Zunahme der relativen Expression des RTT Markergens *4-1BBL* -auch *TNFSF9* genannt- in Proben von aktiv oder latent infizierten TB Patienten sowie gesunden BCG geimpften oder gesunden nicht geimpften Probanden nach Stimulation mit Tuberkulin PPD im Vergleich mit den entsprechenden unstimulierten Proben. Die Werte wurden gegen das Referenzgen *TAF1A* normalisiert ($2^{-\Delta\Delta Cq}$). Je Ansatz wurden $5 \times 10^6$ PBMC in B-Zellmedium mit 10 μg/ml Tuberkulin PPD stimuliert. Aktiv: Patienten mit einer aktiven Tuberkulose; latent: Patienten mit einer latenten Tuberkulose; BCG: Bacillus Calmette-Guerin, abgeschwächter Lebendimpfstoff gegen Tuberkulose.

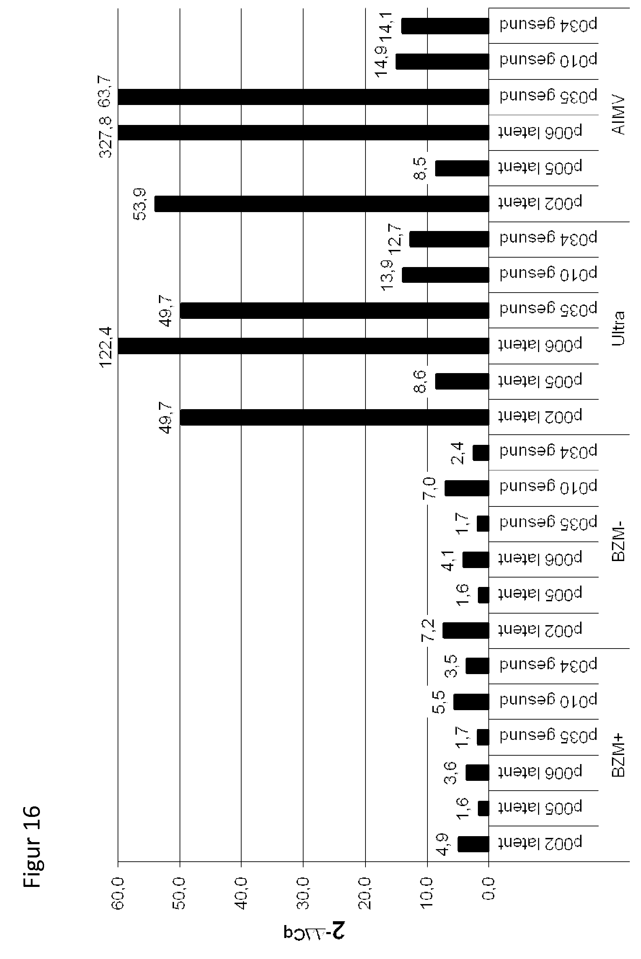

Figur 16 zeigt den Einfluss unterschiedlicher Zellkulturmedien auf den relativen Anstieg des Expressionssignals von *IFN-γ* -auch *IFNG* genannt - in PBMC von Patienten mit einer latenten TB und gesunden Probanden nach Stimulation mit Tuberkulin PPD im Vergleich zu unstimulierten Proben und Normalisierung gegen *TAF1A*. Verwendet wurden B-Zellmedium (BZM+), B-Zellmedium ohne IL-4 (BZM-), serumfreies UltraCULTURE™ Medium (Ultra) von LONZA und AIM V Medium (AIMV) von Invitrogen.

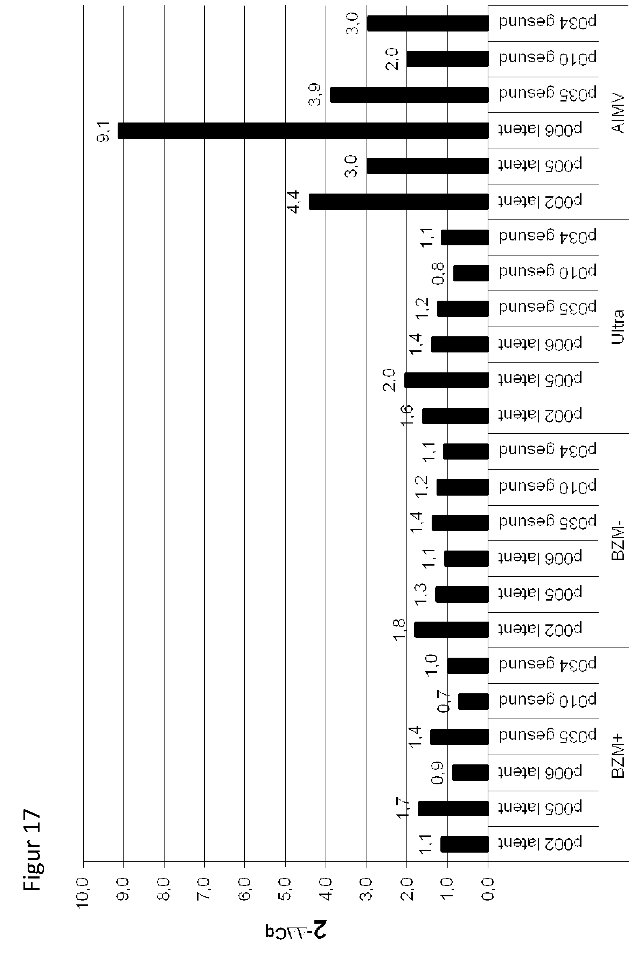

Figur 17 zeigt den Einfluss unterschiedlicher Zellkulturmedien auf den realtiven Anstieg des Expressionssignals von *4-1BBL* -auch *TNFSF9* genannt- in PBMC von Patienten mit einer latenten TB und gesunden Probanden nach Stimulation mit Tuberkulin PPD im Vergleich zu unstimulierten Proben und Normalisierung gegen *TAF1A*. Verwendet wurden B-Zellmedium (BZM+), B-Zellmedium ohne IL-4 (BZM-), serumfreies UltraCULTURE™ Medium (Ultra) von LONZA und AIM V Medium (AIMV) von Invitrogen.

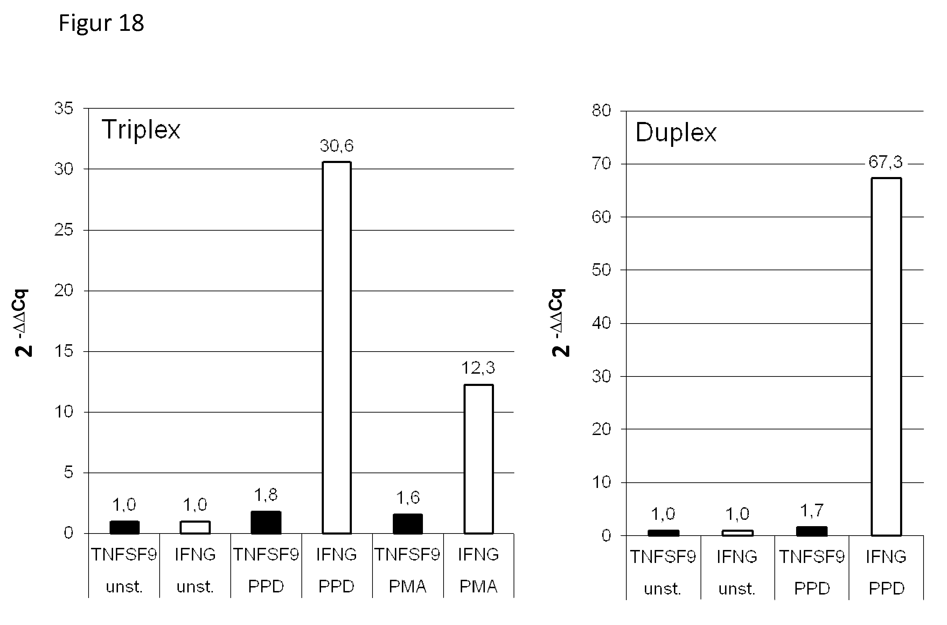

Figur 18 zeigt die Ergebnisse für den zeitgleichen Nachweis der relativen Expressionszunahmen von *TNFSF9* und *IFNG* mit Normalisierung gegen *TAF1A* als Referenzgen in einem Triplex qPCR Ansatz (links) im Vergleich zu den entsprechenden Duplex Reaktionen (rechts). Unst.: nicht stimuliert; PPD: Tuberkulin PPD; PMA: Phorbol-12-Myristat-13-Acetat/Ionomycin.

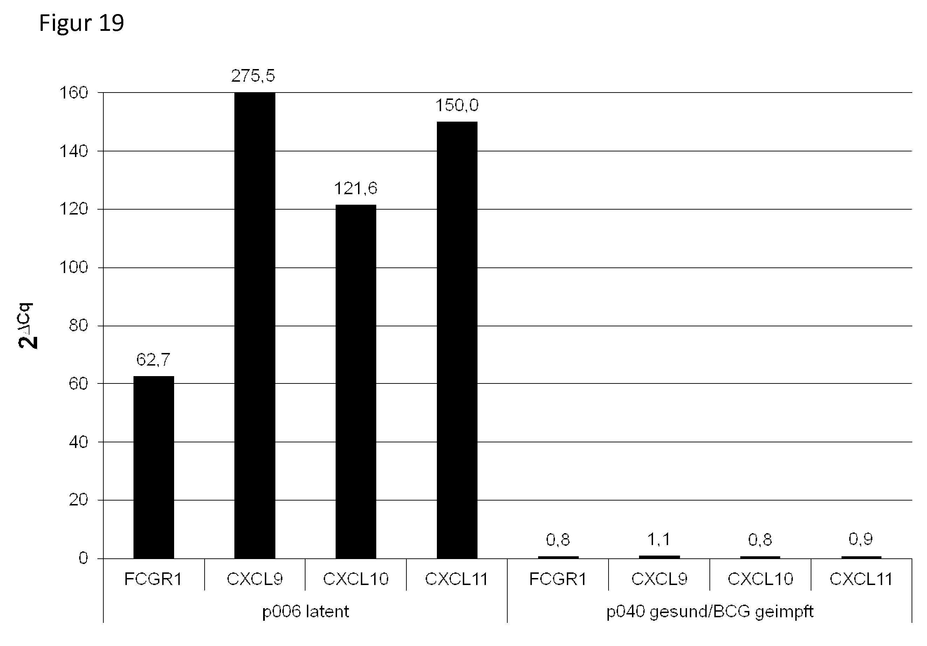

Figur 19 zeigt die relative Expressionszunahme der Gene *FCGR1ABC, CXCL9, CXCL10, CXCL11* in Proben von einem gesunden Probanden und von einem Patienten mit einer latenten TB nach Stimulation mit Tuberkulin PPD im Vergleich zu den entsprechenden unstimulierten Proben. Die Real Time qPCR wurde mit dem SYBR Green System durchgeführt. Auf der x-Achse sind die Gene bzw. Probanden aufgetragen. Die y-Achse zeigt die relative Expressionszunahme ($2^{\Delta Cq}$). BCG: Bacillus Calmette-Guerin, abgeschwächter Lebendimpfstoff gegen Tuberkulose; *CXCL9* bis *11*: chemokine (C-X-C motif) ligand 9 bis 11; *FCGR1ABC:* Fc gamma Rezeptor 1 A, B, C.

Im Zusammenhang mit der vorliegenden Erfindung wird unter einer "T-Zellpopulation" eine definierte Gruppe von T-Zellen mit einem bestimmten Phänotyp verstanden. Erfindungsgemäße T-Zellpopulationen sind insbesondere naive T-Zellen, aktivierte T-Zellen und Gedächtnis-T-Zellen.

Im Zusammenhang mit der vorliegenden Erfindung wird unter dem Ausdruck "T-Zellen" T-Lymphozyten, wie CD4$^+$ T-Zellen oder CD8$^+$ T-Zellen bzw. ein Gemisch von CD4$^+$ T-Zellen, und CD8$^+$ T-Zellen verstanden. Hierbei umfasst die Gruppe der CD4$^+$ T-Zellen, T-Helferzellen, beispielsweise T-Helfer 1 (Th-1) Zellen, T-Helfer 2 (Th-2) Zellen, T-Helfer 17 (Th-17) Zellen, CD4$^+$CD25$^+$ regulatorische T-Zellen (Treg), Tr-1 Zellen und T-Helfer 3 (Th-3) Zellen. Die Gruppe der CD8$^+$ T-Zellen umfasst CD4-CD8$^+$ zytotoxische T-Zellen und T-Zellen, die einen CD4$^+$CD8$^+$ Phänotyp (CD4$^+$CD8$^{dim}$, CD4$^{dim}$CD8$^{bright}$ oder CD4$^{hi}$CD8$^{hi}$) aufweisen.

Im Zusammenhang mit der vorliegenden Erfindung wird unter einer "naiven T-Zelle" eine T-Zelle verstanden, die eine bestimmte Antigenspezifität aufweist, jedoch noch keinen ersten Kontakt mit ihrem Antigen hatte. Naive T-Zellen wie naive CD4$^+$ T-Zellen weisen z.B. CD45RA als Marker auf ihrer Oberfläche auf.

Im Zusammenhang mit der vorliegenden Erfindung wird unter einer "aktivierten T-Zelle" eine T-Zelle verstanden, die ausgehend von einem naiven Zustand vor einem ersten Antigenkontakt durch den ersten Antigenkontakt des T-Zellrezeptors stimuliert wird. Die Erkennung eines auf MHC Molekülen präsentierten Peptids durch den T-Zellrezeptor führt zu einem sogenannten Crosslinking (Quervernetzung) des T-Zellrezeptors. Eine weitere Voraussetzung für die Aktivierung von T-Zellen ist ein zweites Signal, das durch die Interaktion von kostimulatorischen Molekülen und ihren Liganden sowohl auf den APC als auch auf den T-Zellen vermittelt wird. Diese Aktivierung führt zu einer Signalkaskade innerhalb der T-Zellen und schließlich zu einer Proliferation und der Ausprägung verschiedener Effektorfunktionen. Zudem sind aktivierte T-Zellen durch die transiente Expression charakteristischer Oberflächenmoleküle, wie beispielsweise dem CD40 Liganden, 4-1BB, CD69 und/oder CD25 gekennzeichnet. Die Aktivierung von naiven T-Zellen zu aktivierten T-Zellen nach dem ersten Antigenkontakt auf der Oberfläche einer professionellen antigenpräsentierenden Zelle wird auch als "Priming" bezeichnet. Der Begriff "aktivierte T-Zelle" ist synonym zu dem Begriff "Effektor-T-Zelle" und umfasst auch spezifisch *in vivo* durch einen erneuten Kontakt mit ihrem Antigen reaktivierte Gedächtnis-T-Zellen.

Im Zusammenhang mit der vorliegenden Erfindung wird unter einer "Gedächtnis-T-Zelle" eine T-Zelle verstanden, die einen spezifischen Antigenkontakt bereits hatte. Gedächtnis-T-Zellen sind gekennzeichnet durch spezielle Oberflächenmarker, wie CD45RO, CD44 und L-Selektin.

Im Zusammenhang mit der vorliegenden Erfindung werden unter dem Begriff "antigenpräsentierende Zelle" (APC) Zellen verstanden, die in der Lage sind Polypeptide aufzunehmen, zu prozessieren und Bruchstücke dieser Polypeptide, so genannte Epitope dem Immunsystem in Verbindung mit MHC I und MHC II Proteinen zu präsentieren. Insbesondere umfasst der Begriff "Antigenpräsentierende Zelle" sogenannte professionelle antigenpräsentierende Zellen, wie dendritische Zellen, Monozyten, Makrophagen, nicht professionelle APC, wie B-Zellen, aber auch vaskuläre endotheliale Zellen, Fibroblasten der Haut, Epithelzellen des Thymus und der Schilddrüse, Gliazellen des Gehirns, Betazellen der Bauchspeicheldrüse sowie vaskuläre Endothelzellen. Nicht professionelle APC exprimieren die für die Interaktion mit T-Zellen erforderlichen MHC Moleküle der Klassen-1 und -II erst infolge einer Aktivierung durch Zytokine wie IFN-□. Zudem können auch T-Zellen als APC fungieren. Diese APC-T-Zellen entstehen durch den interzellulären Transfer von MHC Klasse-I und -II Molekülen sowie von kostimulatorischen Molekülen, z.B. CD80, CD40 Ligand (CD40L), OX40 Ligand (OX40L) und 4-1BB Ligand (4-1BBL, TNFSF9) infolge eines Kontakts mit einer APC, insbesondere einer dendritischen Zelle (DC).

Im Zusammenhang mit der vorliegenden Erfindung wird unter einem "Marker der APC" bzw. unter einem "Marker der T-Zelle" eine Nukleinsäure, insbesondere eine RNA oder eine DNA, oder ein Nukleinsäurefragment verstanden. Erfindungsgemäß ist vorgesehen, dass der Marker nach einer spezifischen Erkennung des mit MHC Molekülen komplexierten Antigens auf der APC durch eine aktivierte T-Zelle in seiner Expression in der APC messbar gesteigert oder reduziert wird. Erfindungsgemäß ist somit vorgesehen, dass der Marker der APC durch die Antigen-spezifische Interaktion der aktivierten T-Zellen mit der APC induziert und dadurch nachweisbar und quantifizierbar wird.

Im Zusammenhang mit der vorliegenden Erfindung wird unter "induziert" bzw. "Induktion" eines Markers die Veränderung der Expression eines Markers verstanden. Im Falle, dass der Marker eine Nukleinsäure ist, wird erfindungsgemäß unter "Induktion" die gesteigerte oder verminderte Produktion beispielsweise von mRNA eines Gens verstanden. Unter "Induktion" wird außerdem erfindungsgemäß die Modulation eines Gens verstanden, beispielsweise in Form einer Methylierung. Weiterhin wird im Rahmen der vorliegenden Erfindung unter "induziert" die gesteigerte oder die verminderte Expression eines Proteins verstanden. Die Expression des Proteins kann hierbei sowohl auf der Zelle als auch in der Zelle, also intrazellulär, erfolgen.

Im Zusammenhang mit der vorliegenden Erfindung wird unter einem "Antigen" insbesondere ein Protein, ein Polypeptid oder ein Peptid verstanden. Ein Antigen ist insbesondere eine Polypeptidsequenz, die von APC aufgenommen, prozessiert und dessen Bruchstücke, so genannte Epitope, auf MHC-Molekülen T-Zellen präsentiert werden. Ein Antigen ist insbesondere auch ein Peptid, das in Verbindung mit MHC Molekülen T-Zellen präsentiert wird. Darüber hinaus wird unter einem Antigen auch eine RNA, DNA oder ein Expressionsplasmid verstanden, das für ein Polypeptid kodiert.

Der hier verwendete Begriff "Expressionsplasmid" oder "Expressionsvektor" bezeichnet künstlich erschaffene Konstrukte zum Einschleusen und zur Expression von Nukleinsäuren in Zellen. Expressionsvektoren sind beispielsweise bakterielle Plasmide und MIDGES, von Viren abgeleitete Plasmide, Phagemide, Cosmide, Bakteriophagen oder künstlich hergestellte Nukleinsäuren wie artifizielle Chromosomen. Vektoren können zudem einen oder mehrere Selektionsmarker enthalten.

Im Zusammenhang mit der vorliegenden Erfindung wird unter dem Ausdruck "Polypeptid" ein Polymer von Aminosäuren beliebiger Länge verstanden. Der Ausdruck "Polypeptid" umfasst auch die Begriffe Zielepitop, Epitop, Peptid, Oligopeptid, Protein, Polyprotein und Aggregat von Polypeptiden. Weiterhin werden unter dem Ausdruck "Polypeptid" auch Polypeptide verstanden, die posttranslationale Modifikationen, wie Glykosylierungen, Acetylierungen, Phosphorylierungen, Carbamoylierungen und ähnliche Modifikationen aufweisen. Darüber hinaus werden unter dem Ausdruck "Polypeptid" auch Polypeptide verstanden, die ein oder mehrere Analoge von Aminosäuren, wie beispielsweise unnatürliche Aminosäuren, Polypeptide mit substituierten Verknüpfungen sowie andere Modifikationen, wie sie im Stand der Technik bekannt sind, aufweisen, unabhängig davon, ob sie natürlicher Weise vorkommen oder nicht natürlichen Ursprungs sind.

Der hier verwendete Begriff "Epitop" bezeichnet den Bereich eines Polypeptides, das antigene Eigenschaften besitzt und beispielsweise als Erkennungsstelle von T-Zellen oder Immunglobulinen dient. Im Sinne dieser Erfindung sind Epitope beispielsweise solche Bereiche von Polypeptiden, die von Immunzellen wie z.B. CD4+ T-Helferzellen, CD8+ zytotoxischen T-Zellen, CD4+CD8dim zytotoxische T-Zellen, CD56+CD8+ sowie CD56-CD57+CD8+ NKT-Zellen oder CD4+CD25+ regulatorischen T-Zellen erkannt werden. Ein Epitop kann 3 oder mehr Aminosäuren umfassen. Üblicherweise besteht ein Epitop aus mindestens 5 bis 7 Aminosäuren oder, was häufiger ist, aus 8 bis 11 Aminosäuren, oder aber aus mehr als 11 Aminosäuren, oder aber aus mehr als 20 Aminosäuren, seltener sogar aus mehr als 30 Aminosäuren.

Im Zusammenhang mit der vorliegenden Erfindung wird unter "Reverse Transkription quantitative Real-Time Polymerase Kettenreaktion, RT-qPCR" ein Verfahren verstanden, das auf einer herkömmlichen Polymerase-Kettenreaktion (PCR) basiert. Darüber hinaus ermöglicht die RT-qPCR neben einer Vervielfältigung zusätzlich auch eine Quantifizierung der Ziel-mRNA. Hierfür wird aus zu untersuchendem und mit einem Antigen inkubiertem Material und zum Vergleich mit unstimuliertem oder einem irrelevanten Antigen inkubiertem Material die Gesamt-RNA isoliert und anschließend in einer Reversen Transkriptionsreaktion in cDNA umgeschrieben. Unter Verwendung spezifischer Primer wird anschließend in der qPCR die Zielsequenz vervielfältigt. Zur Quantifizierung der Zielsequenz können mehrere Verfahren angewendet werden.

Die einfachste Art der Quantifizierung ist über die Verwendung interkalierender Fluoreszenzfarbstoffe, wie SYBR-Green oder EVA-Green. Diese Farbstoffe lagern sich in doppelsträngigen DNA-Molekülen an, die während der Elongation der spezifischen Produkte entstehen. Die Detektion findet immer am Ende des Elongationsschrittes über die Detektion des emittierten Lichts nach Anregung des Fluoreszenzfarbstoffes statt. Mit zunehmender Menge an PCR-Produkt wird mehr Farbstoff eingelagert, sodass das Fluoreszenzsignal ansteigt.

Eine andere Art der Quantifizierung stellt die Verwendung Sequenz-spezifischer Sonden dar. Es gibt Hydrolyse-(TaqMan) oder Hybridisierungs- (Light-Cycler) Sonden. Hydrolysesonden sind am 5'-Ende mit einem Floureszenzfarbstoff und am 3'-Ende mit einem sogenannten Quencher markiert. Der Quencher ist aufgrund der räumlichen Nähe zum Reporterfarbstoff für die Unterdrückung dessen Fluoreszenzsignals verantwortlich und wird bei der Synthetisierung der Komplementär-DNA während der Elongationsphase abgespalten. Sobald dann am Ende der Elongation der Fluoreszenzfarbstoff über eine Lichtquelle angeregt wird, wird Licht einer bestimmten Wellenlänge frei, das detektiert werden kann.

Hybridisierungssondensysteme bestehen aus zwei Sonden, die nebeneinander an der Zielsequenz binden. Beide Sonden sind mit einem Fluoreszenzfarbstoff markiert. Mittels einer Lichtquelle wird der erste Fluoreszenzfarbstoff am 5'-Ende der ersten Sonde angeregt. Das emittierte Licht wird dann durch Fluoreszenzresonanzenergietransfer (FRET) auf den zweiten Fluoreszenzfarbstoff auf dem 3'-Ende der zweiten Sonde übertragen. Damit wird der Farbstoff angeregt, wodurch Licht einer bestimmten Wellenlänge emittiert wird, das detektiert werden kann. Wird im Laufe der Elongation des Komplementärstrangs der Zielsequenz die erste Sonde durch die Polymerase abgebaut, kann der FRET nicht mehr stattfinden und das Fluoreszenzsignal nimmt in der Folge ab. Die Quantifizierung findet hier deshalb im Gegensatz zu den vorher genannten Methoden immer am Anfang des Elongationsprozesses statt.

Häufig verwendete Fluoreszenzfarbstoffe sind z.B. Fluophor 1, Fluophor 2, Aminocumarin, Fluorescein, Cy3, Cy5, Europium, Terbium, Bodipy, Dansyl, Naphtalen, Ruthenium, Tetramethylrhodamin, 6-Carboxyfluorescein (6-FAM), VIC, YAK, Rhodamin und Texas Red. Häufig verwendete Quencher sind z.B. TAMRA™, 6-Caboxytetramethoylrhodamin, Methylrot oder Dark-Quencher.

Der Ausdruck "Real-Time" bezieht sich auf die konkrete Messung innerhalb jedes Zyklus einer PCR, also in "Echt-Zeit". Die Zunahme der so genannten Zielsequenz korreliert hierbei mit der Zunahme der Fluoreszenz von Zyklus zu Zyklus. Am Ende eines Laufes, der gewöhnlich aus mehreren Zyklen besteht, wird dann anhand der erhaltenen Fluoreszenzsignale die Quantifizierung in der exponentiellen Phase der PCR vorgenommen. Die Messung der Amplifikation erfolgt hierbei in der Regel über Cq (quantification cycle)-Werte, die den Zyklus beschreiben, an dem die Fluoreszenz erstmalig signifikant über die Hintergrund-Fluoreszenz ansteigt. Der Cq-Wert wird einerseits für die Zielnukleinsäure und andererseits für die Referenznukleinsäure ermittelt. Dadurch ist es möglich, absolute oder relative Kopienzahlen der Zielsequenz zu ermitteln.

Im Zusammenhang mit der vorliegenden Erfindung kann unter dem Ausdruck Referenzgen eine Sequenz sowohl auf der Ebene der mRNA als auch auf der Ebene der genomischen DNA verstanden werden. Diese können unter den erfindungsgemäßen Stimulationsbedingungen auch nicht transkriptionell aktiv sein oder nicht-kodierenden DNA-Bereichen des Genoms entsprechen. Ein Referenzgen kann erfindungsgemäß zudem auch eine zu der Zielgen-Probe hinzugefügte DNA oder RNA sein. Das höchste Kriterium eines Referenzgens ist, dass es im Verlauf der Stimulation und durch die Bedingungen des erfindungsgemäßen Verfahrens nicht verändert wird. Dadurch können die experimentellen Ergebnisse hinsichtlich der eingesetzten Template-Menge in unterschiedlichen Ansätzen normalisiert werden. Das Referenzgen ermöglicht somit die Bestimmung der relativen Expression eines Zielgens. Beispiele für Referenzgene sind unter anderem Glycerinaldhyd-3-Phosphat-Dehydrogenase (GAPDH), huP0 (human acidic protein 0), Porphobilinogen Deaminase (PBGD), $\beta$-Aktin oder Tubulin.

In einem ersten Gegenstand der vorliegenden Erfindung ist vorgesehen, ein Verfahren zum Nachweis, Differenzieren und Quantifizieren von T-Zellpopulationen bereitzustellen, das die folgenden Schritte umfasst a) in Kontakt Bringen eines ersten Aliquots einer Körperflüssigkeit eines Individuums mit mindestens einem Antigen, wobei die Körperflüssigkeit antigenpräsentierende Zellen (APC) und T-Zellen enthält, b) Inkubieren des ersten Aliquots mit dem mindestens einen Antigen über einen bestimmten Zeitraum, c) Nachweis und Differenzieren der T-Zellpopulationen durch Detektieren mindestens eines ersten durch die T-Zellen einer bestimmten T-Zellpopulation induzierten Markers der APC in dem ersten Aliquot und in einem zweiten Aliquot der Körperflüssigkeit des Individuums, das nicht mit dem mindestens einen Antigen inkubiert wurde, durch Reverse Transkription quantitative Real-Time Polymerase Kettenreaktion (RT-qPCR), und d) Nachweis und Quantifizieren der T-Zellpopulationen durch Bestimmen des Verhältnisses des detektierten Markers der APC des ersten Aliquots zu dem zweiten Aliquot.

Erfindungsgemäß ist somit vorgesehen, dass ein Nachweis, Differenzieren und Quantifizieren der T-Zellen einer bestimmten T-Zellpopulation durch einen Nachweis und eine relative Quantifizierung mindestens eines Markers der APC in nicht und/oder nicht spezifisch und spezifisch stimulierten Zellkulturansätzen durch die RT-qPCR durchgeführt wird. Das erfindungsgemäße Verfahren sieht vor, dass der Nachweis und die Quantifizierung dadurch ermöglicht wird, dass insbesondere aktivierte T-Zellen über einen Rückkopplungsmechanismus zur Reifung von antigenpräsentierenden Zellen (APC) beitragen können. Dieser Rückkopplungsmechanismus wird im Rahmen der vorliegenden Erfindung auch als Reverse T-Zell-Technologie (RTT) bezeichnet. So induzieren Antigen-spezifische aktivierte T-Zellen, insbesondere T-Helferzellen, vermittelt durch die Erkennung von mit einem Antigen-beladenen MHC Molekülen mittels des TCR und die gleichzeitige weitere Interaktion der T-Zelle mit der APC eine Aktivierung unter anderem beispielsweise von Promotoren verschiedener Gene als Marker der APC, die gesteigerte Produktion von mRNA Molekülen und die gesteigerte Expression dieser Markerproteine. Durch diese Reifungsprozesse erlangt die APC eine gesteigerte Fähigkeit zur Stimulation von erreger- bzw. krankheitsspezifischen zytotoxischen T-Zellen und niederaffinen T-Helferzellen.

Erfindungsgemäß ist somit vorgesehen, dass der Nachweis von T-Zellen einer bestimmten T-Zellpopulation, wie aktivierte T-Zellen, durch einen indirekten Nachweis über einen Marker der APC erfolgt. Ohne an eine Theorie gebunden zu sein ist erfindungsgemäß vorgesehen, dass APC durch die Inkubation mit einem Antigen Bruchstücke dieses Antigens in Verbindung mit MHC Klasse-II oder -I Molekülen auf ihrer Oberfläche aktivierten T-Zellen präsentieren. Dadurch kommt es zu einer spezifischen Bindung der aktivierten T-Zellen an die das Antigen präsentierenden APC. Durch diese spezifische T-Zell-APC-Interaktion wird die APC spezifisch stimuliert. Infolge dieser spezifischen Stimulierung kommt es zur Induktion eines Markers in den APC. Die Bestimmung des Markers in spezifisch stimulierten APC und in APC, die unstimuliert sind, durch Reverse Transkription quantitative PCR (RT-qPCR) ermöglicht den Nachweis, die Differenzierung und die Quantifizierung von aktivierten T-Zellen als bestimmte T-Zellpopulation.

Das erfindungsgemäße Verfahren ermöglicht einen sensitiven und verlässlichen Nachweis von T-Zellen einer bestimmten T-Zellpopulation, wie naive, aktivierte oder Gedächtnis-T-Zellen. Die Sensitivität des Verfahrens beruht insbesondere darauf, dass eine T-Zelle in der APC die Stimulation des Markers unter anderem in Form von einer Vielzahl von mRNA Kopien auslöst. Weiterhin wird diskutiert, dass durch eine T-Zelle unter Umständen eine größere Anzahl von APC stimuliert wird, indem eine T-Zelle sozusagen von einer APC zur nächsten "springt". Insgesamt führt dies zu einer Signalverstärkung und dadurch zu einer erhöhten Sensitivität des erfindungsgemäßen Verfahrens.

Das erfindungsgemäße Verfahren ermöglicht somit den Nachweis und das Differenzieren von bestimmten T-Zellpopulationen und erlaubt dadurch insbesondere eine Abgrenzung von aktivierten T-Zellen gegenüber Gedächtnis-T-Zellen.

In einer bevorzugten Ausführungsform der Erfindung wird, um eine präzise Genexpressionsquantifizierung zu erreichen, die Normalisierung der erhobenen Echtzeit-PCR-Daten (Real-Time-PCR-Daten) mittels eines festen Referenzwertes, der nicht durch die Bedingungen des Experiments beeinflusst wird, durchgeführt. Dazu wird die Expression eines Referenzgens mitgemessen, um einen relativen Mengenvergleich durchzuführen.

Erfindungsgemäß ist vorgesehen, dass zur Bestimmung des Markers der APC ein erstes und ein zweites Aliquot einer Körperflüssigkeit eines Individuums bereitgestellt werden. Im Zusammenhang mit der vorliegenden Erfindung wird unter "Bereitstellen" verstanden, dass das Aliquot der Körperflüssigkeit bereits in einem Behältnis vorliegt. "Bereitstellen" kann erfindungsgemäß aber auch bedeuten, dass das Aliquot der Körperflüssigkeit unmittelbar aus einem Patienten, beispielsweise durch die Entnahme von Blut, zur Verfügung gestellt wird. Das erfindungsgemäße Verfahren sieht vor, dass das erste Aliquot mit mindestens einem Antigen stimuliert wird, das zweite Aliquot aber unstimuliert bleibt. Insgesamt ist somit vorgesehen, dass das erste und zweite Aliquot mit Ausnahme des in Kontakt Bringens mit dem Antigen identisch sind. Insofern dient das zweite unstimulierte Aliquot als eine Art Kalibrator. Die Quantifizierung wird somit relativ zu dem Kalibrator durchgeführt. Zur Bestimmung und Quantifizierung des Markers ist dann vorgesehen, dass die Menge des Markers in dem ersten stimulierten Aliquot durch die Menge des Markers in dem zweiten unstimulierten Aliquot dividiert wird. Es wird somit ein n-facher Mengenunterschied des Markers des ersten stimulierten Aliquots relativ zum Kalibrator, also dem zweiten unstimulierten Aliquot, nachgewiesen. Das erfindungsgemäße Verfahren stellt ein Verfahren dar, das ausschließlich ex vivo durchgeführt wird.

Das erfindungsgemäße Verfahren ermöglicht vorteilhafterweise gegenüber bekannten verfügbaren Verfahren den Nachweis aktivierter T-Helferzellen mit einer gesteigerten Sensitivität, Schnelligkeit und Verlässlichkeit der Versuchsdurchführung. Darüber hinaus ermöglicht das erfindungsgemäße Verfahren eine Abgrenzung sowie eine differenzielle Bestimmung insbesondere von aktivierten T-Zellen gegenüber Gedächtnis-T-Zellen.

Eine wesentliche Schwierigkeit für den gesicherten Nachweis aktivierter T-Zellen bei aktiven mikrobiellen Infektionen und insbesondere bei Autoimmun- und Tumorerkrankungen liegt in der sehr geringen Zahl der in der Zirkulation befindlichen krankheits- bzw. erregerspezifischen aktivierten T-Zellen. T-Zellen werden in lymphatischen Organen durch den Kontakt mit Antigen-beladenen APC aktiviert, proliferieren und wandern dann über das Blut oder die Lymphe zu dem Ort der Infektion oder Erkrankung, wo sie oftmals verweilen und ihre Effektorfunktionen ausüben.

Das erfindungsgemäße Verfahren beruht auf der Bestimmung von T-Zell-induzierbaren Komponenten, wie RNA-Moleküle oder Proteine, deren Produktion in APC nach einem Antigen-spezifischen Kontakt mit einer aktivierten T-Zelle messbar moduliert, das heißt gesteigert, reduziert oder modifiziert wird.

Die Bestimmung T-Zell-induzierter Reifungsprozesse in APC oder APC-haltigen Kulturen erfolgt mit bisher bekannten Methoden durch die Bestimmung von Markern in Form von Proteinen in Kulturen von spezifisch mit Expressionsvektoren stimulierten Zellen unter Einsatz verschiedener Proteinnachweisverfahren, wie FACS, ELISpot oder ELISA. Diese Verfahren haben sich jedoch für eine verlässliche Bestimmung aktivierter T-Zellen für diagnostische Anwendungen als nur begrenzt oder nicht geeignet erwiesen.

Es ist bekannt, dass T-Zell-induzierbare Markermoleküle bei einer Vielzahl unterschiedlicher Erkrankungen und mikrobieller Infektionen in unterschiedlichen Zellpopulationen produziert werden, wodurch die zu untersuchenden Probanden oder Patienten individuell eine bisweilen stark variierende und zum Teil bereits sehr hohe Basisexpression dieser Markerproteine aufweisen. Zudem beinhalten APC oftmals bereits größere Mengen präformierte, also bereits exprimierte Markermoleküle, die unabhängig von einer Antigen-spezifischen Stimulation durch eine aktivierte T-Helferzelle vorliegen. Des Weiteren stimuliert eine aktivierte T-Helferzelle üblicherweise nur eine bzw. nur wenige APC, wobei auf zellulärer Ebene nur eine begrenzte Sensitivitätssteigerung erreicht werden kann. Aufgrund dieser bekannten Umstände wurde bislang davon ausgegangen, dass eine verlässliche Bestimmung von aktivierten T-Zellen durch den Nachweis spezifischer Marker auf Proteinebene in durch die aktivierten T-Zellen stimulierten APC in einem diagnostisch verlässlichen Maßstab nicht möglich sei.

Überraschenderweise konnte gezeigt werden, dass das erfindungsgemäße Verfahren durch die Bestimmung der T-Zell-induzierten Produktion von beispielsweise mRNA Molekülen als Marker in APC durchgeführt werden kann. Aufgrund der vorgenannten Schwierigkeiten im Hinblick auf die variierende Expression bestimmter Markermoleküle ist es als unerwartet anzusehen, dass der erfindungsgemäße Nachweis eines Markers der APC auf RNA-Ebene eine verlässliche und sensitive Bestimmung Antigen-spezifischer aktivierter T-Zellen ermöglicht. So induziert der Kontakt einer aktivierten T-Zelle mit einer APC die Produktion einer sehr hohen Menge an mRNA-Molekülen, was zu einer starken Amplifikation des Markers und somit zu einer deutlich gesteigerten Sensitivität des erfindungsgemäßen Verfahrens führt.

Es bestanden bislang jedoch grundsätzliche technische Vorurteile gegenüber der Nutzung T-Zell-induzierbarer Marker, wie die Produktion von mRNA Molekülen, in APC als Nachweis aktivierter Antigen-spezifischer T-Zellen als Marker für aktive Krankheitsgeschehen. Diese technischen Vorurteile beruhen insbesondere auf der Tatsache, dass spezifische Marker in den APC individuell eine sehr schwankende Basisexpression aufweisen. Eine Kompensation dieser Schwankungen ist jedoch für eine Definition verlässlicher Schwellenwerte für ein aussagekräftiges Testergebnis zwingend erforderlich.

Diese technischen Vorurteile wurden in dem erfindungsgemäßen Verfahren insbesondere durch die Bestimmung der relativen Steigerung der Markerexpression durch den Nachweis des Markers der APC in einem ersten mit mindestens einem Antigen stimulierten Aliquot und einem zweiten unstimulierten Aliquot einer Körperflüssigkeit eines Individuums und das Bestimmen des Verhältnisses des ersten Aliquots und des zweiten Aliquots überwunden.

In einer bevorzugten Ausführungsform der Erfindung ist ein Verfahren vorgesehen, wobei in Schritt c) zusätzlich mindestens ein zweiter Marker in dem ersten und in dem zweiten Aliquot detektiert wird, wobei der zweite Marker ein induzierter Marker der T-Zellen selbst ist, und Schritt d) den Nachweis und das Quantifizieren der T-Zellpopulationen durch Bestimmen des Verhältnisses des detektierten ersten Markers der APC und des zweiten Markers der T-Zelle des ersten Aliquots zu dem zweiten Aliquot umfasst.

Erfindungsgemäß ist durch dieses Verfahren vorgesehen, dass eine Differenzierung zwischen naiven T-Zellen, aktivierten T-Zellen und Gedächtnis-T-Zellen als individuelle T-Zellpopulationen möglich wird. Insofern bietet dieses erfindungsgemäße Verfahren die Möglichkeit zu einer Differenzialdiagnose bei bestimmten Erkrankungen. So ist es unter anderem bei einer Tuberkuloseerkrankung möglich, in Form einer solchen Differenzialdiagnose Patienten mit einer aktiven Erkrankung und einem damit einher gehenden konkreten Behandlungsbedarf von Patienten mit einer latenten Infektion, bei denen kein konkreter Behandlungsbedarf besteht, und gesunden Individuen zu unterscheiden. Diese Differenzialdiagnose wird ermöglicht durch den Nachweis eines ersten und eines zweiten Markers. Der erste Marker stellt hierbei den T-Zell-induzierten Marker der APC dar. Dieser Marker wird nur bei aktiven Krankheitsgeschehen induziert. Der zweite Marker ist ein Marker der T-Zelle selbst. Dieser zweite Marker wird bei Anwesenheit von aktivierten und Gedächtnis-T-Zellen in dem Verfahrensansatz gebildet.

In einer weiteren bevorzugten Ausführungsform der Erfindung ist ein Verfahren vorgesehen, wobei das Verfahren in Schritt a) einen weiteren Schritt a') in Kontakt Bringen des zweiten Aliquots mit mindestens einem Antigen, und in Schritt b) einen weiteren Schritt b') Inkubieren des zweiten Aliquots mit dem Antigen über einen bestimmten Zeitraum, wobei sich der Zeitraum in Schritt b') von dem Zeitraum in Schritt b) unterscheidet, und statt Schritt c) einen Schritt c') Nachweis und Differenzieren der T-Zellpopulationen durch Detektieren des ersten Markers in dem ersten und zweiten Aliquot durch RT-qPCR, und Schritt d) umfasst.

In einer besonders bevorzugten Ausführungsform der Erfindung ist vorgesehen, dass der Zeitraum des Inkubierens in Schritt b') 0 Minuten beträgt. In einer weiteren bevorzugten Ausführungsform der Erfindung beträgt der Zeitraum von 0 bis 60 Minuten, weiter bevorzugt von 0 bis 45 Minuten, weiter bevorzugt von 0 bis 30 Minuten, weiter bevorzugt von 0 bis 20 Minuten, weiter bevorzugt von 0 bis 15 Minuten, weiter bevorzugt von 0 bis 10 Minuten, insbesondere bevorzugt von 0 bis 5 Minuten. Es ist erfindungsgemäß vorgesehen, dass das zweite Aliquot über einen deutlich kürzeren Zeitraum im Vergleich zu dem Zeitraum der Inkubation des ersten Aliquots bis hin zu einer so genannten "Nullprobe", als für eine Dauer von 0 Minuten, mit dem Antigen in Kontakt gebracht wird. Somit ist vorgesehen, dass innerhalb des Zeitraums des Inkubierens des zweiten Aliquots sich noch kein Marker gebildet hat oder nur sehr geringe Mengen des Markers bis dahin vorhanden sind. Insbesondere ist erfindungsgemäß vorgesehen, dass der Zeitraum in Schritt b') deutlich kürzer ist als der Zeitraum in Schritt b). Erfindungsgemäß findet somit die Inkubation des zweiten Aliquots mit dem Antigen in Schritt b') nur in einem Bereich von wenigen Sekunden bis zu einem 0 Minuten hin dauernden Zeitraum bzw. zumindest nicht über einen längeren Zeitraum statt.

In einer weiteren bevorzugten Ausführungsform der Erfindung ist vorgesehen, dass das zweite Aliquot mit einem irrelevanten Antigen in Schritt a') in Kontakt gebracht und in Schritt b') inkubiert wird. Erfindungsgemäß wird unter einem irrelevanten Antigen ein Antigen verstanden, für das das Individuum keine aktivierten oder Gedächtnis-T-Zellen besitzt. Ein solches irrelevantes Antigen kann beispielsweise Albumin oder in HIV seronegativen Personen ein HIV Protein sein.

In einer bevorzugten Ausführungsform der Erfindung ist ein Verfahren vorgesehen, wobei der Schritt c') den Nachweis und das Differenzieren der T-Zellpopulationen durch das Detektieren des ersten und des zweiten Markers umfasst.

In einer weiteren bevorzugten Ausführungsform der Erfindung ist ein Verfahren vorgesehen, wobei das Aliquot der Körperflüssigkeit in ein nur APC enthaltendes Aliquot A und in ein T-Zellen enthaltendes Aliquot B aufgetrennt wird, und wobei Schritt a) einen Schritt a1) in Kontakt Bringen des Aliquots A mit mindestens einem Antigen, und einen darauf folgenden Schritt a2) in Kontakt Bringen des mit dem mindestens einen Antigen in Kontakt gebrachten Aliquots A mit dem Aliquot B umfasst.

Erfindungsgemäß ist somit vorgesehen, dass die APC zuerst mit dem Antigen beladen werden bevor die T-Zellen hinzugefügt werden. Erfindungsgemäß weiter bevorzugt ist vorgesehen, dass das T-Zellen enthaltende Aliquot B auch unbeladene APC, also APC ohne vorherigen Antigenkontakt, enthält.

Erfindungsgemäß ist vorgesehen, dass die T-Zellpopulationen naive T-Zellen, aktivierte T-Zellen oder Gedächtnis-T-Zellen enthalten.

In einer bevorzugten Ausführungsform der Erfindung ist vorgesehen, dass die T-Zellen der T-Zellpopulation CD4$^+$ T-Zellen, insbesondere Th-1 Zellen, Th-2 Zellen, Th-17 Zellen, CD4$^+$CD25$^+$ regulatorische T-Zellen, Th-3 Zellen, CD8$^+$ T-Zellen, insbesondere CD4$^-$CD8$^+$ zytotoxische T-Zellen, CD4$^+$CD8$^+$ T-Zellen, CD161$^+$ NKT-Zellen und/oder ein Gemisch verschiedener T-Zellen sind.

Erfindungsgemäß weiterhin bevorzugt ist vorgesehen, dass die Körperflüssigkeit Blut, Liquor, Lymphe, Perikardflüssigkeit, eine Bronchiallavage, eine Knochenmarkpunktion, eine Suspension von lymphatischem Gewebe oder eine gereinigte PBMC-Population ist.

Im Rahmen der vorliegenden Erfindung wird unter "lymphatischem Gewebe" Lymphknoten, Milz, Tonsillen sowie das lymphatische Gewebe der Magen-Darm-Schleimhaut, wie Peyers Plaques, das lymphatische Gewebe der Atmungsorgane und der Harnwege verstanden.

In einer weiteren bevorzugten Ausführungsform der Erfindung ist vorgesehen, dass die Körperflüssigkeit zusätzlich eine separierte APC-Population enthält. Weiterhin erfindungsgemäß bevorzugt ist ein so genannter Buffy-Coat als Körperflüssigkeit.

In einer weiteren bevorzugten Ausführungsform der Erfindung ist vorgesehen, dass das Antigen ein Peptid, Oligopeptid, ein Polypeptid, ein Protein, eine RNA oder eine DNA ist.

In einer weiteren bevorzugten Ausführungsform ist das Antigen ein Expressionsplasmid.

Erfindungsgemäß weiterhin bevorzugt ist das Antigen ein Fragment, ein Spaltprodukt oder ein Bruchstück eines Oligopeptids, eines Polypeptids, eines Proteins einer RNA oder einer DNA.

Erfindungsgemäß bevorzugt ist vorgesehen, dass das Antigen ein Antigen aus einem Bakterium, Virus, Pflanze, Tier, Pilz oder Parasit ist.

In einer weiteren bevorzugten Ausführungsform der Erfindung ist vorgesehen, dass das Antigen ein Polypeptid aus dem Cytomegalievirus (CMV), Epstein-Barr Virus (EBV), Hepatitis B Virus (HBV), Hepatitis C Virus (HCV), humanem Immundefizienz Virus (HIV), Parvovirus B 19, Varizella Zoster Virus (VZV), Vakziniavirus, Adenovirus, JC- und BK-Virus, A, B, C Typ Influenzavirus, *Mycobacterium tuberculosis,* Borrelien, *Toxoplasma gondii* oder Aspergillen oder aber ein Tumor oder Autoantigen ist.

Erfindungsgemäß bevorzugt ist das Antigen ein Antigen aus Viren mit humanpathogenen Eigenschaften. Nachfolgend aufgeführt sind erfindungsgemäße Beispiele für solche Viren.

Diese Auflistung ist im Rahmen der vorliegenden Erfindung nicht als limitierend aufzufassen, sondern lediglich beispielhaft. Polioviren, Coxsachieviren, Echoviren, Enteroviren, Rhinoviren, Orthomyxo-Viren, insbesondere Typ A, B, C Influenzaviren, Paramyxoviren, insbesondere Parainfluenza-Viren, Mumpsviren, Masernviren, Respiratorische Syncytial-Viren (RS-Virus), Coronaviren, Flaviviren, insbesondere Gelbfieber-, Dengue-, Japan B-Enzephalitis-, Zeckenenzephalitis (FSME)-Viren, das Hepatitis C Virus (HCV), Togaviren, insbesondere Alpha- und Rubiviren, Bunyaviren, insbesondere die Bunya-, Hanta-, Nairo-, Phlebo- und Tospovirus, Generaviren, Röteln-Viren, Tollwut-Viren, Arenaviren, insbesondere das lymphozytäre Chorio-Meningitis Virus (LCMV) und das Lassa-Fieber Virus, Gastroenteritis-Viren, insbesondere Rotaviren, Adenoviren, Caliciviren, Astroviren, Coronaviren, Retroviren, insbesondere Typ A, B, C und D Retroviren, Lentiviren, insbesondere die humanen Immundefizienz-Viren Typ-1 (HIV-1) und -2 (HIV-2), das Affenimmundefizienzvirus (SIV), Katzenimmundefizienzvirus (FIV), Rinderimmundefizienzvirus (BIV), Spumaviren, die humanen T-Zell-Leukämie-Viren Typ-1 (HTLV-1) und -2 (HTLV-2), Parvoviren, insbesondere Parvovirus B19 und Adeno-assoziierte Viren (AAV), Papovaviren, insbesondere Papillomviren, das Virus der progressiven multifokalen Leukoenzephalopathie (PML), BK-Virus, Adenoviren, Herpes-Viren, insbesondere das Herpes Simplex Virus Typ-1 (HSV-1) und -2 (HSV-2), das Varizella Zoster Virus (VZV), Zytomegalie Virus (CMV) und Epstein-Barr Virus (EBV), die Humanen Herpesviren 6, 7 und 8 (HHV 6, 7 und 8), Hepatitis-Viren, insbesondere das Hepatitis A Virus (HAV), Hepatitis B Virus (HBV), Hepatitis C Virus (HCV), Hepatitis D Virus (HDV), Hepatitis E Virus (HEV) und Hepatitis G Virus (HGV) sowie das Transfusion-transmitted Virus (TTV) und Pockenviren, insbesondere Ortho-Pockenviren, wie das Menschenpocken-Virus, Vakzinia-Viren, Kuhpocken-Viren und Parapox-Viren. Des Weiteren können die Polypeptide von viralen Erregern seltener, subakuter oder chronischer Krankheiten, insbesondere Marburg- und Ebolaviren, sowie Bornaviren entstammen.

Erfindungsgemäß besonders bevorzugt ist das Antigen ein Polypeptid des Humanen Immunodefienzvirus (HIV), beispielsweise gp120, gp160, p17, p24, Pr55$^{gag}$, Polymerase (Pol), Reverse Transkriptase (RT) und nef.

Weiterhin erfindungsgemäß besonders bevorzugt ist das Antigen ein Polypeptid des Epstein-Barr Virus (EBV), wie EBNA1, EBNA2, EBNA3A, EBNA3B (EBNA4), EBNA3C (EBNA-6) BZLF1, BMLF1, BMRF1, BHRF1, BARF0, BRLF1, BI'EF4, gp85, gp110, gp220/350, VCA p150, EBNA-LB, LMP1 und LMP2 (z.B. zusammengestellt in Khanna et al. (2000), Annu. Rev, Microbiol. 54:19-48).

Erfindungsgemäß besonders bevorzugt ist weiterhin das Antigen ein Polypeptid des Cytomegalovirus (CMV), wie UL123 (IE1), UL122 (IE-2), UL83 (pp65), UL82, HL99, UL28, UL33, UL37, US3, UL94, UL16, UL55(gB), UL85, UL25, US18, UL45 und UL32 (pp150) (z.B. zusammengestellt in Crough et al. (2009) Clin Microbiol Rev. 22:76-98).

Weiterhin erfindungsgemäß besonders bevorzugt ist das Antigen ein Polypeptid des Varicella Zoster Virus (VZV), wie ORF1, ORF4, ORF10, ORF14, ORF29, ORF62 und ORF68 (gE).

Erfindungsgemäß besonders bevorzugt ist weiterhin das Antigen ein Polypeptid des Hepatitis B Virus, wie HBsAg und HBcAg.

Weiterhin erfindungsgemäß besonders bevorzugt ist das Antigen ein Polypeptid von Adenovirus, wie das AdV5 Hexon Protein.

Erfindungsgemäß weiterhin bevorzugt ist das Antigen ein Antigen aus tierpathogenen Viren, die im Folgenden aufgelistet sind. Diese Auflistung ist im Rahmen der vorliegenden Erfindung nicht als limitierend aufzufassen, sondern lediglich beispielhaft. Das equine Morbillivirus (EMP), Picornaviren, insbesondere Enteroviren, Aphthoviren mit dem Erreger der Maul- und Klauenseuche (MKS), das vesikuläre Stomatitis-Virus, Paramyxoviren, insbesondere Morbilliviren, aviäre Paramyxoviren, Pockenviren, insbesondere Capripoxviren, Bunyaviren, Reoviren, insbesondere Orbiviren, Flaviviren, insbesondere Pestiviren, Orthomyxoviren, insbesondere das Influenza A Virus, Herpesviren, insbesondere Alpha-Herpesviren, Tollwutviren, Retroviren, insbesondere Lentiviren und C-Typ Retroviren, Togaviren, Rhabdoviren, Birnaviren, Coronaviren und Caliciviren.

Eine umfassende Auflistung der derzeit beschriebenen Viren wurde beispielsweise von dem Internationalen Committee für Virustaxonomie (*The International Committee on Taxonomy of Viruses* (ICTV)) zusammengestellt und ist über das Internet abrufbar (http://www.ncbi.nlm.nih.gov/Taxonomy/Browser/wwwtax.cgi?mode=Undef&name=Viruses &lvl=3&srchmode=1&keep=1&unlock).

Erfindungsgemäß weiterhin bevorzugt ist das Antigen ein Antigen aus Bakterien. Insbesondere erfindungsgemäß bevorzugt sind Antigene aus humanpathogenen Bakterien. Nachfolgend aufgeführt sind erfindungsgemäße Beispiele für solche Bakterien. Diese Auflistung ist im Rahmen der vorliegenden Erfindung nicht als limitierend aufzufassen, sondern lediglich beispielhaft. Staphylokokken, Streptokokken, Enterokokken, Neisserien, Enterobakterien, insbesondere *Escherichia coli (E. coli),* inklusive säuglingspathogenen *E. coli* Stämmen (EPEC), enteroaggregativen *E. coli* Stämmen (EAggEC), Klebsiellen, Enterobacter, Serratia, Proteus, Citrobacter und typhösen Salmonellen, Enteritis Salmonellen, Shigellen, Yersinien, Vibrionen, insbesondere *Vibrio cholerae* und *Vibrio El Tor,* Pseudomonaden, Burkholderia, Stenotrophomas, Acinetobacter, Campylobacter, Helicobacter, insbesondere *Helicobacter pylori,* Hämophilus, Bordetellen, Legionellen, Listerien, Brucellen, Francisellen, Erysipelothrix, Korynebakterien, Bacillus, Clostridien, Bacteroide, Prevotella, Porphyromonas, Fusobacterium, Anaerobiospirillum, Anaerorhabdus, Anaerovibrio, Butyrivibrio, Centripedia, Desulfomonas, Dichelobacter, Fibrobacter, Leprotricha, Megamonas, Mitsuokella, Rikenella, Sebaldella, Selenomonas, Succinovibrio, Succinimonas, Tisserella, Mycobacterien, insbesondere *M. tubereulosis,* atypische Mycobakterien (MOTT) und *M. leprae*, Nocardien, Treponemen, insbesondere *T. pallidum* und *T. carateum,* Borrelien, insbesondere *B. burgdorferi sensu lato, B. garinii, B. afzelii, B. valaisiana, B. lusitaniae* und *B. spielmani*/A14S und *B. recurrentis,* Leptospiren, Ricksettsien, Coxiellen, Ehrlichien, Bartonellen, Mycoplasmen, insbesondere *M. pneumoniae* und *M. hominis*, Ureaplasmen, Actinomyceten, Chlamydien. Erfindungsgemäß weiterhin bevorzugt können die Antigene weiteren medizinisch bedeutsamen Bakterien entstammen, wie beispielsweise Tropheryma, Pasteurella, Branhamella, Streptobacillus, Spirillum und Gardnerella.

Erfindungsgemäß besonders bevorzugt ist weiterhin das Antigen ein Polypeptid von *M. tubereulosis,* wie CFP-10, ESAT-6, TB7.7, TB37.6 und MPT63 oder ein Polypeptidgemisch, wie beispielsweise Tuberkulin PPD.

Weiterhin erfindungsgemäß besonders bevorzugt ist das Antigen ein Polypeptid von *Borrelia spec,* wie VlsE, p58 (OppA-2), BBK32, p14, p20 (BBQ03), p21-24 (OspC), p37-38 (FlaA), p41 (Flagellin, FlaB), p19 (OspE), p18, Crasp3, BBA36, BB0323, p26 (OspF), p28 (OspD), p30, p39, (BmpA), p60-65 (common antigen, Hsp60), p83-100, p17 (Osp17), p31-32 (OspA) und p34 (Osp B) oder Borrelienlipide oder ein Lysat von Borrelienstämmen.

In einer weiteren bevorzugten Ausführungsform der Erfindung ist das Antigen ein Antigen aus tierpathogenen Bakterien. Nachfolgend aufgeführt sind erfindungsgemäße Beispiele für solche Bakterien. Diese Auflistung ist im Rahmen der vorliegenden Erfindung nicht als limitierend aufzufassen, sondern lediglich beispielhaft. Mycoplasmen, Bacillus, insbesondere *bacillus anthracis,* Brucellen, Mycobakterien, insbesondere *M. tuberculosis* und *M. bovis,* Campylobacter, Tritrichomonas, Leptospiren, Rickettsien, Salmonellen, Clostridien, Actinobazillen, Clamydien, Echinokokken, Listerien, Yersinien, Corynebakterien und Francisella. Eine umfassende Auflistung der derzeit beschriebenen Bakterien ist über das Internet unter http://www.ncbi.nlm.nih.gov/Taxonomy/Browser/wwwtax.cgi?mode=Root abrufbar.

In einer weiteren Ausführungsform der Erfindung ist das Antigen ein Antigen aus einem Pilz. In einer bevorzugten Ausführungsform der Erfindung stammt das Antigen aus einem humanpathogenen Pilz. Nachfolgend aufgeführt sind erfindungsgemäße Beispiele für solche Pilze. Diese Auflistung ist im Rahmen der vorliegenden Erfindung nicht als limitierend aufzufassen, sondern lediglich beispielhaft. Sproßpilze, insbesondere Candida, Cryptococcus, Malassetia, Fadenpilze, insbesondere Aspergillus, Trichphyton, Microsporum, und Epidermophyton, Dimorphe Pilze, insbesondere Histoplasma, Blastomyces, Coccidioides, Paracoccidioides, Sporothrix, und Pneumocystis.

In einer weiteren Ausführungsform der Erfindung ist das Antigen ein Antigen aus einem Parasiten. In einer bevorzugten Ausführungsform der Erfindung stammt das Antigen aus einem humanpathogenen Parasiten. Nachfolgend aufgeführt sind erfindungsgemäße Beispiele für solche Parasiten. Diese Auflistung ist im Rahmen der vorliegenden Erfindung nicht als limitierend aufzufassen, sondern lediglich beispielhaft. Protozoen, wie Trypanosomen, Leishmanien, Trichomonen, Giardia, Amöben, Plasmodien, Toxoplasma, Kryptosporidien, Mikrosporidien. Trematoden, wie Schistosomen, sowie Cestoden, wie Bandwürmer und Echinococcen, sowie Nematoden, wie Trichuris, Trichinella, Strongyloides, Ancyclostoma, Necator, Enterobius, Ascaris und Filarien. In einer weiteren bevorzugten Ausführungsform der Erfindung ist das Antigen ein Polypeptid aus tierpathogenen Parasiten. Nachfolgend aufgeführt sind erfindungsgemäße Beispiele für solche tierpathogene Parasiten. Diese Auflistung ist im Rahmen der vorliegenden Erfindung nicht als limitierend aufzufassen, sondern lediglich beispielhaft. Protozoen, insbesondere Protomonaden, Diplomonaden, Polymastigiden, Amoben, Toxoplasmen und Coccidien, Microsporen, Helminthen, Trematoden, Cestoden und Nematoden.

In einer weiteren bevorzugten Ausführungsform der Erfindung ist vorgesehen, dass das Antigen ein Tumorantigen oder ein Autoantigen ist. Im Zusammenhang mit der vorliegenden Erfindung wird unter einem "Autoantigen" ein Antigen verstanden, das Strukturen in Form von Peptidfragmenten aufweist, die körpereigene Strukturen darstellen. In Fällen, in denen T-Zellen vorliegen, die eine Reaktivität gegen solche Autoantigene aufweisen, ist das Vorliegen einer Autoimmunerkrankung möglich. Autoantigene werden auch als Selbstantigene oder autoimmune Antigene bezeichnet.

Im Rahmen der vorliegenden Erfindung ist vorgesehen, dass durch die Inkubation mit einem tumorassozierten oder autoimmunen Antigen aktivierte T-Zellen nachgewiesen werden können, die auf eine Tumorerkrankung bzw. auf eine Autoimmunerkrankung hinweisen.

In einer bevorzugten Ausführungsform der vorliegenden Erfindung ist das Antigen ein humanes Tumorantigen. Erfindungsgemäß bevorzugt ist das Prostata-spezifische Antigen (PSA), HER-2/neu, Mucin-1, überexprimiertes Wild-Typ p53 sowie Punktmutationen aufweisendes p53, MAGE Antigen und CEA (Carcino-Embryonales Antigen).

In einer weiteren bevorzugten Ausführungsform ist das Antigen ein autoimmunes Antigen. Erfindungsgemäß bevorzugt ist ein autoimmunes Antigen für Multiple Sklerose (MS), wie das Myelin-basische Protein (MBP), das Myelin Oligodendrocyten Glykoprotein (MOG), das Myelin Proteolipid Protein (PLP), Myelin, das MBP/PLP Fusionsprotein (MP4), das Myelin-assoziierte basische Protein auf Oligodendrozyten (MOBP), das Oligodendrozytenspezifische Protein (OSP), das Myelin-assoziierte Glycoprotein (MAG) aber auch das Glykoprotein P0, das periphere Myelinprotein 22 (PMP-22/PAS-II), das p170k/SAG (Schwann Cell Membrane Glykoprotein), das Oligodendrozyten-Myelin-Glykoprotein (OMgp), das Schwann Cell Myelin Protein (SMP), die Transaldolase, S100ß, Alpha B Crystallin, 2', 3'-cyclic nucleotide 3' phosphodiesterase (CNP), der ciliäre neurotrophe Faktor (CNF) und das saure gliale Faserprotein (GFAP).

Erfindungsgemäß weiter bevorzugt ist ein autoimmunes Antigen für Typ 1 Diabetes (juveniler Diabetes mellitus), wie Insulin B, Pre-pro-Insulin (PPI), die Tyrosin Phosphatase IA-2, die Glutaminsäure Decarboxylase 65 (GAD65), das Hitzeschock Protein Hsp60, das Inselzellprotein ICA69, IGRP, cd4, Chromogranin A (ChgA) (siehe auch Velthuis et al. (2010) Diabetes).

In einer besonders bevorzugten Ausführungsform der Erfindung ist das Antigen ausgewählt aus der Gruppe bestehend aus PSA, HER-2/neu, Mucin-1, MAGE, CEA, Myelin-basisches Protein (MBP), Myelin Oligodendrocyten Glykoprotein (MOG), Myelin Proteolipid Protein (PLP), Myelin, Insulin B, Pre-Pro-Insulin, IA-2, GAD65, Hsp60, ESAT-6, CFP-10, TB7.7, TB37.6, MPT63, Tuberkulin PPD, p14, OspC, p37-38 (FlaA), p41, OspE, OspF, OspD, p39, Osp17, OspA, OSP B, Pr55$^{gag}$, p24, p17, POL, RT, nef, pp65, IE1, IE2, BZLF1, EBNA3, EBNA2, EBNA6, BMLF1, EBNA1, ORF1, ORF4, PRF62, ORF68, HBsAg, HBcAg und AdV5.

In einer weiteren bevorzugten Ausführungsform der Erfindung ist das Antigen ausgewählt aus Polypeptiden, die Epitope von T-Helferzellen beinhalten.

In einer weiteren bevorzugten Ausführungsform der Erfindung ist vorgesehen, dass der Zeitraum des in Kontakt Bringens in Schritt a) und des Inkubieren in Schritt b) von 0 Stunden bis 72 Stunden, bevorzugt 4, 6 oder 8 Stunden, beträgt. Weiterhin bevorzugt ist vorgesehen, dass der Zeitraum in Schritt a) und/oder Schritt b) von 0 Stunden bis 72 Stunden, bevorzugt 4, 6 oder 8 Stunden, beträgt. Weiterhin erfindungsgemäß bevorzugt ist der Zeitraum in Schritt a) und/oder Schritt b) ein Zeitraum von 0 bis 48 Stunden, weiter bevorzugt von 0 bis 36 Stunden, weiter bevorzugt von 0 bis 34 Stunden, weiter bevorzugt von 0 bis 32 Stunden, weiter bevorzugt von 0 bis 30 Stunden, weiter bevorzugt von 0 bis 28 Stunden, weiter bevorzugt von 0 bis 26 Stunden, weiter bevorzugt von 0 bis 24 Stunden, weiter bevorzugt von 0 bis 22 Stunden, weiter bevorzugt von 0 bis 20 Stunden, weiter bevorzugt von 0 bis 18 Stunden, weiter bevorzugt von 0 bis 16 Stunden, weiter bevorzugt von 0 bis 15 Stunden, weiter bevorzugt von 0 bis 14 Stunden, weiter bevorzugt von 0 bis 12 Stunden, weiter bevorzugt von 0 bis 10 Stunden, weiter bevorzugt von 0 bis 9 Stunden, weiter bevorzugt von 0 bis 8 Stunden, weiter bevorzugt von 0 bis 7 Stunden, weiter bevorzugt von 0 bis 6 Stunden, weiter bevorzugt von 0 bis 5 Stunden, weiter bevorzugt von 0 bis 4 Stunden, weiter bevorzugt von 0 bis 3 Stunden, weiter bevorzugt von 0 bis 2 Stunden und weiter bevorzugt von 0 bis 1 Stunde. In einer weiteren bevorzugten Ausführungsform der Erfindung beträgt der Zeitraum in Schritt a) und/oder Schritt b) 0 bis 60 Minuten, weiter bevorzugt 0 bis 50 Minuten, weiter bevorzugt von 0 bis 40 Minuten, weiter bevorzugt 0 bis 30 Minuten, weiter bevorzugt 0 bis 20 Minuten, weiter bevorzugt 0 bis 15 Minuten, weiter bevorzugt 0 bis 10 Minuten und weiter bevorzugt 0 bis 5 Minuten.

Erfindungsgemäß weiterhin bevorzugt ist vorgesehen, dass der erste Marker der APC und der zweite Marker der T-Zelle eine Nukleinsäure, insbesondere eine RNA, eine DNA, oder ein Nukleinsäurefragment, ist und durch das in Kontakt Bringen und Inkubieren mit dem mindestens einen Antigen induziert wird.

In einer weiteren bevorzugten Ausführungsform der Erfindung ist vorgesehen, dass der Marker der APC 4-1BB Ligand (4-1BBL), OX40 Ligand (OX40L), TNFSF (CD70), B7.1 (CD80), B7.2 (CD86), Fc□RIII (CD16), Fc□RII (CD32), Fc□RI (CD64) oder ein weiterer Vertreter der TNF/TNF-Rezeptor und/oder Immunglobulin Superfamilie oder ein Mitglied der CXCL Familie z. B. CXCL9, CXCL10, CXCL11 oder ein Mitglied der Chemokine (C-C motif) ligand Familie z. B. CCL2, CCL 7, CCL8, CCL10 oder IL1RN ist.

In einer weiteren bevorzugten Ausführungsform der Erfindung ist vorgesehen, dass der Marker der T-Zelle IFN-$\beta$, INF-$\gamma$, TNF-$\alpha$, IL-2, IL-4, IL-5, IL-6, IL-10, IL-12, IL-13, GM-CSF, TGF-$\beta$, M1P1a, MIP1b, 4-1BB, CD25, Perforin und/oder Granzym ist. Erfindungsgemäß weiterhin vorgesehen ist der Marker der T-Zelle ein weiteres von aktivierten T-Zellen oder reaktivierten Gedächtnis-T-Zellen produziertes Zytokin oder Chemokin.

Erfindungsgemäß bevorzugt sind als Marker insbesondere Nukleinsäuren, die natürlicherweise nach einer Epitop-spezifischen Erkennung einer APC durch eine T-Zelle in ihrer Produktion in der APC messbar gesteigert werden. Beispiele für solche Nukleinsäuren als erfindungsgemäße Marker der APC sind die mRNA Moleküle des 4-1BB Ligand (4-1BBL) und der OX40 Ligand (OX40L) (Oshima et al. 1997, J. Immunol. 159, 3838-3848; den Haan and Bevan; 2000, PNAS 97, 12950-12952). Weiterhin erfindungsgemäß bevorzugt sind mRNA Moleküle der kostimulatorischen Proteine B7.1 (CD80), B7.2 (CD86) und des Fas Liganden (FasL), sowie anderer Proteine der TNF/TNF Rezeptorfamilie und Immunglobulin Superfamilie, sowie verschiedener Fc-Rezeptoren sowie mRNA Moleküle von Zytokinen und Chemokinen. Erfindungsgemäß bevorzugt sind zudem die Nukleinsäuren jedes beliebigen Polypeptids, dessen Produktion infolge der spezifischen Erkennung eines zusammen mit MHC Proteinen auf der Oberfläche von APC präsentierten Polypeptids durch eine aktivierte T-Zelle in derselben APC messbar gesteigert oder reduziert wird.

In einer weiteren bevorzugten Ausführungsform der Erfindung ist vorgesehen, dass der Nachweis und das Quantifizieren in Schritt c) und d) zusätzlich durch PCR, quantitative PCR (qPCR), Microarray, FACS, ELISpot und/oder ELISA durchgeführt wird.

Hierin beschrieben ist ein Kit zur Durchführung der erfindungsgemäßen Verfahren umfassend mindestens ein Antigen und ein Primerpaar zur Amplifikation des ersten Markers.

Ferner ist beschrieben, dass das Kit weiterhin ein Primerpaar zur Amplifikation des zweiten Markers enthält. Außerdem ist beschrieben, dass das Kit weiterhin ein Primerpaar zur Amplifikation des Referenzgens enthält. Weiterhin beschrieben ist, dass das Kit zusätzlich Sonden sowie ein Zellkulturmedium enthält.

Zudem ist beschrieben, dass das Kit zusätzlich RNA-Stabilisierungsreagenzien, ein RT-Mastermix, ein qPCR-Mastermix, eine Positivkontrolle und ein Positivreagenz umfasst. Unter einer "Positivkontrolle" wird eine definierte Menge der zu amplifizierenden Marker DNA verstanden. Unter einem "Positivreagenz" wird ein Reagenz verstanden, das den Marker der APC auch in Abwesenheit der aktivierten T-Zelle unspezifisch stimuliert. Beispiele für ein "Positivreagenz" sind PMA/Ionomycin oder ein aktivierender anti-CD40 Antikörper in Kombination mit Prostaglandin E2 ($PGE_2$).

Beschrieben ist, dass das Kit weiterhin Primer enthält, die einen Nachweis des Markers der APC durch PCR, qPCR oder Microarray ermöglichen.

Im Folgenden wird die Erfindung durch die nachfolgenden Beispiele erläutert. Diese Beispiele sind als besondere Ausführungsform der Erfindung anzusehen und nicht einschränkend aufzufassen.

**Beispiel 1: Spezifische Induktion der 4-1BB Ligand mRNA Produktion in Kokulturen ESAT-6/CFP-10 Protein-beladener PBMC und *ex vivo* expandierter und voraktivierter ESAT-6/CFP-10-spezifischer Th-Zellen**

Zur Etablierung des Verfahrens zum Nachweis, Differenzieren und zum Quantifizieren bestimmter T-Zellpopulationen, wie aktivierte T-Zellen wurden T-Zell-induzierte Reifungsprozesse in Antigen-präsentierenden Zellen (APC) untersucht. Hierzu wurde ein Zellkulturmodell entwickelt, das auf der Kokultur Antigen-beladener APC mit *ex vivo* expandierten, aktivierten Antigen-spezifischen T-Helferzellen beruht. In diesem Modellsystem dienen Kokulturen (i) unbeladener oder mit einem irrelevanten Antigen beladener APC mit voraktivierten Antigen-spezifischen T-Helferzellen und (ii) Antigen-beladener APC mit unspezifisch aktivierten T-Zellen oder aktivierten T-Helferzellen mit einer Spezifität für ein irrelevantes Antigen als Negativkontrollen. Der Nachweis Antigen-spezifischer T-Helferzellen (Th-Zellen) erfolgt in diesem System durch die vergleichende Quantifizierung der Marker mRNA Produktion in spezifisch stimulierten und unstimulierten Kulturansätzen mittels Reverse Transkription quantitative Real-Time Polymerase Kettenreaktion (RT-qPCR).

a) *Ex vivo* Expansion Antigen-spezifischer Th-Zellen

Zur Generierung ESAT-6/CFP-10-spezifischer aktivierter Th-Zellen wurden CD4$^+$ T-Zellen von Spendern mit einer latenten *Mycobacterium tuberculosis* Infektion über 3-4 Wochen *in vitro* expandiert. Dazu wurde das Blut der Probanden in eine heparinisierte Spritze entnommen, mit PBS$_{ohne}$ (Phosphat-gepufferte Salzlösung ohne bivalente Ionen; Lonza) verdünnt und anschließend die Blutzellen über einen Dichtezentrifugationsgradienten unter Verwendung von Pancoll (Pan Biotech) für 30 min bei 20°C und 800 x g entsprechend ihrer Dichte aufgetrennt. Anschließend wurden die PBMC aus dem Gradienten isoliert und zweimal mit PBS$_{ohne}$ für 10 min bei 300 x g gewaschen. Anschließend wurden die CD4$^+$ T-Zellen unter Verwendung der MACS Technologie (CD4$^+$ T Cell Isolation Kit II, Miltenyi) isoliert und durch wöchentliche Stimulation mit einem ESAT-6/CFP-10 Fusionsprotein (AG Lindner, Institut für Medizinische Mikrobiologie und Hygiene, Universitätsklinikum Regensburg) beladenen, autologen APC (reife dendritische Zellen, PBMC) in einem Verhältnis von 1:3 (Th-Zellen:APC) in komplettem R5 Medium (RPMI 1640 (PAN Biotech), 5 % humanes AB Serum (hergestellt aus dem Blut von Spendern mit der Blutgruppe AB), 1 % Penicillin/Streptavidin (PAN Biotech)) kultiviert. Die APC wurden dazu mit 10 µg/ml des ESAT-6/CFP-10 Fusionsproteins in einer Konzentration von $1 \times 10^7$ Zellen/ml in komplettem R5 Medium für 3 Stunden (h) bei 37°C in befeuchteter Atmosphäre mit 5 % $CO_2$ beladen und vor der Kultivierung mit den isolierten Th-Zellen einer γ-Strahlung von 30 Gy unterzogen. Für eine optimale Proliferation der Th-Zellen wurden die Kulturen mit 50 U/mL rekombinantem IL-2 (Proleukin S, Novartis) versetzt. Die Proliferation der Th-Zellen wurde wöchentlich durch die Bestimmung der Gesamtzellzahl vitaler Zellen unter Verwendung eines Hämozytometers ermittelt. Die Spezifität der Kultur wurde ebenfalls wöchentlich durch die Bestimmung der Zahl IFN-γ und CD40L positiver T-Zellen nach Stimulation mit bestrahlten ESAT-6/CFP-10 beladenen PBMC ermittelt. Dazu wurden die Th-Zellen der Expansionskultur in einem Verhältnis von 1:1 mit frisch isolierten autologen PBMC für 6 h bei 37°C and 5 % $CO_2$ in Anwesenheit von 10 µg/ml ESAT-6/CFP-10 inkubiert. Nach zwei stündiger Inkubation wurde Brefeldin A zur Unterbindung der Zytokinfreisetzung aus den Zellen hinzugefügt. Mit je 1 µg/ml PMA/Ionomycin stimulierte beziehungsweise unstimulierte Proben dienten als Positiv- und Negativkontrollen. Zu jedem Stimulationsansatz wurden 1 µg/ml kostimulatorische monoklonale anti-CD49d und anti-CD28 Antikörper hinzugefügt. Auf die Inkubation folgte ein einmaliger Waschschritt. Hierzu wurden die Zellen mit 9 ml PBS$_{ohne}$ versetzt und bei 4°C und 300 x g für 8 Minuten zentrifugiert. Der Überstand wurde verworfen und die Zellen im Rückfluss resuspendiert. Anschließend wurden Oberflächenmoleküle mit je 5 µl der folgenden Fluorophor-konjugierten Antikörper markiert: anti-CD3 Allophycocyanin-H7 (BD), anti-CD4 Allophycocyanin (BD), anti-CD8 Peridinin Chlorphyll Protein (BD). Die Oberflächenfärbung wurde 20 min im Dunkeln bei Raumtemperatur durchgeführt. Danach wurden die Zellen gewaschen und mit 500 µl 2 % Paraformaldehyd (PFA) (Sigma Aldrich) in PBS$_{ohne}$ für 10 min bei Raumtemperatur im Dunkeln fixiert. Nach einem weiteren Waschvorgang wurde in Anwesenheit von 10 µl 2 % Saponin (Carl Roth) in PBS$_{ohne}$ zur Permeabilisierung der Zellen die Färbung des intrazellulären IFN-γ sowie des intrazellulären CD40L unter Verwendung von 1 µl anti-IFN-γ Fluoresceinisothiocyanat (BD) und 10 µl anti-CD40L R-Phycoerythrin (BD) 30 min bei Raumtemperatur im Dunkeln durchgeführt. Anschließend wurden die Zellen zwei Mal mit 0,1 % Saponin in PBS$_{ohne}$ gewaschen und in 300 µl 1 % PFA in PBS$_{ohne}$ aufgenommen. Die gefärbten Zellen wurden in einem FACS CANTO II Durchflusszytometer (BD) analysiert. Hierzu wurden zunächst Zellpopulationen nach Vorwärtsstreulicht und Seitwärtsstreulicht aufgetrennt. Die Lymphozytenpopulation wurde anschließend hinsichtlich der Expression von CD3 untersucht. Alle CD3$^+$ Zellen wurden anschließend bezüglich der Expression des CD4 und CD8 Proteins analysiert. CD4⁺ T-Zellen wurden dann hinsichtlich der Expression von IFN-γ und CD40L untersucht.

Hierbei wurde der Prozentsatz aktivierter ESAT-6/CFP-10-spezifischer T-Helferzellen anhand der Expression von IFN-γ und CD40L bestimmt (Daten nicht dargestellt). In den Expansionskulturen konnte eine kontinuierliche Proliferation von T-Helferzellen beobachtet werden, welche mit der Zunahme ESAT-6/CFP-10-spezifischer Th-Zellen einherging. Nach 3-4 Wochen konnten somit 5 bis 18 % spezifische Th-Zellen in den Expansionskulturen generiert werden. Die Erzeugung und Charakterisierung EBV- (BZLF1) und CMV- (pp65) spezifischer Th-Zellen erfolgte unter Einsatz von Blutproben EBV- bzw. CMV-seropositiver Spender, entsprechend dem zuvor beschriebenen Versuchsprotokoll.

b) Kokultur *ex vivo* voraktivierter ESAT-6/CFP-10-spezifischer Th-Zellen mit frisch präparierten autologen ESAT-6/CFP-10 Fusionsprotein-beladenen PBMC

Frisch präparierte PBMC von Spendern mit latenter Tuberkulose wurden in Anwesenheit und in Abwesenheit von 10 μg/ml ESAT-6/CFP-10 Fusionsprotein mit *ex vivo* voraktivierten ESAT-6/CFP-10-spezifischen Th-Zellen aus der Expansionskultur in B-Zell-Medium (Iscove's Modified Dulbecco's Medium (IMDM) (Lonza), 10% humanes AB Serum, 6 ng/ml IL-4 (Miltenyi Biotech), 50 ng/ml Transferrin (Roche), 5 μg/ml Insulin (Roche)) in einem Verhältnis von 1:1 bei einer Gesamtzellzahl von $1\times10^6$ Zellen/ml kokultiviert. Die Zellen wurden für 10 h bei 37°C und 5 % CO₂ inkubiert, wobei zu unterschiedlichen Zeiten (z.B. nach 0; 0,5; 1; 2; 3; 4; 6; 8 und 10 h) Proben von jeweils $2\times10^5$ Zellen entnommen wurden. Diese wurden zügig abzentrifugiert, der Überstand verworfen und das Pellet sofort in flüssigem Stickstoff eingefroren. Bis zur weiteren Verarbeitung wurden die Proben bei -80 °C aufbewahrt. Zuvor wurde in einer Kokultur frisch präparierter PBMC mit autologen *ex vivo* voraktivierten Th-Zellen im zeitlichen Verlauf nach einer Stimulation mit ESAT-6/CFP-10 die Anwesenheit des Aktivierungsmarkers CD40L auf der Oberfläche der Th-Zellen untersucht. Diese Untersuchungen zeigten, dass spezifisch *in vitro* restimulierte Th-Zellen eine transiente Expression des CD40L auf ihrer Oberfläche aufweisen, wobei nach 6 stündiger Kokultur mit Antigen-beladenen APC die höchste Zahl CD40L-exprimierender Zellen zu beobachten war (Daten nicht gezeigt).

c) Quantifizierung der 4-1BBL mRNA in PBMC mittels der RT-qPCR als indirekter Nachweis für Antigen-spezifische aktivierte Th-Zellen

Aus den Zellproben wurde unter Verwendung des peqLab peq GOLD Total RNA Kit (peqLab) die Gesamt-RNA isoliert. DNA-Kontaminationen wurden mittels des peqGOLD DNaseI Digest Kit (peqLab) entfernt. Die Gesamt-RNA wurde in 60 μl DEPC Wasser (1 L destilliertes Wasser plus 1 ml Diethylpyrocarbonat (Sigma); Inkubation für 1 h bei 37°C und nachfolgendes Autoklavieren für 15 min bei 121°C) eluiert.

Für die Reverse Transkription (RT-PCR) in cDNA wurden 10 μl der eluierten RNA mit 10 μl des RT-PCR Mastermixes versetzt. Der RT-PCR Mastermix enthält 50 mM Puffer A (TaqMan 1000 RXN Gold mit Buffer A (Applied Biosystems), 5 mM MgCl₂ (TaqMan 1000 RXN Gold mit Buffer A (Applied Biosystems)), 2,5 mM randomisierter Hexamer-Primer (MWG Operons/Eurofins), 5 mM dNTP, 2,5 U/μl MuLV Reverse Transkriptase (Applied Biosystems), 1 U/μl RNase-Inhibitor (Applied Biosystems) in DEPC Wasser. Die Reverse Transkription erfolgte für 15 min bei 23°C, 5 min bei 95°C und 30 min bei 42°C und wurde unter Einsatz eines PTC-200 Peltier Thermocycler (MJ Research) durchgeführt. Anschließend wurden 24 μl qPCR Mastermix (50 mM Puffer A, 6,875 mM MgCl₂, 0,308 μM 4-1BBL Forward Primer GAGGGTCCCGAGCTTTCG, biomers , dargestellt in SEQ ID NO:1; 0,308 μM 4-1BBL Reverse Primer GCCCATCGATCAGCAGAAC, biomers, dargestellt in SEQ ID NO:2; 0,2525 μM 4-1BBL Sonde FAM-CCACCAGCTGCGCAAACATGC-TMR, TIB Molbiol, dargestellt in SEQ ID NO:3; 0,308 μM GAPDH Forward Primer GAAGGTGAAGGTCGGAGTC, biomers, dargestellt in SEQ ID NO:4; 0,308 μM GAPDH Reverse Primer GTAAACCATGTAGTTGAGGTC, biomers, dargestellt in SEQ ID NO:5; 0,2525 μM GAPDH Sonde YAK-TCATTGATGGCAACAATATCCACT-TMR, TIB Molbiol, dargestellt in SEQ ID NO:6; 0,003125 U/μl TaqGold Polymerase in DEPC Wasser mit 6 μl der erzeugten cDNA versetzt. Das Temperaturprofil der qPCR umfasste einen 10 minütigen Schritt bei 95°C und anschließend 50 Zyklen bestehend aus 15 s bei 95°C und 1 min bei 60°C und wurde an einem StepOnePlus Real-Time PCR System (Applied Biosystems) durchgeführt.

THP-1 Zellen, die konstitutiv 4-1BBL mRNA produzieren, dienten als Positivkontrolle und Referenz. Diese wurden in dem Test in gleicher Weise wie die Proben behandelt. Es erfolgte anschließend eine relative Quantifizierung der 4-1BBL mRNA unter Zuhilfenahme der ebenfalls zuvor amplifizierten Glycerinaldehyd-3-phosphat-Dehydrogenase (GAPDH) als Referenzgen nach der folgenden Formel:

$$R = 2^{-\Delta\Delta Cq}$$

$$\Delta\Delta Cq = [(Cq\ 4\text{-}1BBL\ (S) - Cq\ GAPDH\ (S)] - [(Cq\ 4\text{-}1BBL\ (NK) - Cq\ GAPDH\ (NK)]$$

Die Ergebnisse sind angeben als das Verhältnis der Marker mRNA Produktion der stimulierten Probe und der zugehörigen Negativkontrolle, die zuvor mit GAPDH normalisiert wurden. Von jeder Probe wurde eine Dreifachbestimmung durchgeführt.

Diese Untersuchungen zeigen, dass ESAT-6/CFP-10-spezifische, voraktivierte Th-Zellen in Kokulturen mit autologen, ESAT-6/CFP-10 Protein-beladenen PBMC im Vergleich mit unbeladenen PBMC eine messbar gesteigerte 4-1BBL mRNA Produktion induzieren (Figur 3).

**Beispiel 2: Korrelation der Induktion der 4-1BB Ligand mRNA Produktion in Kokulturen ESAT-6/CFP-10 Protein-beladener PBMC mit der Zahl *ex vivo* expandierter ESAT-6/CFP-10 Protein-spezifischer Th-Zellen**

ESAT-6/CFP-10-spezifische Th-Zellen von latent mit *Mycobacterium tuberculosis* infizierten Probanden wurden wie in Beispiel 1 unter Punkt a) beschrieben *ex vivo* expandiert und anschließend mit frisch präparierten autologen PBMC wie in Beispiel 1 unter Punkt b) beschrieben kokultiviert. Dabei wurde die Anzahl ESAT-6/CFP-10-spezifischer Th-Zellen, die mit $1\times10^6$ PBMC kokultiviert wurde, in einer halblogarithmischen Konzentrationsreihe titriert (Konzentrationsstufen: keine, 117, 370, 1170, 3700, 11700, 37000, 117000 voraktivierte Th-Zellen pro $1\times10^6$ PBMC). Nach 0; 0,5; 1; 2; 3; 4; 6; 8 und 10 stündiger Inkubation wurden $2\times10^5$ Zellen herausgenommen, für 5 min bei 300 g pelletiert und das Pellet sofort in flüssigem Stickstoff eingefroren. Bis zum weiteren Gebrauch wurden die Zellen bei -80°C aufbewahrt. Die Gesamt-RNA wurde nach dem Protokoll in Beispiel 1 unter c) isoliert und in der RT-qPCR hinsichtlich der Produktion der 4-1BBL mRNA analysiert.

In diesen Untersuchungen konnte eine klare Korrelation zwischen der Zahl voraktivierter ESAT-6/CFP-10-spezifischer Th-Zellen und der Signalstärke des Markers (gesteigerte Produktion der 4-1BBL mRNA) beobachtet werden (Figur 4).

**Beispiel 3: Induktion der 4-1BBL sowie der IFN-☐ mRNA Produktion in Kokulturen nicht stimulierter und mit ESAT-6/CFP-10 stimulierter PBMC und autologer *ex vivo* voraktivierter *M. tuberculosis* ESAT-6/CFP-10-spezifischer Th-Zellen**

ESAT-6/CFP-10-spezifische Th-Zellen von latent mit *Mycobacterium tuberculosis* infizierten Probanden wurden wie in Beispiel 1 unter Punkt a) beschrieben *ex vivo* expandiert und anschließend mit frisch präparierten autologen PBMC wie in Beispiel 1 unter Punkt b) beschrieben kokultiviert. Dabei wurden 50000 *ex vivo* voraktivierte ESAT-6/CFP-10-spezifische Th-Zellen mit $1\times10^6$ PBMC kokultiviert. In Abständen von 2 Stunden wurden im Zeitraum zwischen 0 und 32 Stunden jeweils $2\times10^5$ Zellen dem Kultivierungsansatz entnommen, für 5 min bei 300 x g pelletiert und das Pellet sofort in flüssigem Stickstoff eingefroren. Bis zum weiteren Gebrauch wurden die Zellen bei -80°C aufbewahrt. Die Gesamt-RNA wurde nach dem Protokoll in Beispiel 1 unter c) isoliert und in der RT-qPCR hinsichtlich der Produktion der 4-1BBL mRNA analysiert. Zudem wurde ebenfalls wie in Beispiel 1 unter c) beschrieben die Produktion der mRNA des Zytokins IFN-γ untersucht, das nach Antigen-spezifischem Kontakt von APC mit spezifischen T-Zellen durch die T-Zelle ausgeschüttet wird. Hierzu wurde das folgende Primer-Sonden-System verwendet, wobei auch hier GAPDH als Referenzgen verwendet wurde: IFN-γ Forward Primer GTGGAGACCATCAAGGAAGACAT, biomers, dargestellt in SEQ ID NO:7; IFN-γ Reverse Primer GGCGACAGTTCAGCCATCA, biomers, dargestellt in SEQ ID NO:8; IFN-γ Sonde FAM-TTCATGTATTGCTTTGCGTTGGACATTCAA-TMR, dargestellt in SEQ ID NO:9.

Bei latent mit *M. tuberculosis* infizierten Personen befinden sich im PBMC Gemisch *M. tuberculosis* spezifische Th-Zellen, die zum Teil auch eine Spezifität für ESAT-6/CFP-10 aufweisen. Aktivierte ESAT-6/CFP-10-spezifische Th-Zellen treten hingegen transient während einer akuten Erkrankung auf und sind nur dann im PBMC Gemisch vorzufinden. Das Zutitrieren *ex vivo* voraktivierter ESAT-6/CFP-10-spezifischer Th-Zellen in PBMC ahmt eine akute *M. tuberculosis* Infektion nach. Diese Untersuchungen zeigen, dass in der Kokultur *ex vivo* voraktivierter ESAT-6/CFP-10-spezifischer Th-Zellen mit autologen PBMC eines Spenders mit latenter Tuberkulose neben der gesteigerten Produktion der 4-1BBL mRNA auch ein relativer Anstieg der IFN-☐ mRNA nachweisbar war (Figur 5A und 5B).

**Beispiel 4: Induktion der 4-1BBL mRNA Produktion in Kokulturen ESAT-6/CFP-10-beladener PBMC mit ESAT-6/CFP-10-spezifischen aktivierten Th-Zellen oder unspezifisch voraktivierten aktivierten Th-Zellen**

Zur Überprüfung der Antigen-Spezifität des RTT Verfahrens wurden PBMC eines Spenders mit latenter Tuberkulose in An- und Abwesenheit des ESAT-6/CFP-10 Proteins mit voraktivierten ESAT-6/CFP-10-spezifischen aktivieren Th-Zellen oder nicht ESAT-6/CFP-10-spezifischen aktivierten Th-Zellen stimuliert und zu verschiedenen Zeiten der relative Anstieg der 4-1BBL Produktion in ESAT-6/CFP-10 stimulierten im Vergleich zu nicht stimulierten Zellkulturen mittels der RT-qPCR Technik ermittelt. Dazu wurden Th-Zellen wie in Beispiel 1 unter a) beschrieben isoliert und die gewonnenen Th-Zellen mit kommerziell erhältlichen T-Zell Expanderbeads (Dynabeads, Invitrogen) in einem Verhältnis von 2:1 in R5 Medium für 14 Tage in einer befeuchteten Atmosphäre bei 37°C und 5 % $CO_2$ kultiviert. Die Aktivierung der T-Zellen wurde durchflusszytometrisch durch das Monitoring der Expression des CD40L ermittelt. Diese Untersuchungen belegten, dass mehr als 40 % der Th-Zellen nach einer 14-tägigen Stimulation mit Expanderbeads den CD40L auf ihrer Oberfläche trugen (Daten nicht gezeigt).

Anschließend wurden $1\times10^6$ frisch präparierte autologe PBMC/ml in An- und Abwesenheit von 10 µg/ml ESAT-6/CFP-10 mit jeweils 0, 210, 6636 oder 66360 *ex vivo* vorstimulierten ESAT-6/CFP-10-spezifischen Th-Zellen (Generierung wie in Beispiel 1 unter a) beschrieben) oder unspezifisch aktivierten Th-Zellen (Generierung wie in Beispiel 1 unter b) beschrieben) kokultiviert. Nach 0; 0,5; 1; 2; 3; 4; 6 und 8 Stunden wurden der Kokultur jeweils $2\times10^5$ Zellen entnommen und wie in Beispiel 1 unter c) in einer RT-qPCR hinsichtlich der Produktion der 4-1BBL mRNA untersucht.

Diese Experimente zeigten einen direkten Zusammenhang zwischen der Zahl voraktivierter ESAT-6/CFP-10-spezifischer Th-Zellen und der relativen Erhöhung der 4-1BBL Expression in spezifisch stimulierten im Vergleich mit unstimulierten Kokulturansätzen. Dagegen war in Kokulivierungsansätzen mit unspezifisch voraktivierter Th-Zellen ein deutlich reduzierter relativer Anstieg der Produktion der mRNA des 4-1BBL zu beobachten (Figur 6).

**Beispiel 5: Spezifität der Induktion der 4-1BBL mRNA Produktion in Kokulturen von PBMC ausgewählter *M. tuberculosis,* EBV und CMV-positiver Spender und *ex vivo* voraktivierten *M. tuberculosis-* ESAT-6/CFP-10-, EBV BZLF1- oder CMV- pp65- spezifischen Th-Zellen in An- und Abwesenheit von ESAT-6/CFP-10, BZLF1 oder pp65**

In einem weiteren Experiment wurde untersucht, ob (i) die Beladung von PBMC mit *M. tuberculosis* ESAT-6/CFP-10, EBV BZLF1 und CMV pp65 Protein *per se* eine unspezifische Aktivierung der 4-1BBL mRNA induziert und ob (ii) die Induktion des RTT Markers infolge einer Interaktion einer APC mit einer aktivierten Th-Zelle Antigen-spezifisch erfolgt. Dazu wurden unter Einsatz von isolierten Th-Zellen von EBV- und CMV-seropositiven Spendern mit einer latenten mit *M. tuberculosis* Infektion wie in Beispiel 1 unter Punkt a) beschrieben *ex vivo* expandiert und voraktiviert. 70000 der *ex vivo* expandierten T-Helferzellen wurden anschließend mit jeweils $1\times10^6$ PBMC/ml frisch präparierter autologer PBMC wie in Beispiel 1 unter Punkt b) beschrieben in An- und Abwesenheit von 10 µg/ml ESAT-6/CFP-10, BZLF-1 oder pp65 kokultiviert. Nach einer 0; 0,5; 1; 2; 3; 4; 6; 8 und 10 stündiger Inkubation wurden der Kokultur jeweils $2\times10^5$ Zellen entnommen, für 5 min bei 300 x g pelletiert und das Pellet sofort in flüssigem Stickstoff eingefroren. Bis zum weiteren Gebrauch wurden die Zellen bei -80°C aufbewahrt. Die Gesamt-RNA wurde nach dem Protokoll wie in Beispiel 1 unter c) beschrieben isoliert und mittels der RT-qPCR Technik hinsichtlich der Produktion der 4-1BBL mRNA analysiert.

Diese Untersuchungen belegten, dass die Stimulation von APC-haltigen PBMC mit verschiedenen Stimulatorantigenen keine unspezifische Aktivierung der 4-1BBL mRNA hervorruft. Zudem belegten diese Untersuchungen, dass eine gesteigerte Produktion der 4-1BBL mRNA in APC ausschließlich nach Antigen-spezifischem Kontakt aktivierter Th-Zellen mit APC stattfindet. Wurden *M. tuberculosis-spezifische* Th-Zellen mit autologen PBMC kokultiviert, lösten sie allein in Anwesenheit von ESAT-6/CFP-10 eine Induktion der 4-1BBL mRNA Produktion aus, jedoch nicht in Anwesenheit von BZLF1 oder pp65 oder in Abwesenheit eines Antigens (Figur 7 und 8a). Zudem induzierten *in vitro* voraktiviere BZLF1- bzw. pp65-spezifische Th-Zellen nur in den Kulturen autologer PBMC eine gesteigerte Produktion der 4-1BBL mRNA, die mit den jeweiligen Zielstrukturen der jeweiligen aktivierten T-Zellen beladen waren (Figur 8b und 8c).

Weiterhin weisen die durchgeführten Untersuchungen darauf hin, dass die Effizienz der Induktion der 4-1BBL mRNA invers mit der Zahl der in PBMC vorliegenden Antigen-spezifischen CTL korreliert. So finden sich im Blut von TB Patienten üblicherweise sehr wenige ESAT-6/CFP-10-spezifische CTL, wohingegen in CMV und insbesondere EBVseropositiven Probanden ein signifikater bzw. dominierender Anteil der vorliegenden Gedächtnis T-Zellen der Subpopulation zytotoxischer T-Zellen (CTL) zuzuordnen sind. Hier war in Kokulturen ESAT-6/CFP-10-spezifischer voraktivierter Th-Zellen mit ESAT-6/CFP-10 beladenen PBMC die stärkste und in Kokulturen BZLF1-spezifischer voraktivierter Th-Zellen mit BZLF1 beladenen PBMC die geringste Aktivierung des 4-1BBL zu beobachten. Dieser Befund kann durch die Lyse signalgebender APC durch spezifische CTL erklärt werden.

**Beispiel 6: Modulation der Induktion der 4-1BB Ligand mRNA Produktion in Kokulturen von BZLFI-beladenen PBMC und *ex vivo* aktivierten BZLF1-spezifischen Th-Zellen durch Zugabe eines blockierenden anti-MHC Klasse 1 Antikörpers**

*M. tuberculosis* Infektionen werden überwiegend durch Th-Zellen kontrolliert. Darüber hinaus spielen auch zytotoxische T-Zellen (CTL) bei der Bekämpfung des Erregers eine Rolle indem sie von Erregern befallene Zellen erkennen und zerstören. Bei bestimmten Infektionen, z. B. einer Epstein-Barr-Virus (EBV) Infektion stellen CTL die dominante T-Lymphozyten Population dar. Im Falle des erfindungsgemäßen Verfahrens, basierend auf der so genannten reversen T-Zelltechnologie, RTT, ist es möglich, dass CTL auch mit den erregerspezifischen Zielstrukturen beladene APC erkennen und zerstören, noch bevor diese den Marker der APC, RTT Marker, exprimieren. Dadurch wird die Zahl möglicher signalgebender APC und somit die Sensitivität des Tests verringert. Um den Einfluss zytotoxischer T-Zellen auf die Sensitivität des RTT Verfahrens zu untersuchen, wurden Kokultivierungsexperimente mit *in vitro* voraktivierten BZLF1-spezifischen Th-Zellen (Generierung in Beispiel 1 unter a) beschrieben) mit BZLF1-beladenen autologen PBMC in An- und Abwesenheit eines MHC-I blockierenden Antikörpers (10$\mu$g/ml) (HLA ABC Antikörper, Cat. No. MCA81EL; Klon W6/32 AbD Serotec) wie in Beispiel 1 unter b) beschrieben durchgeführt. Durch die Bindung des Antikörpers an MHC-I Moleküle auf Antigen-präsentierenden Zellen wird ihre Erkennung durch CTL verhindert, sodass keine Abtötung der Protein-beladenen APC stattfinden kann. Es wurden $1\times10^6$ Zellen/ml in BZM kokultiviert, wobei pro $1\times10^6$ PBMC 70000 BZLF1-spezifische voraktivierte Th-Zellen eingesetzt wurden. Zu den Zeitpunkten 0, 1, 2, 3, 4, 6, 8 und 10 h wurden jeweils $2\times10^5$ Zellen aus dem Ansatz entnommen, pelletiert und sofort in flüssigem Stickstoff eingefroren. Bis zur weiteren Verarbeitung wurden die Proben bei -80 °C aufbewahrt. Anschließend wurde der Gehalt an 4-1BBL mRNA, wie in Beispiel 1 unter c) beschrieben, in einer RT-qPCR analysiert.

In diesen Experimenten konnte durch den Einsatz eines MHC Klasse 1 blockierenden Antikörpers die Induktion der 4-1BBL mRNA in der Kokultur BZLF1-spezifischer Th-Zellen mit BZLF1-beladenen PBMC deutlich gesteigert werden (Figur 9).

**Beispiel 7: Analyse der spezifischen Induktion der 4-1BB Ligand mRNA Produktion in PBMC von Patienten mit aktiver und latenter Tuberkulose, sowie gesunden Spendern zum indirekten Nachweis aktivierter Th-Zellen**

Um zu untersuchen, ob sich das erfindungsgemäße Verfahren, RTT Verfahren, zur Identifizierung von Patienten mit aktiver Tuberkulose und ihre Abgrenzung von Patienten mit latenter Tuberkulose und gesunden Spendern eignet, wurde jeweils von drei (i) nicht mit *M. tuberculosis* infizierten gesunden Spendern (p012, p010, p008), (ii) gesunden Spendern mit einer latenten Tuberkulose (p009, p006, p005), (iii) Spendern, die aufgrund einer aktiven Tuberkulose in den letzten 6 Monaten vor der Untersuchung medikamentös behandelt wurden (p013, p014, p003) und (iv) Spendern mit einer aktiven Tuberkulose vor oder kurz nach Beginn einer kausalen Therapie (p001, p004, p007) Blut in eine heparinisierte Spritze entnommen und daraus PBMC frisch isoliert. Anschließend wurden jeweils $1\times10^6$ Zellen/mL mit 10 $\mu$g/mL ESAT-6/CFP-10 in befeuchteter Atmosphäre bei 37°C und 5% $CO_2$ inkubiert. Zudem wurden PBMC ausgewählter Spender mit den Kontrollantigenen, HIV p24 (Kapsid Antigen) (gesund: p008; latent: p005 und p006; behandelt: p003; aktiv: p007 und p001) oder Rinderserum Albumin (p001) oder als weitere Negativkontrolle in Abwesenheit eines Antigens inkubiert. Jeweils $2\times10^5$ Zellen wurden nach 0,5; 1; 2; 3; 4; 6; 8 und 10 Stunden der Inkubation entnommen, pelletiert und sofort in flüssigem Stickstoff eingefroren. Bis zur weiteren Verarbeitung wurden die Zellen bei -80 °C aufbewahrt. Wie in Beispiel 1 unter c) beschrieben, wurde anschließend der Gehalt an 4-1BBL mRNA in einer RT-qPCR untersucht.

Es konnte gezeigt werden, dass die Stimulation mit ESAT-6/CFP-10 in PBMC gesunder Spender keine Induktion der 4-1BBL mRNA zur Folge hatte (Figur 10a). Ebenfalls konnte keine Induktion der 4-1BBL mRNA in PBMC von latent mit dem *M. tuberculosis* infizierten Probanden (Figur 10b) oder Probanden, die aufgrund einer aktiven Tuberkulose in den letzten 6 Monaten vor der Untersuchung medikamentös behandelt wurden (Figur 10c), gemessen werden. Allein in PBMC akut erkrankter Probanden vor oder kurz nach Beginn einer kausalen Therapie konnte nach Stimulation mit ESAT-6/CFP-10 eine signifikant gesteigerte Induktion der 4-1BBL mRNA in spezifisch stimulierten im Vergleich zu unstimulierten Zellkulturen beobachtet werden (Figur 10d). Die Stimulation der PBMC mit den irrelevanten Antigenen BSA und p24 führte ebenfalls zu keiner gesteigerten Induktion der 4-1BBL mRNA (Figur 10e).

**Beispiel 8: Induktion der 4-1BBL mRNA Produktion in PBMC von Spendern mit akuter und latenter Tuberkulose, sowie von einem gesunden Probanden mit Tuberkulin PPD**

Um zu untersuchen, ob sich die Induktion der 4-1BBL mRNA in PBMC von akuten Tuberkulosepatienten durch den Einsatz eines größeren Antigenspektrums steigern lässt, wurde jeweils einem Probanden mit einer aktiven und latenten Tuberkulose, sowie einem gesunden Spender Blut in eine heparinisierte Spritze entnommen und daraus PBMC frisch isoliert. Anschließend wurden $1\times10^6$ Zellen/mL mit entweder 10 $\mu$g/mL ESAT-6/CFP-10 oder Tuberkulin PPD (Statens Serum Institut, Dänemark) in befeuchteter Atmosphäre bei 37°C und 5 % $CO_2$ inkubiert. Zudem wurden Aliquots der PBMC als Negativkontrolle ohne Antigene und als Positivkontrolle für die Induzierbarkeit der 4-1BBL mRNA Produktion mit je 1 $\mu$g/mL Phorbol-12-Myrestat-13-Acetat (PMA) (Sigma Aldrich) und Ionomycin (Sigma Aldrich) inkubiert. $2\times10^5$ Zellen wurden nach 0, 2, 4, 6, 8, und 10 Stunden der Inkubation entnommen, pelletiert und sofort in flüssigem Stickstoff eingefroren. Bis zur weiteren Verarbeitung wurden die Zellen bei -80°C aufbewahrt. Wie in Beispiel 1 unter c) beschrieben, wurde anschließend der Gehalt an 4-1BBL mRNA in einer RT-qPCR untersucht.

Es konnte gezeigt werden, dass die Stimulation mit Tuberkulin PPD eine vielfach höhere Induktion der 4-1BBL mRNA Produktion in PBMC des Patienten mit einer akuten Tuberkulose hervorruft als ESAT-6/CFP-10 (Figur 11a, obere Abbildung). Gleichzeitig konnte in PBMC des latent infizierten sowie des gesunden Probanden sowohl nach Stimulation mit Tuberkulin PPD als auch mit ESAT-6/CFP-10 keine signifikant gesteigerte Induktion der 4-1BBL mRNA Produktion beobachtet werden (Figuren 11b und 11c obere Abbildungen). Zudem war in mit PMA/Ionomycin stimulierten Aliquots der PBMC aller drei Spender im Vergleich mit unstimulierten Aliquots der PBMC eine deutlich gesteigerte Produktion der 4-1BBL mRNA zu beobachten, wodurch die Funktionalität der PBMC zur Produktion dieses RTT Markers belegt wurde (Figuren 11a-c, untere Abbildungen).

**Beispiel 9: Induktion der 4-1BB Ligand Expression in PBMC eines gesunden Spenders im zeitlichen Verlauf nach einer Stimulation mit den Kombinationen aus PMA/Ionomycin oder PGE2/anti-CD40**

Zur Identifizierung von Positivkontrollen für das erfindungsgemäße RTT Verfahren zum Nachweis aktivierter T-Zellen wurde untersucht, ob und in welcher Intensität die Kombinationen von PMA/Ionomycin oder PGE2/anti-CD40 eine unspezifische Induktion der 4-1BBL mRNA Expression hervorrufen. Dazu wurden $1 \times 10^6$ PBMC/mL eines gesunden Spenders mit jeweils 1 $\mu$g/mL PMA und Ionomycin oder einer Kombination von 5 $\mu$g/mL PGE$_2$ (Alexis Biochemicals) und einem 2 $\mu$g/mL CD40-Antikörper (LEAF-anti CD40; Biozol) inkubiert. Als Negativkontrolle dienten nicht stimulierte PBMC. $5 \times 10^5$ Zellen wurden nach 0, 2, 4, 6 und 8 Stunden der Inkubation entnommen, pelletiert und sofort in flüssigem Stickstoff eingefroren. Bis zur weiteren Verarbeitung wurden die Zellen bei -80°C aufbewahrt. Wie in Beispiel 1 unter c) beschrieben, wurde anschließend der Gehalt an 4-1BBL mRNA in einer RT-qPCR untersucht.

Diese Untersuchungen zeigen, dass PMA und Ionomycin (Figur 12a) einen wesentlich effizienteren unspezifischen Induktor der 4-1BBL mRNA Produktion darstellen als die Kombination aus PGE$_2$ und dem CD40-Antikörper (Figur 12b).

**Beispiel 10: Vergleichende Bestimmung der relativen Induktion der 4-1BBL mRNA Produktion in nicht stimulierten und mit ESAT-6/CFP-10 stimulierten Kokulturen *in vitro* voraktivierter *M. tuberculosis-spezifischer* Th- Zellen und autologer PBMC unter Verwendung unterschiedlicher Referenzgene**

Zur Identifizierung geeigneter Referenzgene für das erfindungsgemäße RTT Verfahren wurden in den in Beispiel 3 beschriebenen cDNA Proben unter Verwendung der zugehörigen Primer/Sondensysteme die Referenzgene GAPDH, huP0 und PBGD zur Normalisierung der Menge an 4-1BBL mRNA amplifiziert. Hierbei wurden die Primer/Sondensysteme zur Amplifikation der Gene entsprechend den in Beispiel 1 unter c) beschrieben RT-qPCR Bedingungen verwendet.

Hierzu wurden folgende Primer/Sondensysteme verwendet:

huP0 Forward Primer GTGGTGCTGATGGGCAAGA, biomers, dargestellt in SEQ ID NO:10; huP0 Reverse Primer GCAGCAGTTTCTCCAGAGCTG, biomers, dargestellt in SEQ ID NO:11; huP0 Sonde FAM-ACCATGATGCGCAAGGCCATCC-TMR, TIB Molbiol, dargestellt in SEQ ID N0:12; PBGD Forward Primer CCAGCTCCCTGCGAAGAG, biomers, dargestellt in SEQ ID NO: 13; PBGD Reverse Primer CACTGAACTCCTGCTGCTCG, biomers, dargestellt in SEQ ID NO:14; PBGD Sonde FAM-CCCAGCTGCAGAGAAAGTTCCCGC-TMR, TIB Molbiol, dargestellt in SEQ ID NO:15. Ausgewertet wurde nach der $2^{-\Delta\Delta Cq}$ Methode unter Verwendung von GAPDH, huP0 und PBGD als Referenzgen und der unstimulierten Kontrolle als Kalibrator.

Diese Untersuchungen ergaben, dass der Gehalt an 4-1BBL mRNA mit allen drei Referenzgenen normalisiert werden kann, ohne das Endergebnis maßgeblich zu beeinflussen (Figur 13). Obwohl bei der Normalisierung mit den drei Referenzgenen leichte Unterschiede in der Stärke der Induktion der 4-1BBL mRNA beobachtet werden konnten, war die Tendenz vergleichbar.

**Beispiel 11: Durchflusszytometrische Bestimmung der 4-1BB Ligand Expression in Kokulturen ESAT-6/CFP- 10-beladener PBMC und *ex vivo* expandierter ESAT6/CFP-10-spezifischer Th-Zellen**

*In vitro* expandierte aktivierte ESAT-6/CFP-10 spezifische Th-Zellen (Generierung wie in Beispiel 1 unter a)) wurden 1:1 mit autologen PBMC gemischt und in An- oder Abwesenheit von rekombinantem ESAT-6/CFP-10 in einer Konzentration von $1 \times 10^6$ Zellen/ml in R5 für 28 h kokultiviert. Während der letzten 26 h wurde die Proteinsekretion durch Brefeldin A inhibiert. Anschließend wurde die Proteinexpression des 4-1BBL in B-Zellen intrazellulär nach dem Protokoll in Beispiel 1 unter a) markiert und anschließend durchflusszytometrisch bestimmt. Für die Oberflächenfärbung wurden 5 $\mu$l anti-CD19 R-Phycoerythrincyanin 7 (PeCy7) (BD) und für die intrazelluläre Färbung des 4-1BBL 20 $\mu$l anti- 4-1BBL PE (BD) verwendet.

Diese Untersuchungen zeigten, dass bereits unstimulierte B-Zellen einen sehr hohen Gehalt an intrazellulär vorliegendem 4-1BBL aufweisen können, wodurch nach einer Stimulation mit ESAT-6/CFP-10 keine weitere Induktion der 4-1BBL Expression auf Proteinebene festzustellen ist (Figur 2). Diese Untersuchungen belegen, dass die Bestimmung T-Zell-induzierter Reifungsprozesse auf Proteinebene mittels Durchflusszytometrie sowie mittels der ELISA und ELISpot Technologie aufgrund des unterschiedlichen und zum Teil sehr hohen Gehalts des RTT Markers nur mit Einschränkungen möglich ist.

**Beispiel 12: Identifikation von Referenzgenen für das RTT Verfahren zur Normalisierung der RT-qPCR. Hierdurch sollen Variationen im Expressionsniveau in unterschiedlichen Reaktionsansätzen, die nicht durch die biologische Änderung der Expression in der Zelle hervorgerufen werden korrigiert werden.**

Zur Identifizierung von Referenzgenen, die im RTT Verfahren für die Analyse klinischer Proben geeignet sind, wurden PBMC Proben von verschiedenen Probandengruppen unter drei verschiedenen Bedingungen stimuliert und die RT-qPCR Ergebnisse für die Referenzgene mit den Ergebnissen der entsprechenden unstimulierten Probe verglichen. Es wurden Natriumheparin Blutproben von zwei aktiv infizierten TB Patienten, einem latent infizierten TB Patienten, sowie einem gesunden und einem gesunden BCG geimpften Probanden prozessiert. Dazu wurden die PBMC wie in Beispiel 1 unter a) angegeben isoliert. Anschließend wurden 6x $10^6$ PBMC je 6 ml Ansatz in B-Zell Medium stimuliert. Die Stimulationen erfolgten entweder mit ESAT6/CFP10 (10 $\mu$g/ml), Tuberkulin PPD (10 $\mu$g/ml) oder unspezifisch mit PMA/Ionomycin (jeweils 1 $\mu$g/ml) über einen Zeitraum von 6 h in befeuchteter Atmosphäre bei 37°C und 5 % $CO_2$. Nach der Inkubation wurden die Zellen bei 300 x g 10 min pelletiert, der Überstand verworfen, das Pellet in RLT Puffer (QIAGEN) mit 1 % $\beta$-Mercaptoethanol lysiert und sofort in flüssigem Stickstoff eingefroren. Die RNA wurde mit dem RNeasy Mini Kit (QIAGEN) einschließlich eines DNase Verdaus auf der Säule nach Herstelleranweisung isoliert. Die cDNA Synthese wurde mit dem QuantiTect Reverse Transkription Kit von QIAGEN durchgeführt. Die qPCR wurde mit Custom TaqMan® Gene Expression Array 96-Well Fast Plates (Applied Biosystems) und dem TaqMan Fast Universal Master Mix (Applied Biosystems) auf einem ABI StepOnePlus Real Time PCR System (Applied Biosystems) entsprechend den Angaben des Herstellers durchgeführt.

Das Expressionsniveau eines gut geeigneten Referenzgens für eine RT-qPCR sollte unter allen verwendeten experimentellen Bedingungen und den darin vorgesehenen Stimulationen konstant sein. Daneben sollte die Höhe der Expression des Referenzgens dem Expressionsniveau der Zielgene entsprechen. Auch die qPCR Effizienz sollte der der Zielgene ähnlich sein. Für alle fünf Proben wurden die Ergebnisse verglichen und ausgewertet.

Das Ergebnis ist in Figur 14 dargestellt. Die Expression der RTT Markergene *TNFSF9* und *IFNG* für die unterschiedlichen Stimulationsbedingungen wurde zwischen Cq 23 und Cq 30 detektiert (nicht gezeigt). Die Gene *TAF1A* und *HMBS* eignen sich aufgrund der konstanten Expression für alle Stimulationsbedingungen sowie aufgrund der Höhe der Expression bei Cq 28-Cq 29 und aufgrund der ähnlichen Effizienz der RT-qPCR im Vergleich zu der der Zielgene (nicht gezeigt) als Referenzgene für die RTT Marker RNA von *4-1BBL* -auch *TNFSF9* genannt- und *IFN-$\gamma$* -auch *IFNG* genannt. Für alternative Marker mit höheren Expressionen kommen *HPRT1* oder *RPLP0* als geeignete Referenzgene in Frage.

Insgesamt konnten durch diese Untersuchungen somit mehrere geeignete Referenzgene identifiziert werden, deren Expressionsniveau für alle experimentellen Bedingungen konstant bleibt. Je nach Expressionsstärke der RTT Markergene können *TAF1A, HMBS* für niedrige Expression, *HPRT1* für eine mittlere Expression oder *RPLP0* für eine hohe Expression als Referenzgene verwendet werden. Für die Marker *TNFSF9* und *IFNG* ist *TAF1A* als Referenzgen geeignet.

**Beispiel 13: Bestimmung der *4-1BBL* -auch *TNFSF9* genannt- Expression in Tuberkulin PPD stimulierten PBMC im Vergleich zu nicht stimulierten Proben von unterschiedlichen Probanden zur Ermittlung der Hintergrundexpression der RTT Marker und eines Grenzwertes für die Diskriminierung von Patienten mit einer aktiven TB Infektion und Gesunden**

An Blutproben von 21 Probanden wurden das erfindungsgemäße RTT Verfahren durchgeführt, um Informationen über die durchschnittliche Hintergrundexpression des RTT Markers *4-1BBL* -auch *TNFSF9* genannt- in Gesunden und Patienten mit einer latenten TB zu erhalten. Dazu wurden die PBMC wie in Beispiel 1 unter a) angegeben isoliert. Je Ansatz wurden 5 x $10^6$ PBMC in B-Zellmedium in An- und Abwesenheit von 10 $\mu$g/ml Tuberkulin PPD über einen Zeitraum von 6 h in befeuchteter Atmosphäre bei 37°C und 5 % $CO_2$ inkubiert. Die Stimulationen wurden in Duplikaten angesetzt und unabhängig prozessiert. Nach der Inkubation wurden die Zellen pelletiert, in RLT Puffer (QIAGEN GmbH, Hilden, Deutschland) mit 40 mM DTT lysiert und das Lysat sofort in flüssigem Stickstoff eingefroren. Die RNA wurde mit dem RNeasy Mini Kit von QIAGEN einschließlich eines DNase Verdaus auf der Säule nach Herstelleranweisung isoliert. cDNA Synthesen wurde mit jeweils 1 $\mu$g RNA mit dem QuantiTect Reverse Transkription Kit (QIAGEN) nach den Angaben des Herstellers durchgeführt. Die qPCR wurde mit 1 $\mu$l cDNA in 10 $\mu$l Gesamtreaktionsvolumen unter Verwendung des TaqMan® Fast Universal PCR Master Mix von Applied Biosystems durchgeführt. Der Mastermix hatte folgende Zusammensetzung: 300 nM *TNFSF9* Forward Primer GAGGGTCCCGAGCTTTCG, dargestellt in SEQ ID NO:1; 300 nM *TNFSF9* Reverse Primer GCCCATCGATCAGCAGAAC, dargestellt in SEQ ID NO:2; 200 nM *TNFSF9* Sonde FAM-CCACCAGCTGCGCAAACATGC-TMR, dargestellt in SEQ ID NO:3; 1x TaqMan® Fast Universal PCR Master Mix, 1x TaqMan® Gene Expression Assay TAF1A VIC dye labeled MGB Probe (primer limited) von Applied Biosystems. Das Temperaturprofil der qPCR umfasste 20 s Denaturierung bei 95°C und anschließend 40 Zyklen bestehend aus 3 s bei 95°C und 30 s bei 60°C. Die qPCR wurde auf dem StepOnePlus Real-Time PCR System (Applied Biosystems) durchgeführt. In Figur 15 sind die Mittelwerte der Ergebnisse aus jeweils zwei unabhängigen Stimulationen der vergleichenden Cq Analyse ($2^{-\Delta\Delta Cq}$) für die 21 Proben dargestellt.

Diese Untersuchungen zeigen, dass die relative Expressionserhöhung des RTT Markers *TNFSF9* nach Stimulation für Probanden mit einer latenten TB, Gesunde und BCG geimpfte Gesunde unter oder maximal bei 2-fach lag, während aktiv infizierte TB Patienten eine Expressionserhöhung von größer 2-fach zeigten. Dadurch konnte ein vorläufiger Grenzwert von $2^{-\Delta\Delta Cq} = 2{,}0$ festgelegt werden. Eine Differenzierung von aktiv infizierten TB Patienten und Gesunden bzw. Probanden mit einer latenten TB mit dem RTT Verfahren ist somit möglich.

**Beispiel 14: Verbesserung der Expression von *IFN-γ* -auch *IFNG* genannt- durch den Einsatz von alternativen zum Teil synthetischen serumfreien Medien**

Um zu untersuchen, ob sich das *IFN-γ* -auch *IFNG* genannt- Signal durch Verwendung von alternativen Zellkulturmedien steigern lässt wurden mehrere zum Teil synthetische Medien getestet. Das herkömmlich verwendete B-Zellmedium enthält IL-4, das zur Inhibition des IFNG Signals führen könnte. Darüber hinaus enthält das Medium AB Serum aus Eigenproduktion, wodurch Standardbedingungen, also gleichbleibende Bedingungen ohne Qualitätsschwankungen, nicht optimal eingehalten werden könnten. Für Blutproben von drei Probanden mit einer latenten TB und von drei Gesunden wurde das erfindungsgemäße RTT Verfahren durchgeführt. Dazu wurden die PBMC wie in Beispiel 1 unter a) angegeben isoliert. Je Ansatz wurden 5x $10^6$ PBMC in 2,5 ml der unterschiedlichen Zellkulturmedien in An- bzw. Abwesenheit von 10 µg/ml Tuberkulin PPD 6 h in befeuchteter Atmosphäre bei 37°C und 5 % $CO_2$ inkubiert. Es wurden folgende Medien getestet: B-Zellmedium (BZM+), B-Zellmedium ohne IL-4 (BZM-), serumfreies UltraCULTURE™ Medium (Ultra) von LONZA und AIM V Medium (AIMV) von Invitrogen. Die Zelllyse, RNA Extraktion, cDNA Synthese und qPCR wurde wie in Beispiel 13 beschrieben durchgeführt. Die Expressionszunahme wurde für *IFNG* und *4-1BBL* -auch *TNFSF9* genannt- ermittelt (siehe Figur 16 und Figur 17). Der Mastermix für *IFNG* hatte folgende Zusammensetzung: 300 nM *IFNG* Forward Primer GTGGAGACCATCAAGGAAGACAT (biomers), dargestellt in SEQ ID NO:7; 300 nM *IFNG* Reverse Primer GGCGACAGTTCAGCCATCA (biomers), dargestellt in SEQ ID NO:8; 200 nM *IFNG* Sonde FAM-TTCATGTATTGCTTTGCGTTGGACATTCAA-TMR (TIB MOLBIOL), dargestellt in SEQ ID NO:9; 1x TaqMan® Fast Universal PCR Master Mix, 1x TaqMan® Gene Expression Assay TAF1A VIC dye labeled MGB Probe (primer limited) von Applied Biosystems. Der Mastermix für *TNFSF9* hatte folgende Zusammensetzung: 300 nM *TNFSF9* Forward Primer GAGGGTCCCGAGCTTTCG (biomers), dargestellt in SEQ ID NO:1; 300 nM *TNFSF9* Reverse Primer GCCCATCGATCAGCAGAAC (biomers), dargestellt in SEQ ID NO:2; 200 nM *TNFSF9* Sonde FAM-CCACCAGCTGCGCAAACATGC-TMR (TIB MOLBIOL), dargestellt in SEQ ID NO:3; 1x TaqMan® Fast Universal PCR Master Mix, 1x TaqMan® Gene Expression Assay TAF1A VIC dye labeled MGB Probe (primer limited) von Applied Biosystems. Das Temperaturprofil der qPCR umfasste 20 s Denaturierung bei 95°C und anschließend 40 Zyklen bestehend aus 3 s bei 95°C und 30 s bei 60°C. Die qPCR wurde auf dem StepOnePlus Real-Time PCR System (Applied Biosystems) durchgeführt.

Die relative *IFNG* Expressionszunahme ($2^{-\Delta\Delta Cq}$) war nach Stimulation sowohl in UltraCULTURE™ als auch in AIM V Medium deutlich stärker als im herkömmlichen BZM+. Das Entfernen von IL-4 aus dem B-Zellmedium (BZM-) führte zu keiner Signalverstärkung (Figur 16). Eine relative Expressionszunahme von *TNFSF9* in Proben von Patienten mit einer latenten TB und in gesunden Probanden konnte für die Stimulation in UltraCULTURE™ Medium nicht detektiert werden. Dagegen führte die Stimulation in AIM V Medium zu einer starken unspezifischen Induktion der *TNFSF9* RNA Expression im Vergleich zu den B-Zellmedien bzw. dem UltraCULTURE™ Medium (Figur 17).

Diese Untersuchungen zeigen, dass die Wahl des Mediums einen starken Einfluss auf die relative Expression von RTT Markergenen haben kann. Die Verwendung des serumfreien UltraCULTURE™ Mediums von LONZA führt zu einer deutlichen Verstärkung des *IFNG* Signals in Proben von Probanden mit einer latenten TB bei konstanter Hintergrundexpression des RTT Markergens *TNFSF9.*

**Beispiel 15: Synchrone Detektion von zwei RTT Markergenen bei gleichzeitiger Normalisierung gegen ein Referenzgen unter Einsatz einer Triplex qPCR**

Zur Vereinfachung und Verbesserung der Praktikabilität sowie der Erweiterung des Anwendungsbereichs des erfindungsgemäßen RTT Verfahrens wurde eine Triplex RT-qPCR entwickelt, die es ermöglicht in einem Reaktionsansatz den RTT Marker *4-1BBL* -auch *TNFSF9* genannt- und den Marker für den Nachweis einer latenten TB Infektion *IFN-γ* -auch *IFNG* genannt- zeitgleich nachzuweisen und zusätzlich die Signale mit Hilfe eines Referenzgens zu normalisieren. Eine Probe eines Probanden mit einer latenten TB wurde dem erfindungsgemäßen Verfahren entsprechend, wie in Beispiel 12 beschrieben, prozessiert. Die qPCR wurde mit 1 µl cDNA in 10 µl Gesamtreaktionsvolumen unter Verwendung des TaqMan® Fast Universal PCR Master Mix von Applied Biosystems durchgeführt. Der Mastermix hatte folgende Zusammensetzung: 150 nM *TNFSF9* Forward Primer GAGGGTCCCGAGCTTTCG (biomers), dargestellt in SEQ ID NO:1; 150 nM *TNFSF9* Reverse Primer GCCCATCGATCAGCAGAAC (biomers), dargestellt in SEQ ID NO:2; 200 nM *TNFSF9* Sonde FAM-CCACCAGCTGCGCAAACATGC-BBQ (TIB MOLBIOL), dargestellt in SEQ ID NO:3; 300 nM *IFNG* Forward Primer GTGGAGACCATCAAGGAAGACAT (biomers), dargestellt in SEQ ID NO:7; 300 nM *IFNG* Reverse Primer GGCGACAGTTCAGCCATCA (biomers), dargestellt in SEQ ID NO:8; 200 nM *IFNG* Sonde BoTMR-TTCATGTATTGCTTTGCGTTGGACATTCAA-BBQ (TIB MOLBIOL), dargestellt in SEQ ID NO:9; 1x TaqMan® Fast Universal PCR Master Mix, 1x TaqMan® Gene Expression Assay TAF1A VIC dye labeled MGB Probe (primer limited) von Applied Biosystems. Das Temperaturprofil der qPCR umfasste 20 s Denaturierung bei 95°C und anschließend 40 Zyklen bestehend aus 3 s bei 95°C und 30 s bei 60°C. Die qPCR wurde auf dem StepOnePlus Real-Time PCR System (Applied Biosystems) durchgeführt. In Figur 18 sind die Ergebnisse der vergleichenden Cq Analyse ($2^{-\Delta\Delta Cq}$) für eine Probe eines Probanden mit einer latenten TB dargestellt. Es sind die Ergebnisse der Triplex (links) und als Vergleich die Ergebnisse der entsprechenden Duplex qPCRs (rechts) gezeigt. Die Ansätze für die Duplex qPCR wurden wie in Beispiel 14 beschrieben hergestellt.

Diese vergleichende Analyse zeigt, dass die Detektion von RTT Markern prinzipiell in einer Multiplex Reaktion mit gleichzeitiger Normalisierung der Werte durch ein Referenzgen möglich ist.

**Beispiel 16: Bestimmung der Expression von weiteren Markergenen für das erfindungsgemäße RTT Verfahren in Tuberkulin PPD stimulierten PBMC im Vergleich zu nicht stimulierten Proben von zwei Probanden**

Um eine höhere Sensitivität des erfindungsgemäßen RTT Verfahrens zu erreichen, wurden weitere Markergene in einer RT-qPCR mit dem SYBR Green System getestet. Hierzu wurden jeweils eine Probe eines Patienten mit einer latenten TB und eines gesunden Probanden dem erfindungsgemäßen Verfahren entsprechend, wie in Beispiel 12 beschrieben, prozessiert. Die qPCR wurde mit 1 μl cDNA in 20 μl Gesamtreaktionsvolumen unter Verwendung des Brilliant III Ultra-Fast SYBR Green QPCR Master Mix von Agilent Technologies durchgeführt. Der Mastermix hatte folgende Zusammensetzung: 300 nM forward Primer, 300 nM reverse Primer, 1x Brilliant III Ultra-Fast SYBR Green QPCR Master Mix, 300 nM reference dye. Folgende Primerpaare wurden verwendet: FCGR1A/B/C Forward Primer GAAGGGGTGCACCGGAA (TIB MOLBIOL), dargestellt in SEQ ID NO:16; FCGR1A/B/C Reverse Primer CTCACGGGGAGCAAGTGG (TIB MOLBIOL), dargestellt in SEQ ID NO:17; CXCL9 Forward Primer GAGTGCAAGGAACCCCAGTAGT, dargestellt in SEQ ID NO:18; CXCL9 Reverse Primer GGTGGATAGTCCCTTGGTTGGT, dargestellt in SEQ ID NO:19; CXCL10 Forward Primer TCCACGTGTTGAGATCATTGC, dargestellt in SEQ ID NO:20; CXCL10 Reverse Primer TCTTGATGGCCTTCGATTCTG, dargestellt in SEQ ID NO:21; CXCL11 Forward Primer CAAGGCTTCCCCATGTTCA, dargestellt in SEQ ID NO:22; CXCL11 Reverse Primer CCCAGGGCGTATGCAAAGA, dargestellt in SEQ ID NO:23 (alle CXCL Primer aus Doktorarbeit Theresa Knoblach, 2010, Das Cytomegalievirus IE1-Protein als Regulator des humanen Transkriptoms und Zielstruktur RNAi-basierter Therapiestrategien, Universität Regensburg). Das Temperaturprofil der qPCR umfasste 3 min Denaturierung bei 95°C und anschließend 40 Zyklen bestehend aus 5 s bei 95°C und 10 s bei 60°C. Die qPCR wurde auf dem StepOnePlus Real-Time PCR System (Applied Biosystems) durchgeführt. In Figur 19 sind die Ergebnisse der vergleichenden Cq Analyse ($2^{\Delta Cq}$) für beide Proben dargestellt.

Diese vergleichende Analyse von stimulierten und nicht stimulierten Proben von einem latent infizierten TB Patienten bzw. einem Gesunden deutet darauf hin, dass sich diese Gene als Marker für den Nachweis von latenten TB Erkrankungen eignen könnten.

**Patentansprüche**

1.  Verfahren zum Nachweis, Differenzieren und Quantifizieren von T-Zellpopulationen, umfassend die folgenden Schritte:

    a) in Kontakt Bringen eines ersten Aliquots einer Körperflüssigkeit eines Individuums mit mindestens einem Antigen, wobei die Körperflüssigkeit antigenpräsentierende Zellen (APC) und T-Zellen enthält,
    b) Inkubieren des ersten Aliquots mit dem mindestens einen Antigen über einen bestimmten Zeitraum,
    c) Nachweis und Differenzieren der T-Zellpopulationen durch Detektieren mindestens eines ersten durch die T-Zellen einer bestimmten T-Zellpopulation induzierten Markers der APC in dem ersten Aliquot und in einem zweiten Aliquot der Körperflüssigkeit des Individuums, das nicht mit dem mindestens einen Antigen inkubiert wurde, durch Reverse Transkription quantitative Real-Time Polymerase Kettenreaktion (RT-qPCR), und
    d) Nachweis und Quantifizieren der T-Zellpopulationen durch Bestimmen des Verhältnisses des detektierten Markers der APC des ersten Aliquots zu dem zweiten Aliquot.

2.  Verfahren nach Anspruch 1, wobei in Schritt c) zusätzlich mindestens ein zweiter Marker in dem ersten und in dem zweiten Aliquot detektiert wird, wobei der zweite Marker ein induzierter Marker der T-Zellen selbst ist, und Schritt d) den Nachweis und das Quantifizieren der T-Zellpopulationen durch Bestimmen des Verhältnisses des detektierten ersten Markers der APC und des zweiten Markers der T-Zelle des ersten Aliquot zu dem zweiten Aliquot umfasst.

3.  Verfahren nach Anspruch 1 oder 2, wobei das Verfahren in Schritt a) einen weiteren Schritt a') in Kontakt Bringen des zweiten Aliquots mit mindestens einem Antigen, und in Schritt b) einen weiteren Schritt b') Inkubieren des zweiten Aliquots mit dem Antigen über einen bestimmten Zeitraum, wobei sich der Zeitraum in Schritt b') von dem Zeitraum in Schritt b) unterscheidet, und statt Schritt c) einen Schritt c') Nachweis und Differenzieren der T-Zellpopulationen durch Detektieren des ersten Markers in dem ersten und zweiten Aliquot durch RT-qPCR, und Schritt d) umfasst.

4.  Verfahren nach einem der Ansprüche 1 bis 3, wobei der Schritt c') den Nachweis und das Differenzieren der T-Zellpopulationen durch das Detektieren des ersten und des zweiten Markers umfasst.

5.  Verfahren nach einem der Ansprüche 1 bis 4, wobei das Aliquot der Körperflüssigkeit in ein nur APC enthaltendes Aliquot A und in ein T-Zellen enthaltendes Aliquot B aufgetrennt wird, und wobei Schritt a) einen Schritt a1) in Kontakt Bringen des Aliquots A mit mindestens einem Antigen, und einen darauf folgenden Schritt a2) in Kontakt Bringen des mit dem mindestens einen Antigen in Kontakt gebrachten Aliquots A mit dem Aliquot B umfasst.

6.  Verfahren nach einem der Ansprüche 1 bis 5, wobei die T-Zellpopulationen naive T-Zellen, aktivierte T-Zellen oder Gedächtnis-T-Zellen enthalten.

7. Verfahren nach einem der Ansprüche 1 bis 6, wobei die T-Zellen der T-Zellpopulationen CD4$^+$ T-Zellen, Th-1 Zellen, Th-2 Zellen, Th-17 Zellen, CD4$^+$CD25$^+$ regulatorische T-Zellen, Th-3 Zellen, CD8$^+$ T-Zellen, CD4$^-$CD8$^+$ zytotoxische T-Zellen, CD4$^+$CD8$^+$ T-Zellen, CD161$^+$ NKT-Zellen und/oder ein Gemisch verschiedener T-Zellen sind.

8. Verfahren nach einem der Ansprüche 1 bis 7, wobei die Körperflüssigkeit Blut, Liquor, Lymphe, Perikardflüssigkeit, eine Bronchiallavage, eine Knochenmarkpunktion, eine Suspension von lymphatischem Gewebe oder eine gereinigte PBMC-Population ist.

9. Verfahren nach einem der Ansprüche 1 bis 8, wobei das Antigen ein Peptid, Oligopeptid, ein Polypeptid, ein Protein, eine RNA oder eine DNA ist.

10. Verfahren nach einem der Ansprüche 1 bis 9, wobei das Antigen ein Antigen aus einem Bakterium, Virus, Pflanze, Tier, Pilz oder Parasit ist und/oder das Antigen ausgewählt ist aus der Gruppe bestehend aus PSA, HER-2/neu, Mucin-1, MAGE, CEA, Myelin-basisches Protein (MBP), Myelin Oligodendrocyten Glykoprotein (MOG), Myelin Proteolipid Protein (PLP), Myelin, Insulin B, Pre-Pro-Insulin, IA-2, GAD65, Hsp60, ESAT-6, CFP-10, TB7.7, TB37.6, MPT63, Tuberkulin PPD, VlsE, p58 (BBQ03), p14, p21-24 (OspC), p37-38 (FlaA), p41, p19 (OspE), p18, Crasp3, BB0323, p26 (OspF), p28 (OspD), p30, p39 (BmpA), p60-65 (common antigen, Hsp60), p83-100, p17 (Osp17), p31-32 (OspA), p34 (Osp B), Borrelienlipide, ein Lysat von Borreleinestämmen, Pr55$^{gag}$, p24, p17, POL, RT, nef, pp65, IE1, IE2, BZLF1, EBNA3, EBNA2, EBNA6, BMLF1, EBNA1, ORF1, ORF4, PRF62, ORF68, HBsAg, HBcAg und AdV5.

11. Verfahren nach einem der Ansprüche 1 bis 10, wobei der Zeitraum des in Kontakt Bringens in Schritt a) und des Inkubierens in Schritt b) von 0 Stunden bis 72 Stunden, bevorzugt 4, 6 oder 8 Stunden, beträgt.

12. Verfahren nach einem der Ansprüche 1 bis 11, wobei der erste Marker der APC bzw. der zweite Marker der T-Zelle eine Nukleinsäure, insbesondere eine RNA, eine DNA oder ein Nukleinsäurefragment ist und durch das in Kontakt Bringen und Inkubieren mit dem mindestens einen Antigen induziert wird.

13. Verfahren nach einem der Ansprüche 1 bis 12, wobei der Marker der APC 4-1BB Ligand (4-1BBL), OX40 Ligand (OX40L), TNFSF (CD70), B7.1 (CD80), B7.2 (CD86), Fc$\gamma$RIII (CD16), Fc$\gamma$RII (CD32), Fc$\gamma$RI (CD64) oder ein weiterer Vertreter der TNF/TNF-Rezeptor oder Immunglobulin Superfamilie ist und/oder der Marker der T-Zelle IFN-$\beta$, INF-$\gamma$, TNF-$\alpha$, IL-2, IL-4, IL-5, IL-6, IL-10, IL-12, IL-13, GM-CSF, TGF-$\beta$, MIP1a, MIP1b, 4-1BB, CD25, Perforin und/oder Granzym ist.

14. Verfahren nach einem der Ansprüche 1 bis 13, wobei der Nachweis und das Quantifizieren in Schritt c) und d) zusätzlich durch PCR, quantitative PCR (qPCR), Microarray, FACS, ELISpot oder ELISA durchgeführt wird.

**Claims**

1. Method for the detection, differentiation and quantification of T cell populations, comprising the following steps:

   a) contacting a first aliquot of a body fluid of an individual with at least one antigen, wherein the body fluid contains antigen presenting cells (APC) and T cells,
   b) incubating the first aliquot with the at least one antigen over a certain period of time,
   c) detection and differentiation of the T cell population by detecting in the first aliquot and in a second aliquot of the body fluid of the individual, which has not been incubated with the at least one antigen, at least a first marker of the APC induced by T cells in a specific T cell population using reverse transcription quantitative real-time polymerase chain reaction (RT-qPCR), and
   d) detection and quantification of the T cell population by determining the ratio of the detected marker of the APC of the first aliquot to the second aliquot.

2. Method according to claim 1, wherein in step c) additionally at least a second marker is detected in the first and in the second aliquot, wherein the second marker is an induced marker of the T cells themselves, and step d) comprises the detection and quantification of the T cell population by determining the ratio of the detected first marker of the APC and the second marker of the T cell in the first aliquot to the second aliquot.

3. Method according to claim 1 or 2, wherein the method comprises in step a) a further step a') contacting the second aliquot with at least one antigen, and comprises in step b) a further step b') incubating the second aliquot for a certain period of time, wherein the period of time in step b') is different from the period of time in step b), and comprises instead of step c) a step c') detection and differentiation of the T cell population by detecting the first marker in the first and second aliquot by RT-qPCR, and comprises step d).

4. Method according to anyone of claims 1 to 3, wherein step c') comprises the detection and differentiation of the T cell populations by detecting the first and the second marker.

5. Method according to anyone of claims 1 to 4, wherein the aliquot of the body fluid is separated into an aliquot A containing only APCs and into an aliquot B containing T cells, and wherein step a) comprises a step a1) contacting the aliquot A with at least one antigen, and a subsequent step a2) contacting the aliquot A contacted with the at least one antigen with aliquot B.

6. Method according to anyone of claims 1 to 5, wherein the T cell populations contain naive T cells, activated T cells or memory T cells.

7. Method according to anyone of claims 1 to 6, wherein the T cells of the T cell populations are CD4$^+$ T cells, Th-1 cells, Th-2 cells, Th-17 cells, CD4$^+$CD25$^+$ regulatory T cells, Th-3 cells, CD8$^+$ T cells, CD4$^-$CD8$^+$ cytotoxic T cells, CD4$^+$CD8$^+$ T cells, CD161$^+$ NKT cells and/or a mixture of various T cells.

8. Method according to anyone of claims 1 to 7, wherein the body fluid is blood, cerebrospinal fluid, lymph, pericardial fluid, a bronchial lavage, a bone marrow aspirate, a suspension of lymphatic tissue or a purified PBMC population.

9. Method according to anyone of claims 1 to 8, wherein the antigen is a peptide, oligopeptide, a polypeptide, a protein, a RNA or a DNA.

10. Method according to anyone of claims 1 to 9, wherein the antigen is an antigen of a bacterium, virus, plant, animal, fungi or parasite and/or the antigen is selected from the group consisting of PSA, HER-2/neu, Mucin-1, MAGE, CEA, myelin basic protein (MBP), myelin oligodendrocytes glycoprotein (MOG), myelin proteolipid protein (PLP), myelin, insulin B, preproinsulin, IA-2, GAD65, Hsp60, ESAT-6, CFP-10, TB7.7, TB37.6, MPT63, tuberculin PPD, VlsE, p58 (BBQ03), p14, p21-24 (OspC), p37-38 (FlaA), p41, p19 (OspE), p18, Crasp3, BB0323, p26 (OspF), p28 (OspD), p30, p39 (BmpA), p60-65 (common antigen, Hsp60), p83-100, p17 (Osp17), p31-32 (OspA), p34 (Osp B), borrelia lipids, a lysate of borrelia strains, Pr55$^{gag}$, p24, p17, POL, RT, nef, pp65, IE1, IE2, BZLF1, EBNA3, EBNA2, EBNA6, BMLF1, EBNA1, ORF1, ORF4, PRF62, ORF68, HBsAg, HBcAg and AdV5.

11. Method according to anyone of claims 1 to 10, wherein the period of time for contacting in step a) and incubation in step b) is 0 hours to 72 hours, preferably 4, 6 or 8 hours.

12. Method according to anyone of claims 1 to 11, wherein the first marker of the APC and the second marker of the T cell, respectively, is a nucleic acid, in particular a RNA, a DNA, a nucleic acid fragment and is induced by said contacting and incubating with the at least one antigen.

13. Method according to anyone of claims 1 to 12, wherein the marker of the APC is 4-1BB ligand (4-1BBL), OX40 ligand (OX40L), TNFSF (CD70), B7.1 (CD80), B7.2 (CD86), FcγRIII (CD16), FcγRII (CD32), FcγRI (CD64) or a further representative of the TNF/TNF receptor or immunoglobulin superfamily and/or the marker of the T cell is INF-β, INF-γ, TNF-α, IL-2, IL-4, IL-5, IL-6, IL-10, IL-12, IL-13, GM-CSF, TGF-β, MIP1a, MIP1b, 4-1BB, CD25, perforin and/or granzyme.

14. Method according to anyone of claims 1 to 13, wherein the detection and the quantification in step c) and d) is performed additionally by using PCR, quantitative PCR (qPCR), microarray, FACS, ELISpot or ELISA.

**Revendications**

1. Procédé pour la détection, la différenciation et la quantification de populations de lymphocytes T, comprenant les étapes suivantes :

a) la mise en contact d'une première aliquote d'un fluide corporel d'un individu avec au moins un antigène, dans lequel le fluide corporel contient des cellules présentatrices d'antigène (CPA) et des lymphocytes T,
b) l'incubation d'une première aliquote avec au moins un antigène pendant une durée déterminée,
c) la détection et la différenciation des populations de lymphocytes T grâce à la détection d'au moins un premier marqueur des CPA induit par des lymphocytes T d'une population déterminée de lymphocytes T dans la première aliquote et dans une deuxième aliquote du fluide corporel de l'individu, qui n'a pas été incubée avec le au moins un antigène, par réaction en chaîne polymérase quantitative en temps réel par transcription inverse (RT-qPCR), et
d) la détection et la quantification des populations de lymphocytes T grâce à la détermination du rapport du marqueur détecté des CPA de la première aliquote à la deuxième aliquote.

2. Procédé selon la revendication 1, dans lequel, à l'étape c), au moins un deuxième marqueur est en outre détecté dans la première et la deuxième aliquote, dans lequel le deuxième marqueur est lui-même un marqueur induit des lymphocytes T, et l'étape d) comprend la détection et la quantification des populations de lymphocytes T par la détermination du rapport du premier marqueur détecté des CPA et du deuxième marqueur des lymphocytes T de la première aliquote à la deuxième aliquote.

3. Procédé selon la revendication 1 ou 2, dans lequel le procédé à l'étape a) comprend une étape ultérieure a') de mise en contact de la deuxième aliquote avec au moins un antigène, et à l'étape b), une étape ultérieure b') d'incubation de la deuxième aliquote avec l'antigène pendant une durée déterminée, la durée de l'étape b') étant différente de la durée de l'étape b), et une étape c') à la place de l'étape c) comprenant la détection et la différenciation des populations de lymphocytes T grâce à la détection du premier marqueur dans la première aliquote et la deuxième aliquote par RT-qPCR, et l'étape d).

4. Procédé selon l'une des revendications 1 à 3, dans lequel l'étape c') comprend la détection et la différenciation des populations de lymphocytes T grâce à la détection du premier et du deuxième marqueurs.

5. Procédé selon l'une des revendications 1 à 4, dans lequel l'aliquote de fluide corporel est divisée en une aliquote A contenant seulement les CPA et en une aliquote B contenant des lymphocytes T et dans lequel l'étape a) comprend une étape a1) de mise en contact de l'aliquote A avec au moins un antigène, et une étape immédiatement consécutive a2) de mise en contact de l'aliquote A ayant été mise en contact avec le au moins un antigène avec l'aliquote B.

6. Procédé selon l'une des revendications 1 à 5, dans lequel les populations de lymphocytes T contiennent des lymphocytes T naïfs, des lymphocytes T activés ou des lymphocytes T à mémoire.

7. Procédé selon l'une des revendications 1 à 6, dans lequel les lymphocytes T des populations de lymphocytes T sont des lymphocytes T CD4$^+$, des lymphocytes T Th-1, des lymphocytes T Th-2, des lymphocytes T Th-17, des lymphocytes T régulateurs CD4$^+$CD25$^+$, des lymphocytes T Th-3, des lymphocytes T CD8$^+$, des lymphocytes T cytotoxiques CD4$^-$CD8$^+$, des lymphocytes CD4$^+$ CD8$^+$, des lymphocytes NKT CD161$^+$ et/ou un mélange de différents lymphocytes T.

8. Procédé selon l'une des revendications 1 à 7, dans lequel le fluide corporel est le sang, le liquide céphalo-rachidien, la lymphe, le fluide péricardiaque, un lavage bronchique, une ponction de moelle osseuse, une suspension de tissus lymphatiques ou une population de PBMC purifiée.

9. Procédé selon l'une des revendications 1 à 8, dans lequel l'antigène est un peptide, un oligopeptide, un polypeptide, une protéine, un ARN ou un ADN.

10. Procédé selon l'une des revendications 1 à 9, dans lequel l'antigène est un antigène provenant d'une bactérie, d'un virus, d'un végétal, d'un animal, d'un champignon ou d'un parasite et/ou l'antigène est choisi dans le groupe constitué de PSA, HER-2/neu, mucine-1, MAGE, CEA, protéine à base de myéline (MBP), glycoprotéine d'oligodendrocytes de myéline (MOG), protéine de protéolipide de myéline (PLP), myéline, insuline B, pré-pro-insuline, IA-2, GAD65, Hsp60, ESAT-6, CFP-10, TB7.7, TB37.6, MPT63, tuberculine PPD, VlsE, p58 (BBQ03), p14, p21-24 (OspC), p37-38 (FlaA), p41, p19 (OspE), p18, Crasp3, BB0323, p26 (OspF), p28 (OspD), p30, p39 (BmpA), p60-65 (antigène commun, Hsp60), p83-100, p17 (Osp17), p31-32 (OspA), p34 (Osp B), lipides de borrélies, un lysat de souches de borrélies, Pr55$^{gag}$, p24, p17, POL, RT, nef, pp65, IE1, IE2, BZLF1, EBNA3, EBNA2, EBNA6, BMLF1, EBNA1, ORF1, ORF4, PRF62, ORF68, HBsAg, HBcAg et AdV5.

**11.** Procédé selon l'une des revendications 1 à 10, dans lequel la durée de la mise en contact à l'étape a) et de l'incubation à l'étape b) est de 0 heure à 72 heures, de préférence de 4, 6 ou 8 heures.

**12.** Procédé selon l'une des revendications 1 à 11, dans lequel le premier marqueur des CPA ou le deuxième marqueur des lymphocytes T est un acide nucléique, en particulier un ARN, un ADN ou un fragment d'acide nucléique, et est induit par la mise en contact et l'incubation avec le au moins un antigène.

**13.** Procédé selon l'une des revendications 1 à 12, dans lequel le marqueur des CPA est le ligand 4-1BB (4-1BBL), le ligand OX40 (OX40L), le TNFSF (CD70), B7.1 (CD80), B7.2 (CD86), FcγRIII (CD16), FcγRII (CD32), FcγRI (CD64), ou un autre représentant des récepteurs TNF/TNF ou de la superfamille des globulines, et/ou le marqueur des lymphocytes T est IFN-β, IFN-γ, TNF-α, IL-2, IL-4, IL-5, IL-6, IL-10, IL-12, IL-13, GM-CSF, TGF-β, MIP1a, MIP1b, 4-1BB, CD25, la perforine et/ou la granzyme.

**14.** Procédé selon l'une des revendications 1 à 13, dans lequel la détection et la quantification à l'étape c) et d) sont réalisées en plus par PCR, PCR quantitative (qPCR), micropuce, FACS, ELISpot ou ELISA.

# Figur 1 A

Proliferation

Produktion
von
Markerzytokinen

Gedächtnis
Th Zelle

TCR   MHC-II
Peptid

APC

Krankheits /
Erregerspezifisches
Polypeptid

# Figur 1 B

aktivierte
Th Zelle

RTT

TCR   MHC-II
Peptid

APC

gesteigerte
Expression der
Marker mRNA
(Reverse Transkriptase
Relative Quantitative
PCR)

Krankheits /
Erregerspezifisches
Polypeptid

# Figur 2

**Isotyp IgG1**

**4-1BBL
intrazellulär**

unstimuliert

**Isotyp IgG1**

**4-1BBL
intrazellulär**

spezifisch
stimuliert

# Figur 3

**Figur 4**

## Figur 5 A

## Figur 5 B

# Figur 6

Legend:
- ESAT-6/CFP-10-beladene PBMC
- ESAT-6/CFP-10-beladene PBMC plus 210 voraktivierte ESAT-6/CFP-10-spezifische Th-Zellen
- ESAT-6/CFP-10-beladene PBMC plus 210 Bead-aktivierte Th-Zellen
- ESAT-6/CFP-10-beladene PBMC plus 6636 voraktivierte ESAT-6/CFP-10-spezifische Th-Zellen
- ESAT-6/CFP-10-beladene PBMC plus 6636 Bead-aktivierte Th-Zellen
- ESAT-6/CFP-10-beladene PBMC plus 66360 voraktivierte ESAT-6/CFP-10-spezifische Th-Zellen
- ESAT-6/CFP-10-beladene PBMC plus 66360 Bead-aktivierte Th-Zellen

Ratio vs. Zeit (h)

## Figur 7

Legend:
- *M. tuberculosis* Antigen-beladene PBMC
- EBV Antigen-beladene PBMC
- CMV Antigen-beladene PBMC
- *M. tuberculosis* Antigen-beladene PBMC plus voraktivierte *M. tuberculosis*-spezifische T-Helferzellen (Th Zellen)
- EBV Antigen-beladene PBMC plus voraktivierte *M. tuberculosis*-spezifische Th-Zellen
- CMV Antigen-beladene PBMC plus voraktivierte *M. tuberculosis*-spezifische Th-Zellen

Y-axis: Ratio (0, 2, 4, 6, 8, 10, 12, 14)
X-axis: Zeit (h) — 0, 0,5, 1, 2, 3, 4, 6, 8, 10

# Figur 8 A

# Figur 8 B

CMV pp65-beladene PBMC

CMV pp65-beladene PBMC plus voraktivierte *M. tuberculosis*
*ESAT-6/CFP-10*-spezifische Th-Zellen

CMV pp65-beladene PBMC plus voraktivierte EBV BZLF1-spezifische Th-Zellen

CMV pp65-beladene PBMC plus voraktivierte CMV pp65-spezifische Th-Zellen

# Figur 8 C

Legend:
- ■ EBV BZLF1-beladene PBMC
- ▨ EBV BZLF1-beladene PBMC plus voraktivierte *M. tuberculosis ESAT-6/CFP-10*-spezifische Th-Zellen
- ▨ EBV BZLF1-beladene PBMC plus voraktivierte EBV BZLF1-spezifische Th-Zellen
- ☐ EBV BZLF1-beladene PBMC plus voraktivierte CMV pp65-spezifische Th-Zellen

Zeit (h)

Figur 9

**Fig. 10A**

**Fig. 10B**

**Fig. 10C**

Fig. 10D

Fig. 10E

Fig. 11A

# Figur 11B

Figur 11 C

**Fig. 12A**

**Fig. 12B**

# Figur 13

Figur 14

## Amplifikations Plot

HMBS · TAF1A · RPLP0 · GAPDH · ALAS1 · GUSB · HPRT1 · 18S

unstim · PPD · ESAT6/CFP10 · PMA/Iono

$C_q$

Plattenposition

Figur 15

EP 2 616 816 B1

Figur 16

EP 2 616 816 B1

Figur 17

Figur 18

EP 2 616 816 B1

Figur 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 102010037622 A

**In der Beschreibung aufgeführte Nicht-Patentliteratur**

- **SOKKE UMESHAPPA et al.** *J. Virol.,* 2009, vol. 182, 193-206
- **TOMARU et al.** *Nat Med.,* 2003, vol. 9, 469
- **KHANNA et al.** *Annu. Rev, Microbiol.,* 2000, vol. 54, 19-48
- **CROUGH et al.** *Clin Microbiol Rev.,* 2009, vol. 22, 76-98
- **VELTHUIS et al.** *Diabetes,* 2010
- **OSHIMA et al.** *J. Immunol.,* 1997, vol. 159, 3838-3848
- **DEN HAAN ; BEVAN.** *PNAS,* 2000, vol. 97, 12950-12952